(19) Europäisches Patentamt
European Patent Office
Office européen des brevets

(11) EP 1 364 023 B1

(12) **EUROPEAN PATENT SPECIFICATION**

(45) Date of publication and mention of the grant of the patent:
**07.06.2006 Bulletin 2006/23**

(21) Application number: **01994210.1**

(22) Date of filing: **07.12.2001**

(51) Int Cl.:
*C12N 15/12* (2006.01) *C07K 14/47* (2006.01)
*C07K 14/81* (2006.01) *A61K 38/17* (2006.01)

(86) International application number:
**PCT/US2001/047933**

(87) International publication number:
**WO 2002/059308 (01.08.2002 Gazette 2002/31)**

(54) **Gene highly expressed in cartilage tissues**

In Knorpelgeweben stark exprimiertes Gen

Gène exprimé fortement dans le cartilage

(84) Designated Contracting States:
**AT BE CH CY DE DK ES FI FR GB GR IE IT LI LU MC NL PT SE TR**

(30) Priority: **08.12.2000 US 254513 P**

(43) Date of publication of application:
**26.11.2003 Bulletin 2003/48**

(73) Proprietor: **GENENTECH, INC.**
**South San Francisco, CA 94080-4990 (US)**

(72) Inventors:
- **FILVAROFF, Ellen**
**San Francisco, CA 94121 (US)**
- **GODDARD, Audrey**
**San Francisco, CA 94131 (US)**
- **GRIMALDI, J., Christopher**
**San Francisco, CA 94122 (US)**
- **WOOD, William, I.**
**Hillsborough, CA 94010 (US)**

(74) Representative: **Kiddle, Simon John et al**
**Mewburn Ellis LLP**
**York House,**
**23 Kingsway**
**London WC2B 6HP (GB)**

(56) References cited:

- **BOST FREDERIC ET AL: "Inter-alpha-trypsin inhibitor proteoglycan family: A group of proteins binding and stabilizing the extracellular matrix." EUROPEAN JOURNAL OF BIOCHEMISTRY, vol. 252, no. 3, March 1998 (1998-03), pages 339-346, XP002241011 ISSN: 0014-2956**
- **SALIER JEAN-PHILIPPE ET AL: "The inter-alpha-inhibitor family: From structure to regulation." BIOCHEMICAL JOURNAL, vol. 315, no. 1, 1996, pages 1-9, XP002241012 ISSN: 0264-6021**
- **SOURY E ET AL: "THE H4P HEAVY CHAIN OF INTER-ALPHA-INHIBITOR FAMILY LARGELY DIFFERSIN THE STRUCTURE AND SYNTHESIS OF ITS PROLIN-RICH REGION FROM RAT TO HUMAN" BIOCHEMICAL AND BIOPHYSICAL RESEARCH COMMUNICATIONS, ACADEMIC PRESS INC. ORLANDO, FL, US, vol. 243, 1998, pages 522-530, XP000971500 ISSN: 0006-291X**
- **DAVEAU M ET AL: "HEPATIC AND EXTRA-HEPATIC TRANSCRIPTION OF INTER-ALPHA-INHIBITOR FAMILY GENES UNDER NORMAL OR ACUTE INFLAMMATORY CONDITIONS IN RAT" ARCHIVES OF BIOCHEMISTRY AND BIOPHYSICS, NEW YORK, US, US, vol. 350, no. 2, 15 February 1998 (1998-02-15), pages 315-323, XP000974534 ISSN: 0003-9861**
- **HASHIMOTO K ET AL: "PRIMARY STRUCTURE OF THE PIG HOMOLOGUE OF HUMAN IHRP: INTER-ALPHA-TRYPSIN INHIBITOR FAMILY HEAVY CHAIN-RELATED PROTEIN" JOURNAL OF BIOCHEMISTRY, JAPANESE BIOCHEMICAL SOCIETY, TOKYO, JP, vol. 119, no. 3, 1996, pages 577-584, XP000971480 ISSN: 0021-924X**
- **KOBAYASHI HIROSHI ET AL: "Immunolocalization of hyaluronic acid and inter-alpha-trypsin inhibitor in mice." CELL & TISSUE RESEARCH, vol. 296, no. 3, June 1999 (1999-06), pages 587-597, XP002241013 ISSN: 0302-766X cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- WISNIEWSKI H-G ET AL: "TSG-6, AN ARTHRITIS-ASSOCIATED HYALURONAN BINDING PROTEIN, FORMS A STABLE COMPLEX WITH THE SERUM PROTEIN INTER-ALPHA-INHIBITOR" BIOCHEMISTRY, AMERICAN CHEMICAL SOCIETY. EASTON, PA, US, vol. 33, no. 23, 1994, pages 7423-7429, XP009004340 ISSN: 0006-2960

Sequence Listing

<110> Genentech, Inc. et al.

<120> METHOD OF DIAGNOSING AND TREATING CARTILAGINOUS DISORDERS

<130> P2925-1R1PCT

<140> PCT/US01/47933
<141> 2001-12-07

<150> US 60/254,513
<151> 2000-12-08

<160> 6

<210> 1
<211> 4684
<212> DNA
<213> Homo Sapiens

<400> 1

```
gaacaacaga tccaggccag aggtggcatc atgtctgggt ggaggtacct 50
catctgtgtc agctttttgc tgaccattct tcttgaactg acataccagg 100
gaccccctgt ccccgcttca tcaagcacaa agttgttaat gacaagctat 150
tctatgcgct ccacggtggt gtctcgctat gcccacacct tggtcacctc 200
tgtcctgttt aatccacatg ctgaagccca tgaagccatc tttgacctgg 250
atctgcctca tcttgccttt atctccaatt tcactatgac catcaacaat 300
aaagtctaca ttgcagaagt caaagagaag caccaggcaa agaaaatcta 350
tgaagaagcc catcagcagg gaaagacagc tgctcatgta ggcatcaggg 400
accgggaatc agagaagttc cgcatctcca ccagcctggc agcaggcaca 450
gaggtgactt tttccctggc ctatgaggaa ctgcttcagc ggcaccaggg 500
ccagtaccag ctggtggtga gcctgaggcc tggccaattg gtgaagaggc 550
tgagcataga ggttacagtg tcagaaagga caggcatctc ctatgtgcac 600
ataccacccc tgaggaccgg ccgtctgcgc accaatgccc atgcaagtga 650
ggtggattca cccccatcca ccaggatcga gaggggagag acctgtgtcc 700
gaatcaccta ctgcccgaca ttgcaagacc agtcgtccat ctctgggtca 750
ggcatcatgg ctgacttcct ggttcagtac gatgtggtca tggaggacat 800
cattggagac gtgcagattt acgatgacta tttcattcac tactttgccc 850
ccagaggcct tccacctatg gagaagaatg tggtttttgt tattgacgta 900
agcagctcca tgtttggtac caagatggaa cagactaaaa cggccatgaa 950
tgtgatcctc agtgaccttc aagccaatga ctacttcaac atcatctcct 1000
tttctgacac agttaatgtt tggaaagctg gaggctcaat ccaggccacc 1050
```

atccagaatg tccacagtgc caaggactac ctgcattgca tggaagccga 1100
tggctggaca gacgtcaact cagctctgct ggcagctgct tcagtgctga 1150
accatagcaa ccaggagcct gggaggggcc ccagtgtggg gaggatccct 1200
cttatcatct tcctgacgga tggggagccc acggccggcg tgacgacccc 1250
cagtgtgatc ctctccaatg tccgtcaggc gctaggccac agggtatccc 1300
ttttcagctt ggcctttggg gatgatgctg actttacact gctgcgccgc 1350
ctgtccctgg aaaaccgggg aatagcccgg cgcatatatg aggacactga 1400
tgcggcccta cagctgaagg gcctctatga ggagatctcc atgcctctgc 1450
tggcagatgt gcgtctgaac tacctgggtg gcttggttgg ggcctcccct 1500
tgggccgttt tccccaacta ctttggtggc tctgagctgg tggtggcagg 1550
acaggtgcag ccaggcaaac aggaactggg catccacctg gcagcccgtg 1600
gccccaagga tcagcttctt gtggcccacc acagtgaagg ggccaccaac 1650
aacagccaga aggcctttgg ttgcccaggg gagccagccc ccaatgtggc 1700
ccacttcatc cgccgcctct gggcctatgt caccattgga gaactgctgg 1750
atgcacactt ccaagctcgt gacaccacca ctcgccacct gctggctgcc 1800
aaagtcctca acctgtccct tgaatacaac tttgtcacac ctctgacttc 1850
actggtcatg gtgcaaccca aacaggccag tgaggagacc aggagacaga 1900
cttccacctc tgctgggcca gacaccatca tgccctcatc cagcagcagg 1950
catggcctag ggtaagcac agctcagcca gccttggtgc ccaaggtcat 2000
ctcccccaaa tcaaggcctg tgaaaccaaa gttctactta tcctcaacta 2050
ctactgcctc taccaagaag atgctaagtt ccaaagagct ggagccattg 2100
ggagagagcc ctcataccct gtcaatgccc acatacccaa aggccaaaat 2150
tccagcacaa caggattctg gcaccttggc ccagccaact ctcaggacaa 2200
aacctaccat tcttgtgccc tcaaattctg gtactctgtt gcctctgaag 2250
cccggctctc tatcacacca gaatcctgat atattaccca cgaactccag 2300
gacacaagtc ccacctgtga aacctggcat cccagcctcg cccaaagctg 2350
acactgtgaa atgtgttact ccactgcatt ccaaacctgg tgctccatcg 2400
cacccccaac ttggggcact cacatcacag gcacctaaag gcctgccaca 2450
gtcaagacct ggagtctcta cacttcaggt tcccaagtac ccactacaca 2500
ccagacctag ggttcctgct cccaagaccc gaaacaacat gccacacctg 2550
gggcctggaa tcctcttgtc caagacccct aaaatcttat tatctcttaa 2600
accgagtgcc ccaccacacc aaatttccac aagcatatca ctttccaagc 2650

ctgagacccc aaaccccccat atgcctcaaa ccccactacc tcctagacct 2700
gacagaccaa ggcccccact tcctgagagc ctaagcacat tcccaaatac 2750
aatctcaagt tccacaggtc ccagcagtac cacaaccacc tctgtccttg 2800
gagaacccct ccccatgccc tttactccca ctctgccccc tggaaggttc 2850
tggcatcagt atgacctcct cccgggtccc cagaggacca ggcaagttct 2900
gggaccatct aggccaggag ttccaacaat gagcctactc aacagctcca 2950
ggcctacacc agaaggcagc cctccaaacc tgccaatctt gctgccttct 3000
agcatcctcc ctgaggccat cagtctgctc cttctccctg aggagctaga 3050
gctgctgtct gaatcaatgg tggaatccaa gttcgtggag tccttgaacc 3100
caccagcttt ctataccttc ctcactcctg atgaagatgg aagtccaaac 3150
tgggatggca attctgagga gatcctggga ggagctggag gcagcatgga 3200
atctcaagga agttctgtgg ggttagcaaa aggcacattg cctagcatct 3250
tcaccttctc ctcctcagtg gacggggacc cccactttgt gatccaaatc 3300
ccacactcag aagagaagat ctgcttcaca ctgaatgggc accctgggga 3350
cttgctgcag ctcatagagg acccaaaggc agggctgcat gtgagtggga 3400
agctgcttgg cgcaccacca aggccgggcc acaaggacca gactcgcacc 3450
tacttccaga tcatcacagt cactacagac aaaccccggg cctatactat 3500
caccatcagc cgcagttcta tatctttgcg aggcgagggt accttgcgcc 3550
tgtcctggga ccaacctgcc ctgctgaaga ggccccagct ggagctctat 3600
gtggctgctg cagcccgcct taccctccgc cttgggccct accttgagtt 3650
cctagtcctc cgacaccgct acaggcatcc cagtaccctg caactacccc 3700
acctggggtt ctacgtggcc aatggctcag gcctcagccc ctcagcccgt 3750
ggcctgatag ggcagttcca gcacgcagac atccgactgg tgacaggacc 3800
tatggggcca tgcttacgaa ggcaccatgg cccagatgtg cctgtgattc 3850
taggcaagag gctgctgaag gactcaccaa ggctgctgcc ccgctgggct 3900
tcctgctggc tggtgaagcg ctctcatgta gagctgcttc tgggccaccc 3950
ctacctctcc tatgtcctgt gatggcttct gaattccaga gccaggagac 4000
ctgaatttgg gagatccaga aaccagggca tggggtgagc cagggacaga 4050
gacccaagga catggaagca cacacaggga cacacagact cacgcatgtt 4100
ctaaggaaca catatacaag ggtatacaaa cacatagaca tgcagagaca 4150
tagagtcagg gactcctgca tcttcatggt ccttcacatc tccagtctca 4200
ccctctccaa atccatgctc ctcaccagcc ctacaagcca cttttgtaaa 4250

acgaaaaaat gatcagtagt tctgctcaca gctttcccta actcctatgg 4300

catattcaag gctctcagag tttccctaac aaaccatgct cactcctacc 4350

actggaccat tgcatgtgct tttccttctg catgaaacac tcttcctttg 4400

ttcctgtagg tgagtataat tcatccttca aatcctagtt gttacctctt 4450

ctaggaggcc tttcctgatg cctgccctag attgagtttg gtcctgtcct 4500

ctgggttcct atagcccacc cccaaccctc cacgctcaca gtatttatac 4550

tgtgtagtaa tcatctgttt acttgtctgg atccctgact tagactgtga 4600

gatctttgag ggcaaggacc ttttctgaat catctatgta tccccagtgc 4650

ctctcacata ataggtgctt agtaaatatt tgtt 4684

<210> 2
<211> 1313
<212> PRT
<213> Homo Sapiens

<400> 2

```
Met Ser Gly Trp Arg Tyr Leu Ile Cys Val Ser Phe Leu Leu Thr
 1               5                  10                  15

Ile Leu Leu Glu Leu Thr Tyr Gln Gly Pro Pro Val Pro Ala Ser
                20                  25                  30

Ser Ser Thr Lys Leu Leu Met Thr Ser Tyr Ser Met Arg Ser Thr
                35                  40                  45

Val Val Ser Arg Tyr Ala His Thr Leu Val Thr Ser Val Leu Phe
                50                  55                  60

Asn Pro His Ala Glu Ala His Glu Ala Ile Phe Asp Leu Asp Leu
                65                  70                  75

Pro His Leu Ala Phe Ile Ser Asn Phe Thr Met Thr Ile Asn Asn
                80                  85                  90

Lys Val Tyr Ile Ala Glu Val Lys Glu Lys His Gln Ala Lys Lys
                95                 100                 105

Ile Tyr Glu Glu Ala His Gln Gln Gly Lys Thr Ala Ala His Val
               110                 115                 120

Gly Ile Arg Asp Arg Glu Ser Glu Lys Phe Arg Ile Ser Thr Ser
               125                 130                 135

Leu Ala Ala Gly Thr Glu Val Thr Phe Ser Leu Ala Tyr Glu Glu
               140                 145                 150

Leu Leu Gln Arg His Gln Gly Gln Tyr Gln Leu Val Val Ser Leu
               155                 160                 165

Arg Pro Gly Gln Leu Val Lys Arg Leu Ser Ile Glu Val Thr Val
               170                 175                 180

Ser Glu Arg Thr Gly Ile Ser Tyr Val His Ile Pro Pro Leu Arg
               185                 190                 195
```

Thr Gly Arg Leu Arg Thr Asn Ala His Ala Ser Glu Val Asp Ser
200 205 210

Pro Pro Ser Thr Arg Ile Glu Arg Gly Glu Thr Cys Val Arg Ile
215 220 225

Thr Tyr Cys Pro Thr Leu Gln Asp Gln Ser Ser Ile Ser Gly Ser
230 235 240

Gly Ile Met Ala Asp Phe Leu Val Gln Tyr Asp Val Val Met Glu
245 250 255

Asp Ile Ile Gly Asp Val Gln Ile Tyr Asp Asp Tyr Phe Ile His
260 265 270

Tyr Phe Ala Pro Arg Gly Leu Pro Pro Met Glu Lys Asn Val Val
275 280 285

Phe Val Ile Asp Val Ser Ser Ser Met Phe Gly Thr Lys Met Glu
290 295 300

Gln Thr Lys Thr Ala Met Asn Val Ile Leu Ser Asp Leu Gln Ala
305 310 315

Asn Asp Tyr Phe Asn Ile Ile Ser Phe Ser Asp Thr Val Asn Val
320 325 330

Trp Lys Ala Gly Gly Ser Ile Gln Ala Thr Ile Gln Asn Val His
335 340 345

Ser Ala Lys Asp Tyr Leu His Cys Met Glu Ala Asp Gly Trp Thr
350 355 360

Asp Val Asn Ser Ala Leu Leu Ala Ala Ala Ser Val Leu Asn His
365 370 375

Ser Asn Gln Glu Pro Gly Arg Gly Pro Ser Val Gly Arg Ile Pro
380 385 390

Leu Ile Ile Phe Leu Thr Asp Gly Glu Pro Thr Ala Gly Val Thr
395 400 405

Thr Pro Ser Val Ile Leu Ser Asn Val Arg Gln Ala Leu Gly His
410 415 420

Arg Val Ser Leu Phe Ser Leu Ala Phe Gly Asp Asp Ala Asp Phe
425 430 435

Thr Leu Leu Arg Arg Leu Ser Leu Glu Asn Arg Gly Ile Ala Arg
440 445 450

Arg Ile Tyr Glu Asp Thr Asp Ala Ala Leu Gln Leu Lys Gly Leu
455 460 465

Tyr Glu Glu Ile Ser Met Pro Leu Leu Ala Asp Val Arg Leu Asn
470 475 480

Tyr Leu Gly Gly Leu Val Gly Ala Ser Pro Trp Ala Val Phe Pro
485 490 495

Asn Tyr Phe Gly Gly Ser Glu Leu Val Val Ala Gly Gln Val Gln
500 505 510

Pro Gly Lys Gln Glu Leu Gly Ile His Leu Ala Ala Arg Gly Pro
515 520 525

Lys Asp Gln Leu Leu Val Ala His His Ser Glu Gly Ala Thr Asn
530 535 540

Asn Ser Gln Lys Ala Phe Gly Cys Pro Gly Glu Pro Ala Pro Asn
545 550 555

Val Ala His Phe Ile Arg Arg Leu Trp Ala Tyr Val Thr Ile Gly
560 565 570

Glu Leu Leu Asp Ala His Phe Gln Ala Arg Asp Thr Thr Thr Arg
575 580 585

His Leu Leu Ala Ala Lys Val Leu Asn Leu Ser Leu Glu Tyr Asn
590 595 600

Phe Val Thr Pro Leu Thr Ser Leu Val Met Val Gln Pro Lys Gln
605 610 615

Ala Ser Glu Glu Thr Arg Arg Gln Thr Ser Thr Ser Ala Gly Pro
620 625 630

Asp Thr Ile Met Pro Ser Ser Ser Ser Arg His Gly Leu Gly Val
635 640 645

Ser Thr Ala Gln Pro Ala Leu Val Pro Lys Val Ile Ser Pro Lys
650 655 660

Ser Arg Pro Val Lys Pro Lys Phe Tyr Leu Ser Ser Thr Thr Thr
665 670 675

Ala Ser Thr Lys Lys Met Leu Ser Ser Lys Glu Leu Glu Pro Leu
680 685 690

Gly Glu Ser Pro His Thr Leu Ser Met Pro Thr Tyr Pro Lys Ala
695 700 705

Lys Ile Pro Ala Gln Gln Asp Ser Gly Thr Leu Ala Gln Pro Thr
710 715 720

Leu Arg Thr Lys Pro Thr Ile Leu Val Pro Ser Asn Ser Gly Thr
725 730 735

Leu Leu Pro Leu Lys Pro Gly Ser Leu Ser His Gln Asn Pro Asp
740 745 750

Ile Leu Pro Thr Asn Ser Arg Thr Gln Val Pro Pro Val Lys Pro
755 760 765

Gly Ile Pro Ala Ser Pro Lys Ala Asp Thr Val Lys Cys Val Thr
770 775 780

Pro Leu His Ser Lys Pro Gly Ala Pro Ser His Pro Gln Leu Gly
785 790 795

Ala Leu Thr Ser Gln Ala Pro Lys Gly Leu Pro Gln Ser Arg Pro
800 805 810

Gly Val Ser Thr Leu Gln Val Pro Lys Tyr Pro Leu His Thr Arg
815 820 825

Pro Arg Val Pro Ala Pro Lys Thr Arg Asn Asn Met Pro His Leu
830 835 840

Gly Pro Gly Ile Leu Leu Ser Lys Thr Pro Lys Ile Leu Leu Ser
845 850 855

Leu Lys Pro Ser Ala Pro Pro His Gln Ile Ser Thr Ser Ile Ser
860 865 870

Leu Ser Lys Pro Glu Thr Pro Asn Pro His Met Pro Gln Thr Pro
875 880 885

Leu Pro Pro Arg Pro Asp Arg Pro Arg Pro Pro Leu Pro Glu Ser
890 895 900

Leu Ser Thr Phe Pro Asn Thr Ile Ser Ser Ser Thr Gly Pro Ser
905 910 915

Ser Thr Thr Thr Thr Ser Val Leu Gly Glu Pro Leu Pro Met Pro
920 925 930

Phe Thr Pro Thr Leu Pro Pro Gly Arg Phe Trp His Gln Tyr Asp
935 940 945

Leu Leu Pro Gly Pro Gln Arg Thr Arg Gln Val Leu Gly Pro Ser
950 955 960

Arg Pro Gly Val Pro Thr Met Ser Leu Leu Asn Ser Ser Arg Pro
965 970 975

Thr Pro Glu Gly Ser Pro Pro Asn Leu Pro Ile Leu Leu Pro Ser
980 985 990

Ser Ile Leu Pro Glu Ala Ile Ser Leu Leu Leu Leu Pro Glu Glu
995 1000 1005

Leu Glu Leu Leu Ser Glu Ser Met Val Glu Ser Lys Phe Val Glu
1010 1015 1020

Ser Leu Asn Pro Pro Ala Phe Tyr Thr Phe Leu Thr Pro Asp Glu
1025 1030 1035

Asp Gly Ser Pro Asn Trp Asp Gly Asn Ser Glu Glu Ile Leu Gly
1040 1045 1050

Gly Ala Gly Gly Ser Met Glu Ser Gln Gly Ser Ser Val Gly Leu
1055 1060 1065

Ala Lys Gly Thr Leu Pro Ser Ile Phe Thr Phe Ser Ser Ser Val
1070 1075 1080

Asp Gly Asp Pro His Phe Val Ile Gln Ile Pro His Ser Glu Glu
1085 1090 1095

Lys Ile Cys Phe Thr Leu Asn Gly His Pro Gly Asp Leu Leu Gln
1100 1105 1110

Leu Ile Glu Asp Pro Lys Ala Gly Leu His Val Ser Gly Lys Leu
1115 1120 1125

Leu Gly Ala Pro Pro Arg Pro Gly His Lys Asp Gln Thr Arg Thr
1130 1135 1140

Tyr Phe Gln Ile Ile Thr Val Thr Thr Asp Lys Pro Arg Ala Tyr
1145 1150 1155

Thr Ile Thr Ile Ser Arg Ser Ser Ile Ser Leu Arg Gly Glu Gly
1160 1165 1170

Thr Leu Arg Leu Ser Trp Asp Gln Pro Ala Leu Leu Lys Arg Pro
1175 1180 1185

Gln Leu Glu Leu Tyr Val Ala Ala Ala Ala Arg Leu Thr Leu Arg
1190 1195 1200

Leu Gly Pro Tyr Leu Glu Phe Leu Val Leu Arg His Arg Tyr Arg
1205 1210 1215

His Pro Ser Thr Leu Gln Leu Pro His Leu Gly Phe Tyr Val Ala
1220 1225 1230

Asn Gly Ser Gly Leu Ser Pro Ser Ala Arg Gly Leu Ile Gly Gln
1235 1240 1245

Phe Gln His Ala Asp Ile Arg Leu Val Thr Gly Pro Met Gly Pro
1250 1255 1260

Cys Leu Arg Arg His His Gly Pro Asp Val Pro Val Ile Leu Gly
1265 1270 1275

Lys Arg Leu Leu Lys Asp Ser Pro Arg Leu Leu Pro Arg Trp Ala
1280 1285 1290

Ser Cys Trp Leu Val Lys Arg Ser His Val Glu Leu Leu Leu Gly
1295 1300 1305

His Pro Tyr Leu Ser Tyr Val Leu
1310

<210> 3
<211> 565
<212> DNA
<213> Artificial Sequence

<220>
<223> Virtual DNA sequence assembled from EST fragments

<400> 3

atgctgccta ttctgggcag taggcacccc tctagcctag ggcaggcccg 50
aaagttggat tataggccat ccaagagcca atgcctagaa cctgggaccc 100
caagagcctg cttggtgctc tacacccttg tggccaagct atttataatg 150
actatttcat tcactacttt gcccccagag gccttccacc tatggagaag 200
aatgtggttt ttgttattga cataagtggc ttcatgtttg gtaccaagat 250
gaaacaggat cacaacttct cactagcaag ggaacaaaac tggacagaga 300
atgagtttga caaattgatg gaagtagact tcagaaggtg ggtaataaca 350
aactcctccg agctaaagga gcatgttcta acccaatgca aggaagctaa 400
caaccttgaa aaaaggttag atgaattgct aactagaata accagtgtag 450

agaagaacat aaatgacctg atggagctga aaaacacagc aggagaactt 500
cgtgaagcat acacaagttt caatagctgc aatgatcaag cagaagaaag 550
gataacagtg attta 565

<210> 4
<211> 29
<212> DNA
<213> Artificial Sequence

<220>
<223> Forward PCR primer used in isolation of DNA 153576-2925

<400> 4
agaggccttc cacctatgga gaagaatgt 29

<210> 5
<211> 27
<212> DNA
<213> Artificial Sequence

<220>
<223> Reverse PCR primer used in isolation of DNA 153576-2925

<400> 5
gggggcaaag tagtgaatga aatagtc 27

<210> 6
<211> 47
<212> DNA
<213> Artificial Sequence

<220>
<223> Hybridization probe used in isolation of DNA 153576-2925

<400> 6
gttattgaca taagtggctt catgtttggt accaagatga aacagga 47

## Description

### FIELD OF THE INVENTION

The present invention relates generally to the identification and isolation of novel DNA and to the recombinant production of novel polypeptides having sequence similarity to human inter-alpha-trypsin inhibitor (ITI), designated herein as "PRO21074" polypeptides. The present invention further relates generally to the diagnosis and treatment of cartilaginous disorders.

### BACKGROUND OF THE INVENTION

Extracellular proteins play important roles in, among other things, the formation, differentiation and maintenance of multicellular organisms. The fate of many individual cells, *e.g.*, proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. These secreted polypeptides or signaling molecules normally pass through the cellular secretory pathway to reach their site of action in the extracellular environment.

Secreted proteins have various industrial applications, including as pharmaceuticals, diagnostics, biosensors and bioreactors. Most protein drugs available at present, such as thrombolytic agents, interferons, interleukins, erythropoietins, colony stimulating factors, and various other cytokines, are secretory proteins. Their receptors, which are membrane proteins, also have potential as therapeutic or diagnostic agents. Efforts are being undertaken by both industry and academia to identify new, native secreted proteins. Many efforts are focused on the screening of mammalian recombinant DNA libraries to identify the coding sequences for novel secreted proteins. Examples of screening methods and techniques are described in the literature [see, for example, Klein *et al., Proc. Natl. Acad. Sci.*, 93:7108-7113 (1996); U.S. Patent No. 5,536,637)].

Inter-alpha-trypsin inhibitor (TIT) is a human plasma glycoprotein comprised of a complex of three different proteins: bikunin and heavy chains, H1 and H2. Morelle *et al.* demonstrated that these three chains are covalently linked by a chondrotin sulphate chain. (*Eur. J. Biochem.*, 221(2):881-8 (1994).) See also, Enghild, J. J. *et al., J. Biol. Chem.* 268(12):8711-8716 (1993). Bost *et al.* report that mRNA for the ITI heavy chain, H1, is found exclusively in liver tissue. Bost F. *et al., Eur. J. Biochem.* 218(2):283-91 (1993).

Human ITI has been found to inactivate human trypsin, chymotrypsin, neutrophil elastase and cathespin G. Morii, M. and Travis, J., Biol. Chem. Hoppe Seyler 366(1):19-21 (1985). It has been reported that the heavy chains contain potential calcium binding sites and regions homologous to the proposed reactive site for thiol-proteinase inhibitors, indicating that ITI is a complex, multi-functional protein. Salier, J. P. *et al., Proc. Nat=l. Acad. Sci. U.S.A.* 84(23):8272-6 (1987). Moreover, the interaction of ITI and hyaluronic acid is believed to play a critical role in the organization and stabilization of the extracellular matrix. H. Kobayashi *et al., Cell Tissue Res.* 296:587-597 (1999).

It has also been suggested that ITI may act as a carrier of hyaluronan in serum, or as a binding protein between hyaluronan and other matrix proteins, and that it may play a role in the stimulation of phagocytic cells.

It has further been suggested that ITI, an inhibitor of serine proteases, may protect articular chondrocytes and prevent cartilage damage in cartilaginous disorders. D.C. Fischer *et al., Arthritis Rheum.* 42(9): 1936-45 (1999).

We herein describe the identification and characterization of novel polypeptides having sequence similarity to human inter-alpha-trypsin inhibitor (ITI), designated herein as PRO21074 polypeptides, and their potential use in the diagnosis and treatment of cartilaginous disorders.

### SUMMARY OF THE INVENTION

The present application describes methods for the diagnosis and treatment of cartilaginous disorders. Furthermore, the present invention provides a cDNA clone (designated herein as DNA153576-2925) - identified herein as having homology to nucleic acid encoding human inter-alpha-trypsin inhibitor (ITI) - that encodes a novel polypeptide, designated in the present application as "PRO21074".

In one embodiment, the invention provides an isolated nucleic acid moiecuie comprising a nucleotide sequence that encodes a PRO21074 polypeptide.

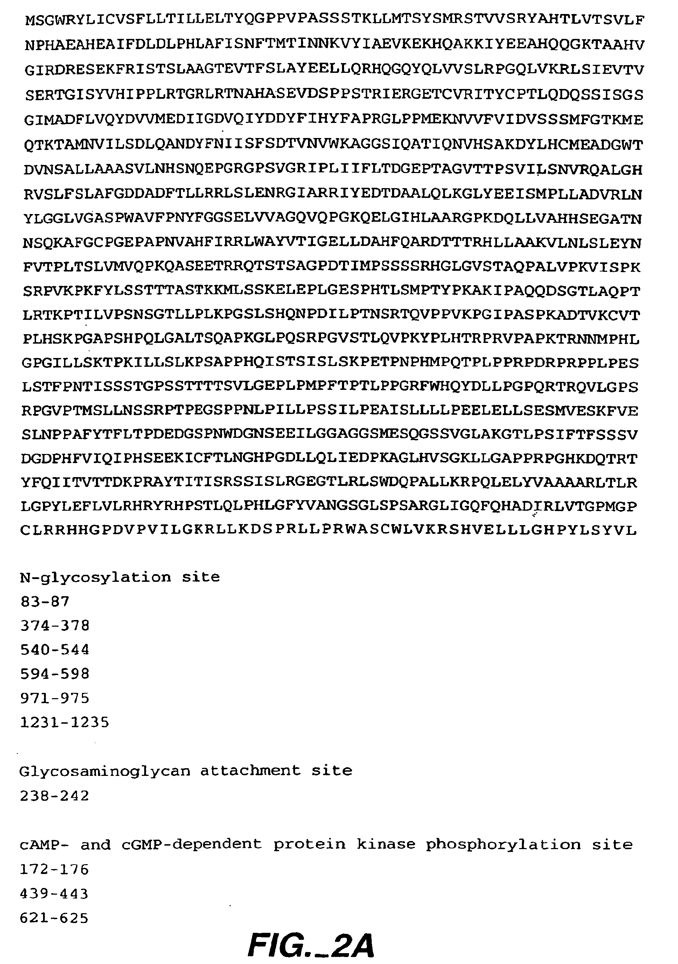

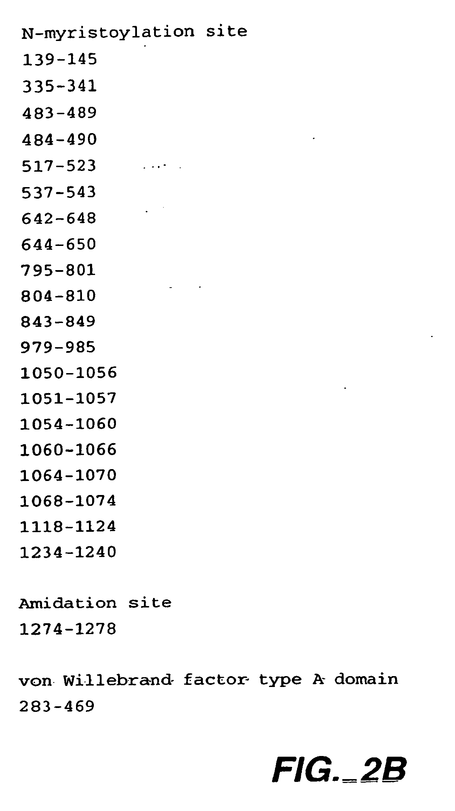

In one aspect, the isolated nucleic acid molecule comprises a nucleotide sequence having at least 80% sequence identity, alternatively at least about 81% sequence identity, alternatively at least about 82% sequence identity, alternatively at least about 83% sequence identity, alternatively at least about 84% sequence identity, alternatively at least about 85% sequence identity, alternatively about 86% sequence identity, alternatively at least about 87% sequence identity, alternatively at least about 88% sequence identity, alternatively at least about 89% sequence identity, alternatively at least about 90% sequence identity, alternatively at least about 91% sequence identity, alternatively at least about 92% sequence identity, alternatively at least about 93% sequence identity, alternatively at least about 94% sequence identity, alternatively at least about 95% sequence identity, alternatively at least about 96% sequence identity, alternatively at least about 97% sequence identity, alternatively at least about 98% sequence identity and alternatively at least about 99% sequence identity to (a) a DNA molecule encoding a PR021074 polypeptide having the sequence of amino acid residues from 1 or 24 to 1313, inclusive, of Figure 2 (SEQ ID NO: 2), or (b) the complement of the DNA molecule of (a).

In another aspect, the isolated nucleic acid molecule comprises (a) a nucleotide sequence encoding a PR021074 polypeptide having the sequence of amino acid residues from 1 or 24 to 1313, inclusive, of Figure 2 (SEQ ID NO: 2), or (b) the complement of the nucleotide sequence of (a).

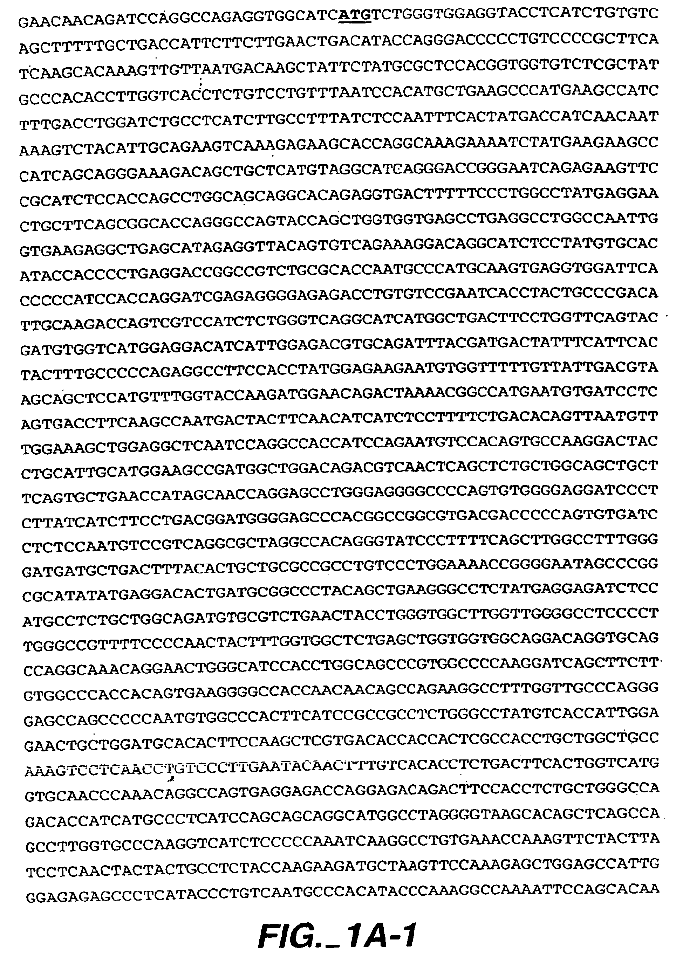

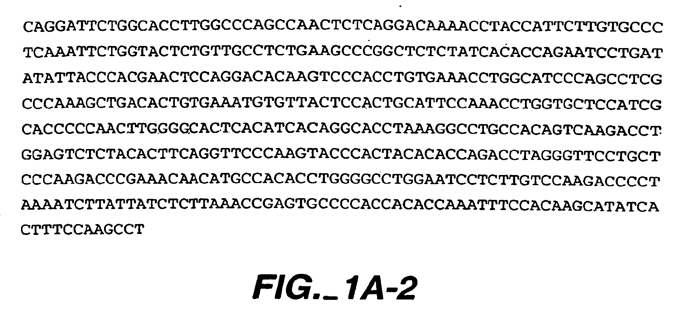

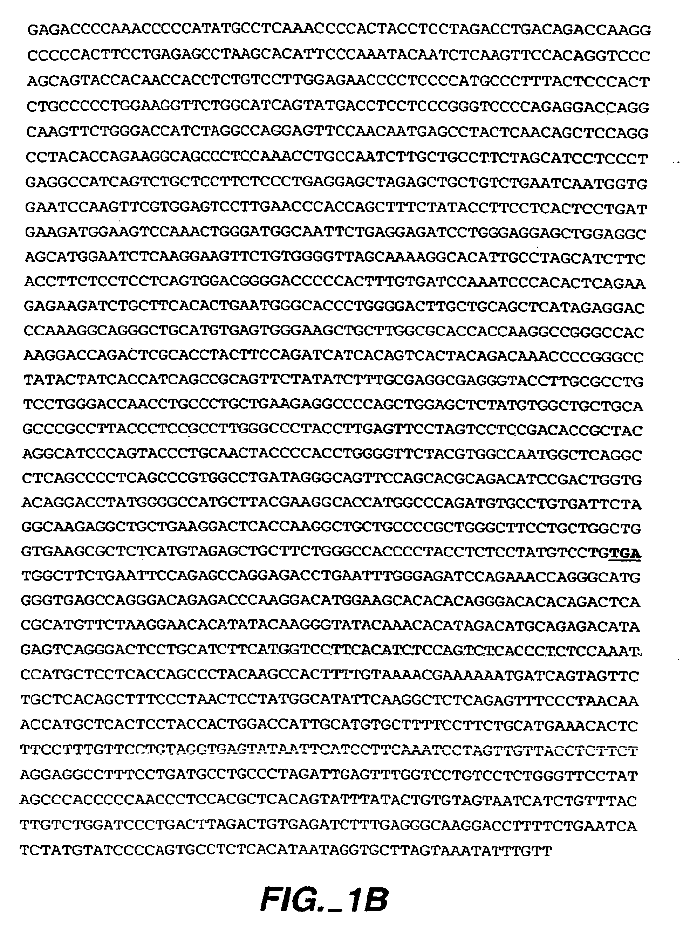

The isolated nucleic acid molecule may comprise a nucleotide sequence having at least 80% sequence identity, alternatively at least about 81% sequence identity, alternatively at least about 82% sequence identity, alternatively at least about 83% sequence identity, alternatively at least about 84% sequence identity, alternatively at least about 85% sequence identity, alternatively at least about 86% sequence identity, alternatively at least about 87% sequence identity, alternatively at least about 88% sequence identity, alternatively at least about 89% sequence identity, alternatively at least about 90% sequence identity, alternatively at least about 91% sequence identity, alternatively at least about 92% sequence identity, alternatively at least about 93% sequence identity, alternatively at least about 94% sequence identity, alternatively at least about 95% sequence identity, alternatively at least about 96% sequence identity, alternatively at least about 97% sequence identity, alternatively at least about 98% sequence identity and alternatively at least about 99% sequence identity to (a) a DNA molecule having the sequence of nucleotides from 31 or 100 to 3969, inclusive, of Figure 1 (SEQ ID NO: 1), or (b) the complement of the DNA molecule of (a).

The isolated nucleic acid molecule may comprise (a) the nucleotide sequence of from 31 or 100 to 3969, inclusive, of Figure 1 (SEQ ID NO: 1), or (b) the complement of the nucleotide sequence of (a).

In a further aspect, the invention provides an isolated nucleic acid molecule comprising a nucleotide sequence having at least 80% sequence identity, alternatively at least about 81 % sequence identity, alternatively at least about 82% sequence identity, alternatively at least about 83% sequence identity, alternatively at least about 84% sequence identity, alternatively at least about 85% sequence identity, alternatively at least about 86% sequence identity, alternatively at least about 87% sequence identity, alternatively at least about 88% sequence identity, alternatively at least about 89% sequence identity, alternatively at least about 90% sequence identity, alternatively at least about 91% sequence identity, alternatively at least about 92% sequence identity, alternatively at least about 93% sequence identity, alternatively at least about 94% sequence identity, alternatively at least about 95% sequence identity, alternatively at least about 96% sequence identity, alternatively at least about 97% sequence identity, alternatively at least about 98% sequence identity and alternatively at least about 99% sequence identity to (a) a DNA molecule that encodes the same mature polypeptide encoded by the human cDNA deposited with the ATCC on May 23, 2000 under ATCC Deposit No. 1907-PTA (DNA153576-2925) or (b) the complement of the DNA molecule of (a). In a preferred embodiment, the isolated nucleic acid molecule comprises (a) a nucleotide sequence encoding the same mature polypeptide encoded by the human cDNA deposited with the ATCC on May 23, 2000 under ATCC Deposit No. 1907-PTA (DNA 153576-2925) or (b) the complement of the nucleotide sequence of (a).

In another aspect, the invention provides an isolated nucleic acid molecule comprising a nucleotide sequence having at least 80% sequence identity, alternatively at least about 81% sequence identity, alternatively at least about 82% sequence identity, alternatively at least about 83% sequence identity, alternatively at least about 84% sequence identity, alternatively at least about 85% sequence identity, alternatively at least about 86% sequence identity, alternatively at least about 87% sequence identity, alternatively at least about 88% sequence identity, alternatively at least about 89% sequence identity, alternatively at least about 90% sequence identity, alternatively at least about 91% sequence identity, alternatively at least about 92% sequence identity, alternatively at least about 93% sequence identity, alternatively at least about 94% sequence identity, alternatively at least about 95% sequence identity, alternatively at least about 96% sequence identity, alternatively at least about 97% sequence identity, alternatively at least about 98% sequence identity and alternatively at least about 99% sequence identity to (a) the full-length polypeptide coding sequence of the human cDNA deposited with the ATCC on May 23, 2000 under ATCC Deposit No. 1907-PTA (DNA153576-2925) or (b) the complement of the nucleotide sequence of (a). In a preferred embodiment, the isolated nucleic acid molecule comprises (a) the full-length polypeptide coding sequence of the DNA deposited with the ATCC on May 23, 2000 under ATCC Deposit No. 1907-PTA (DNA 153576-2925) or (b) the complement of the nucleotide sequence of (a).

In another aspect, the invention provides an isolated nucleic acid molecule which encodes an active PRO21074 polypeptide as defined below comprising a nucleotide sequence comprising at least 2036 nucleotides that hybridizes to the complement of a nucleic acid sequence that encodes amino acids 1 or 24 to 1313, inclusive, of Figure 2 (SEQ ID NO: 2). Hybridization occurs under stringent hybridization and wash conditions.

The application also describes an isolated nucleic acid molecule which encodes an active PRO21074 polypeptide as defined below comprising a nucleotide sequence that hybridizes to the complement of the nucleic acid sequence between about nucleotides 31 or 100 and 3969, inclusive, of Figure 1 (SEQ ID NO: 1). Preferably, hybridization occurs under stringent hybridization and wash conditions.

In a further aspect, the invention provides an isolated nucleic acid molecule having at least 2036 nucleotides and which is produced by hybridizing a test DNA molecule under stringent conditions with (a) a DNA molecule encoding a PR021074 polypeptide having the sequence of amino acid residues from 1 or 24 to 1313, inclusive, of Figure 2 (SEQ ID NO: 2), or (b) the complement of the DNA molecule of (a), and, if the test DNA molecule has at least an 80% sequence identity, alternatively at least about an 81% sequence identity, alternatively at least about an 82% sequence identity, alternatively at least about an 83% sequence identity, alternatively at least about an 84% sequence identity, alternatively at least about an 85% sequence identity, alternatively at least about an 86% sequence identity, alternatively at least about an 87% sequence identity, alternatively at least about an 88% sequence identity, alternatively at least about an 89% sequence identity, alternatively at least about a 90% sequence identity, alternatively at least about a 91% sequence identity, alternatively at least about a 92% sequence identity, alternatively at least about a 93% sequence identity, alternatively at least about a 94% sequence identity, alternatively at least about a 95% sequence identity, alternatively at least about a 96% sequence identity, alternatively at least about a 97% sequence identity, alternatively at least about a 98% sequence identity and alternatively at least about a 99% sequence identity to (a) or (b), and isolating the test DNA molecule.

In a specific aspect, the invention provides an isolated nucleic acid molecule comprising DNA encoding a PRO21074 polypeptide without the N-terminal signal sequence and/or the initiating methionine, or is complementary to such encoding nucleic acid molecule. The signal peptide has been tentatively identified as extending from about amino acid position 1 to about amino acid position 23 in the sequence of Figure 2 (SEQ ID NO: 2). It is noted, however, that the C-terminal boundary of the signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (*e.g.*, Nielsen *et al., Prot. Eng.* 10:1-6 (1997) and von Heinje *et al., Nucl. Acids. Res.* 14:4683-4690 (1986)). Moreover, it is also recognized that, in some cases, cleavage of a signal sequence from a secreted polypeptide is not entirely uniform, resulting in more than one secreted species. These polypeptides, and the polynucleotides encoding them, are contemplated by the present invention. As such, for purposes of the present application, the signal peptide of the PRO21074 polypeptide shown in Figure 2 (SEQ ID NO: 2) extends from amino acids 1 to X of Figure 2 (SEQ ID NO: 2), wherein X is any amino acid from 18 to 28 of Figure 2 (SEQ ID NO: 2). Therefore, mature forms of the PRO21074 polypeptide which are encompassed by the present invention include those comprising amino acids X to 1313 of Figure 2 (SEQ ID NO: 2), wherein X is any amino acid from 18 to 28 of Figure 2 (SEQ ID NO: 2) and variants thereof as described below. Isolated nucleic acid molecules encoding these polypeptides are also contemplated.

The present application also describes polynucleotide fragments of a PRO21074 polypeptide coding sequence that may find use as, for example, hybridization probes or for encoding fragments of a PRO21074 polypeptide that may optionally encode a polypeptide comprising a binding site for an anti-PRO21074 antibody. Such nucleic acid fragments can be at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. The nucleotide sequence fragment can be derived from any segment shown in Figure 1 (SEQ ID NO: 1), *e.g.*, coding and untranslated regions. In an alternative embodiment, the nucleotide sequence fragment is derived from any coding region of the nucleotide sequence shown in Figure 1 (SEQ ID NO: 1). It is noted that novel fragments of a PRO21074 polypeptide-encoding nucleotide sequence may be determined in a routine manner by aligning the PRO21074 polypeptide-encoding nucleotide sequence with other known nucleotide sequences using any of a number of well known sequence alignment programs and determining which PRO21074 polypeptide-encoding nucleotide sequence fragment(s) are novel. All of such PRO21074 polypeptide-encoding nucleotide sequences are contemplated herein and can be determined without undue experimentation. Also contemplated are the PRO21074 polypeptide fragments encoded by these nucleotide molecule fragments, preferably those PRO21074 polypeptide fragments that comprise a binding site for an anti-PRO21074 antibody.

In another embodiment, the invention provides a vector comprising a nucleotide sequence encoding PRO21074. The vector may comprise any of the isolated nucleic acid molecules hereinabove identified.

In another embodiment, the invention provides a host cell comprising a vector encoding PRO21074 or its variants. By way of example, the host cells may be CHO cells, *E. coli*, or yeast.

In another embodiment, the invention provides a process for producing PRO21074 polypeptides comprising culturing host cells under conditions suitable for expression of PRO21074 and recovering PRO21074 from the cell culture.

In another embodiment, the invention provides isolated PRO21074 polypeptide encoded by any of the isolated nucleic acid sequences hereinabove identified.

In a specific aspect, the invention provides isolated native sequence PRO21074 polypeptide, which in certain embodiments, includes an amino acid sequence comprising residues from about 1 or about 24 to about 1313 of Figure 2 (SEQ ID NO: 2).

In another aspect, the invention provides an isolated PRO21074 polypeptide, comprising an amino acid sequence having at least 80% sequence identity, alternatively at least about 81% sequence identity, alternatively at least about 82% sequence identity, alternatively at least about 83% sequence identity, alternatively at least about 84% sequence identity, alternatively at least about 85% sequence identity, alternatively at least about 86% sequence identity, alternatively at least about 87% sequence identity, alternatively at least about 88% sequence identity, alternatively at least about 89% sequence identity, alternatively at least about 90% sequence identity, alternatively at least about 91% sequence identity, alternatively at least about 92% sequence identity, alternatively at least about 93% sequence identity, alternatively at least about 94% sequence identity, alternatively at least about 95% sequence identity, alternatively at least about 96% sequence identity, alternatively at least about 97% sequence identity, alternatively at least about 98% sequence identity and alternatively at least about 99% sequence identity to the sequence of amino acid residues from 1 or 24 to 1313, inclusive, of Figure 2 (SEQ ID NO: 2).

In a further aspect, the invention provides an isolated PRO21074 polypeptide comprising an amino acid sequence having at least 80% sequence identity, alternatively at least about 81% sequence identity, alternatively at least about 82% sequence identity, alternatively at least about 83% sequence identity, alternatively at least about 84% sequence identity, alternatively at least about 85% sequence identity, alternatively at least about 86% sequence identity, alternatively at least about 87% sequence identity, alternatively at least about 88% sequence identity, alternatively at least about 89% sequence identity, alternatively at least about 90% sequence identity, alternatively at least about 91 % sequence identity, alternatively at least about 92% sequence identity, alternatively at least about 93% sequence identity, alternatively at least about 94% sequence identity, alternatively at least about 95% sequence identity, alternatively at least about 96% sequence identity, alternatively at least about 97% sequence identity, alternatively at least about 98% sequence identity and alternatively at least about 99% sequence identity to an amino acid sequence encoded by the human cDNA deposited with the ATCC on May 23, 2000 under ATCC Deposit No. 1907-PTA (DNA153576-2925). In a preferred embodiment, the isolated PRO21074 polypeptide comprises an amino acid sequence encoded by the human cDNA deposited with the ATCC on May 23, 2000 under ATCC Deposit No. 1907-PTA (DNA153576-2925).

In a specific aspect, the invention provides an isolated PRO21074 polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PRO21074 polypeptide and recovering the PRO21074 polypeptide from the cell culture.

In yet another aspect, the invention provides an isolated PRO21074 polypeptide, comprising the sequence of amino acid residues from 1 or 24 to 1313, inclusive, of Figure 2 (SEQ ID NO: 2). The identification of PRO21074 polypeptide fragments that possess biological activity or provide a binding site for an anti-PRO21074 antibody may be accomplished in a routine manner using techniques which are well known in the art. Preferably, the PRO21074 fragment retains a qualitative biological activity of a native PRO21074 polypeptide.

In a still further aspect, the invention provides a polypeptide produced by (i) hybridizing a test DNA molecule under stringent conditions with (a) a DNA molecule encoding a PRO21074 polypeptide having the sequence of amino acid residues from 1 or 24 to 1313, inclusive, of Figure 2 (SEQ ID NO: 2), or (b) the complement of the DNA molecule of (a), and if the test DNA molecule has at least an 80% sequence identity, alternatively at least about an 81% sequence identity, alternatively at least about an 82% sequence identity, alternatively at least about an 83% sequence identity, alternatively at least about an 84% sequence identity, alternatively at least about an 85% sequence identity, alternatively at least about an 86% sequence identity, alternatively at least about an 87% sequence identity, alternatively at least about an 88% sequence identity, alternatively at least about an 89% sequence identity, alternatively at least about a 90% sequence identity, alternatively at least about a 91 % sequence identity, alternatively at least about a 92% sequence identity, alternatively at least about a 93% sequence identity, alternatively at least about a 94% sequence identity, alternatively at least about a 95% sequence identity, alternatively at least about a 96% sequence identity, alternatively at least about a 97% sequence identity, alternatively at least about a 98% sequence identity and alternatively at least about a 99% sequence identity to (a) or (b), (ii) culturing a host cell comprising the test DNA molecule under conditions suitable for expression of the polypeptide, and (iii) recovering the polypeptide from the cell culture.

In another embodiment, the invention provides chimeric molecules comprising a PRO21074 polypeptide fused to a heterologous polypeptide or amino acid sequence, wherein the PRO21074 polypeptide may comprise any PRO21074 polypeptide, variant or fragment thereof as hereinbefore described. An example of such a chimeric molecule comprises a PRO21074 polypeptide fused to an epitope tag sequence or a Fc region of an immunoglobulin.

In another embodiment, the invention provides an antibody as defined below which specifically binds to a PRO21074 polypeptide as hereinbefore described. Optionally, the antibody is a monoclonal antibody, an antibody fragment or a single chain antibody.

The present application also describes agonists and antagonists of a native PRO21074 polypeptide as defined below. The agonist or antagonist may be an anti-PRO21074 antibody or a small molecule.

The application also describes a method of identifying agonists or antagonists to a PRO21074 polypeptide which comprise contacting the PRO21074 polypeptide with a candidate molecule and monitoring a biological activity mediated by said PRO21074 polypeptide. Preferably, the PRO21074 polypeptide is a native PRO21074 polypeptide.

In a still further embodiment, the invention provides a composition of matter comprising a PRO21074 polypeptide, or an anti-PRO21074 antibody, in combination with a carrier. The carrier is a pharmaceutically acceptable carrier.

The present application also provides the use of a PRO21074 polypeptide, or an agonist or antagonist thereof as herein described, or an anti-PRO21074 antibody, for the preparation of a medicament useful in the treatment of a condition which is responsive to the PRO21074 polypeptide, an agonist or antagonist thereof or an anti-PRO21074 antibody.

Also described herein is a method of using a PRO21074 polypeptide for a method of diagnosing or monitoring the progression of a cartilaginous disorder. Said method may comprise measuring the level of the PRO21074 polypeptide in the serum or synovial fluid, wherein a change in the level of said polypeptide relative to normal tissue correlates with the relative severity or prognosis of said disorder. The PRO21074 polypetpide may be measured in the urine or cartilage matrix.

Also described herein is a method of treating a mammal suffering from a cartilaginous disorder, comprising administering to said mammal a therapeutically effective amount of a PRO21074. Optionally the cartilaginous disorder is a degenerative cartilaginous disorder. The degenerative cartilaginous disorder may be arthritis, more specifically osteoarthritis or rheumatoid arthritis. Optionally, the cartilage is articular cartilage. The method may further comprise the combination of PRO21074 with a standard surgical technique and/or an effective amount of at least one cartilage agent. Optionally, the PRO21074 further comprises a carrier, excipient or stabilizer.

The application also describes a method of treating a mammal suffering from a cartilaginous disorder, comprising administering to said mammal a therapeutically effective amount of a PRO21074 antagonist. Optionally the cartilaginous disorder is a degenerative cartilaginous disorder. The degenerative cartilaginous disorder may be arthritis, more specifically osteoarthritis or rheumatoid arthritis. The PRO21074 antagonist may be an anti-PRO21074 antibody. Optionally, the cartilage is articular cartilage. The method may further comprise the combination of PRO21074 antagonist with a standard surgical technique and/or an effective amount of at least one cartilage agent. Optionally, the PRO21074 antagonist further comprises a carrier, excipient or stabilizer.

The application also describes a method for the treatment of damaged cartilage or for preventing damage to cartilage, comprising contacting said cartilage with an effective amount of a PRO21074 or antagonist thereof. The PRO21074 antagonist may be an anti-PRO21074 antibody. The cartilage may be articular cartilage. More specifically, the cartilage damage results from a cartilaginous a disorder, even more specifically, a degenerative cartilaginous disorder. Even more specificly, the cartilaginous disorder is arthritis, including, *e.g.*, rheumatoid and osteoarthritis. Alternatively, said damage can result from injury, *e.g.,* microdamage or blunt trauma, a chondral fracture, an osteochondral fracture, damage to tendons, menisci or ligaments or the result of excessive mechanical stress or other biomechanical instability resulting from an injury or obesity. The cartilage may be contained within a mammal, including humans, and the amount administered to said mammal is a therapeutically effective amount. PRO21074 polypeptide or antagonist may be administered via injection or infusion by intravenous, intraarterial, intraperitoneal, intramuscular, intralesional, intraarticular or topical administration. Alternatively, the composition may be injected directly into the afflicted cartilaginous region or joint. The method may further comprise an effective amount of a cartilage agent and/or a standard surgical technique. The PRO21074 polypeptide or antagonist may be administered prior, after and/or simultaneous to the standard cartilage surgical technique. The effective amount of PRO21074 polypeptide or antagonist may further comprise an effective amount of cartilage agent.

Also described herein is a method of treating damaged cartilage or preventing initial or continued damage comprising contacting said cartilage with an effective amount of a PRO21074 polypeptide in combination with an effective amount of a cartilage agent. Optionally, the cartilage is present inside a mammal and the amount administered is a therapeutically effective amount.

Also described herein is a method of maintaining, enhancing or promoting the growth of chondrocytes in serum-free culture by culturing the chondrocytes with an effective amount of PRO21074 polypeptide. Alternatively, the method provides contacting said chondrocytes with an effective amount of PRO21074 polypeptide in a sustained or extended-release formulation.

Also described herein is a method of treating damaged cartilage or preventing the initial or continued damage, comprising treating cells in and around the joints with an effective amount of PRO21074 polypeptide. Said treatment may occur by exogenous application of the PRO21074 polypeptide, or alternatively, through introduction of nucleic acid encoding PRO21074 or fragments thereof, into said cells via transfection, infection, or other standard *in vitro* or *in vivo* techniques. An *in vitro* therapy technique may comprise removing a "cell in and around the joint" introducing said nucleic acid into said cell, and transplanting the treated cell back into the tissue from which it was initially removed. The cartilage related cells may be chondrocytes, synoviocytes, fibroblasts, cells within tendons or ligaments, osteoblasts or myoblasts.

Alternatively, the application describes a method of promoting the adherence of chondrocytes to each other or to cartilage matrix. In a specific aspect, the adherence occurs in an *in vitro* serum-free culture. The adherence may occur *in vivo*.

The application also describes a therapeutic kit, comprising PRO21074 and a carrier, excipient, and/or stabilizer (*e.g.*, a buffer) in suitable packaging. The kit preferably contains instructions for using the PRO21074 to treat damaged cartilage or to prevent initial or continued damage to cartilage as a result of injury or a cartilaginous disorder. Alternatively, the kit may contain instructions for using PRO21074 to treat a cartilaginous disorder. Also described herein is an article of manufacture, comprising:

a container;
an instruction on the container; and
a composition comprising an active agent contained within the container;
wherein the composition is effective for treating a cartilaginous disorder, the instruction on the container indicates that the composition can be used to treat a cartilaginous disorder, and the active agent in the composition is a PRO21074 polypeptide.

BRIEF DESCRIPTION OF THE DRAWINGS

Figure 1 shows the nucleotide sequence (SEQ ID NO: 1) of a cDNA containing a nucleotide sequence (nucleotides 31-3969) encoding native sequence PRO21074, wherein the nucleotide sequence (SEQ ID NO: 1) is a clone designated herein as ADNA153576-2925". Also presented in bold font and underlined are the positions of the respective start and stop codons.

Figures 2A-B show the amino acid sequence (SEQ ID NO: 2) of a native sequence PRO21074 polypeptide as derived from the coding sequence of SEQ ID NO: 1. Also shown are the approximate locations of various other important polypeptide domains.

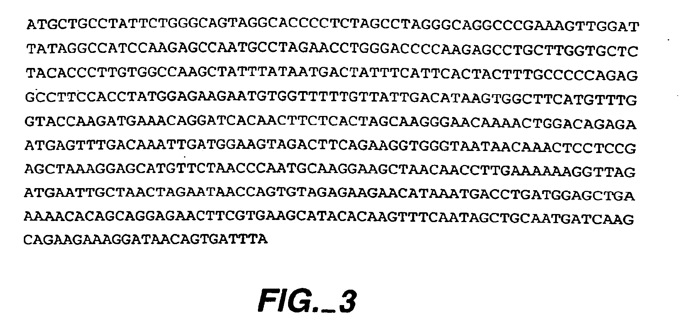

Figure 3 shows a nucleotide sequence designated herein as DNA144306 (SEQ ID NO: 3).

Figure 4 shows an analysis of expression of DNA 153576 in libraries prepared from a number of different tissues relative to the expression observed in fetal articular cartilage. As indicated, expression appeared to be highly specific to cartilage. For all samples, 50ng of cDNA library was used per reaction. All samples were normalized to beta actin and plotted relative to DNA153576 expression in the fetal articular cartilage library.

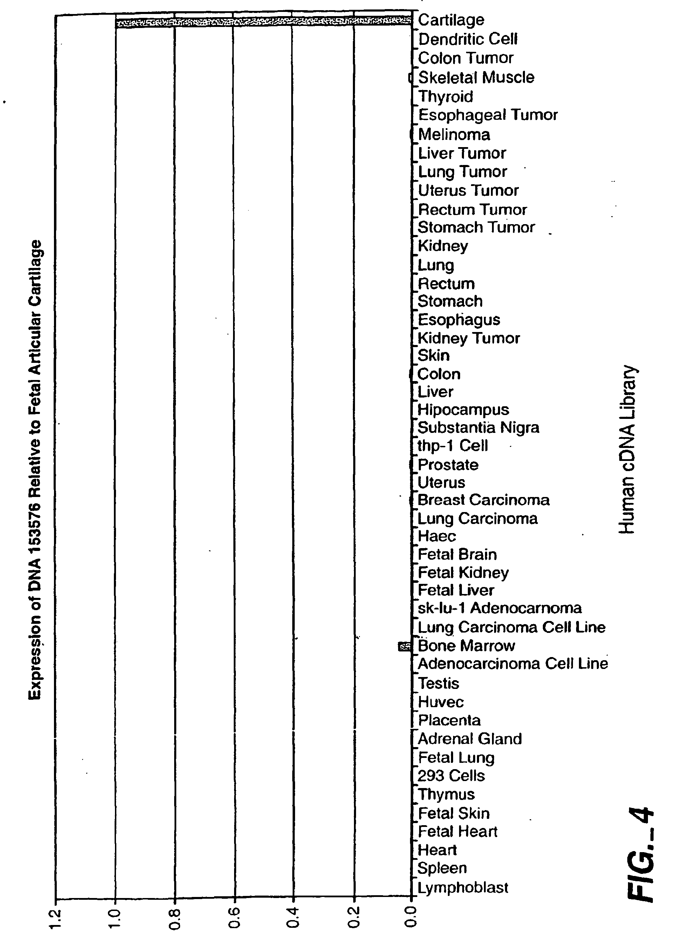

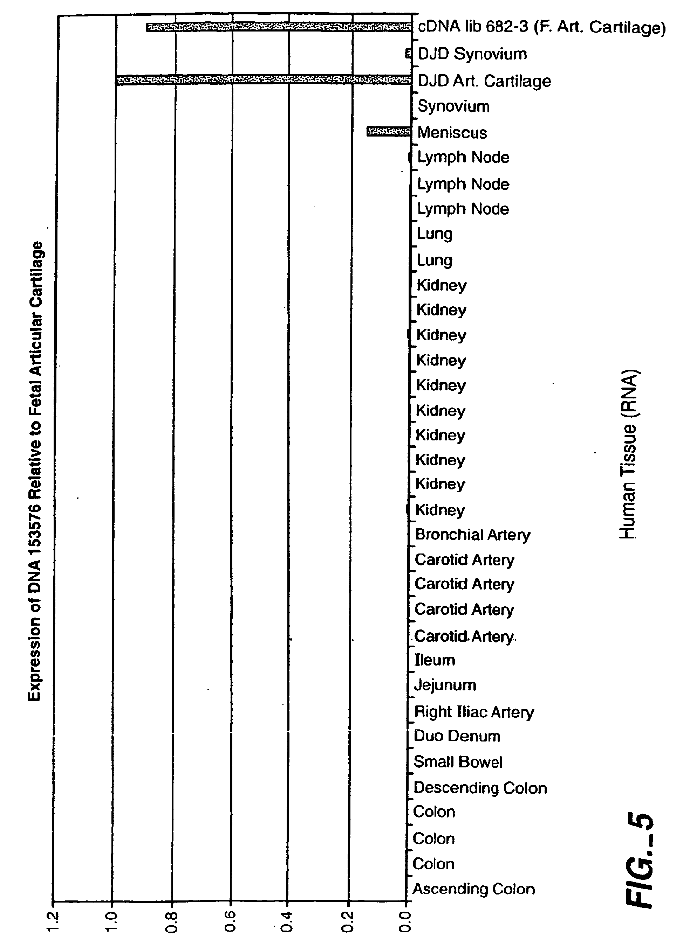

Figure 5 shows an analysis of the expression of DNA 153576 in various tissues relative to the expression observed in fetal articular cartilage. The graph illustrates relative expression that is very specific to cartilage (healthy adult, diseased and fetal) and the meniscus. The amount of RNA used per tissue sample was 6-50 ng depending on the sample. All samples were normalized to beta actin and plotted relative to DNA153576 expression in the degenerative joint disease (DJD) articular cartilage sample. The expression level of DNA153576 in cDNA library 682-3 fetal articular cartilage was taken from "cartilage" in Figure 4 and was included to allow relative comparisons between Figures 4 and 5.

Figure 6 shows an expanded logarithmic analysis of Figure 5, further illustrating the expression of DNA 153576 in tissues of the joint relative to the expression observed in fetal articular cartilage.

## DETAILED DESCRIPTION OF THE PREFERRED EMBODIMENTS

### I. Definitions

The terms "PRO21074 polypeptide", "PRO21074 protein" and "PRO21074" when used herein encompass native sequence PRO21074 and PRO21074 polypeptide variants (which are further defined herein). The PRO21074 polypeptide may be isolated from a variety of sources, such as from human tissue types or from another source, or prepared by recombinant and/or synthetic methods.

A "native sequence PRO21074" comprises a polypeptide having the same amino acid sequence as a PRO21074 derived from nature. Such native sequence PRO21074 can be isolated from nature or can be produced by recombinant and/or synthetic means. The term "native sequence PRO21074" specifically encompasses naturally-occurring truncated or secreted forms (*e.g.*, an extracellular domain sequence), naturally-occurring variant forms (*e.g.*, alternatively spliced forms) and naturally-occurring allelic variants of the PRO21074. In one embodiment of the invention, the native sequence PRO21074 is a full-length native sequence PRO21074 comprising amino acids 1 to 1313 of Figure 2 (SEQ ID NO: 2). Also, while the PRO21074 polypeptide disclosed in Figure 2 (SEQ ID NO: 2) is shown to begin with the methionine residue designated herein as amino acid position 1, it is conceivable and possible that another methionine residue located either upstream or downstream from amino acid position 1 in Figure 2 (SEQ ID NO: 2) may be employed as the starting amino acid residue for the PRO21074 polypeptide.

"PRO21074 variant polypeptide" means an active PRO21074 polypeptide as defined below having at least about 80% amino acid sequence identity with the amino acid sequence of (a) residues 1 or about 24 to 1313 of the PRO21074 polypeptide shown in Figure 2 (SEQ ID NO: 2), (b) X to 1313 of the PRO21074 polypeptide shown in Figure 2 (SEQ ID NO: 2), wherein X is any amino acid residue from 18 to 28 of Figure 2 (SEQ ID NO: 2), or (c) another specifically derived fragment of the amino acid sequence shown in Figure 2 (SEQ ID NO: 2). Such PRO21074 variant polypeptides include, for instance, PRO21074 polypeptides wherein one or more amino acid residues are added, or deleted, at the N- and/or C-terminus, as well as within one or more internal domains, of the sequence of Figure 2 (SEQ ID NO: 2). Ordinarily, a PRO21074 variant polypeptide will have at least 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity with (a) residues 1 or about 24 to 1313 of the PRO21074 polypeptide shown in Figure 2 (SEQ ID NO: 2), (b) X to 1313 of the PRO21074 polypeptide shown in Figure 2 (SEQ ID NO: 2), wherein X is any amino acid residue from 18 to 28 of Figure 2 (SEQ ID NO: 2), or (c) another specifically derived fragment of the amino acid sequence shown in Figure 2 (SEQ ID NO: 2). PRO21074 variant polypeptides do not encompass the native PRO21074 polypeptide sequence. Ordinarily, PRO21074 variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, more often at least about 150 amino acids in length, more often at least about 200 amino acids in length, more often at least about 250 amino acids in length, more often at least about 300 amino acids in length, or more.

"Percent (%) amino acid sequence identity" with respect to the PRO21074 polypeptide sequences identified herein is defined as the percentage of amino acid residues in a candidate sequence that are identical with the amino acid residues in a PRO21074 sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance; using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are obtained as described below by using the sequence comparison computer program ALIGN-2, wherein the complete source code for the ALIGN-2 program is provided in Table 2. The ALIGN-2 sequence comparison computer program was authored by Genentech, Inc. and the source code shown in Table 2 has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 2. The ALIGN-2 program should be compiled for use on a UNIX operating system, preferably digital UNIX V4.0D. All sequence comparison parameters are set by the ALIGN-2 program and do not vary.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations, Table 1A-B demonstrate how to calculate the % amino acid sequence identity of the amino acid sequence designated "Comparison Protein" to the amino acid sequence designated "PRO".

Unless specifically stated otherwise, all % amino acid sequence identity values used herein are obtained as described above using the ALIGN-2 sequence comparison computer program. However, % amino acid sequence identity may also be determined using the sequence comparison program NCBI-BLAST2 (Altschul *et al., Nucleic Acids Res.* 25:3389-3402 (1997)). The NCBI-BLAST2 sequence comparison program may be downloaded from http://www.ncbi.nlm.nih.gov or otherwise obtained from National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y .

where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

"PRO21074 variant polynucleotide" or "PRO21074 variant nucleic acid sequence" means a nucleic acid molecule which encodes an active PRO21074 polypeptide as defined below and which has at least about 80% nucleic acid sequence identity with either (a) a nucleic acid sequence which encodes residues 1 or about 24 to 1313 of the PRO21074 polypeptide shown in Figure 2 (SEQ ID NO: 2), (b) a nucleic acid sequence which encodes amino acids X to 1313 of the PRO21074 polypeptide shown in Figure 2 (SEQ ID NO: 2), wherein X is any amino acid residue from 18 to 28 of Figure 2 (SEQ ID NO: 2), or (c) a nucleic acid sequence which encodes another specifically derived fragment of the amino acid sequence shown in Figure 2 (SEQ ID NO: 2). Ordinarily, a PRO21074 variant polynucleotide will have at least 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with either (a) a nucleic acid sequence which encodes residues 1 or about 24 to 1313 of the PR021074 polypeptide shown in Figure 2 (SEQ ID NO: 2), (b) a nucleic acid sequence which encodes amino acids X to 1313 of the PRO21074 polypeptide shown in Figure 2 (SEQ ID NO: 2), wherein X is any amino acid residue from 18 to 28 of Figure 2 (SEQ ID NO: 2), or (c) a nucleic acid sequence which encodes another specifically derived fragment of the amino acid sequence shown in Figure 2 (SEQ ID NO: 2). PRO21074 polynucleotide variants do not encompass the native PRO21074 nucleotide sequence.

Ordinarily, PRO21074 variant polynucleotides are at least about 30 nucleotides in length, often at least about 60 nucleotides in length, more often at least about 90 nucleotides in length, more often at least about 120 nucleotides in length, more often at least about 150 nucleotides in length, more often at least about 180 nucleotides in length, more often at least about 210 nucleotides in length, more often at least about 240 nucleotides in length, more often at least about 270 nucleotides in length, more often at least about 300 nucleotides in length, more often at least about 450 nucleotides in length, more often at least about 600 nucleotides in length, more often at least about 900 nucleotides in length, or more.

"Percent (%) nucleic acid sequence identity" with respect to the PRO21074 polypeptide-encoding nucleic acid sequences identified herein is defined as the percentage of nucleotides in a candidate sequence that are identical with the nucleotides in a PRO21074 polypeptide-encoding nucleic acid sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % nucleic acid sequence identity values are obtained as described below by using the sequence comparison computer program ALIGN-2, wherein the complete source code for the ALIGN-2 program is provided in Table 2. The ALIGN-2 sequence comparison computer program was authored by Genentech, Inc. and the source code shown in Table 2 has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 2. The ALIGN-2 program should be compiled for use on a UNIX operating system, preferably digital UNIX V4.0D. All sequence comparison parameters are set by the ALIGN-2 program and do not vary.

For purposes herein, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$100 \text{ times the fraction } W/Z \quad .$$

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Table 1C-D demonstrate how to calculate the % nucleic acid sequence identity of the nucleic acid sequence designated "Comparison DNA" to the nucleic acid sequence designated "PRO-DNA".

Unless specifically stated otherwise, all % nucleic acid sequence identity values used herein are obtained as described above using the ALIGN-2 sequence comparison computer program. However, % nucleic acid sequence identity may also be determined using the sequence comparison program NCBI-BLAST2 (Altschul *et al., Nucleic Acids Res.* 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$100 \text{ times the fraction } W/Z$$

where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In other embodiments, PRO21074 variant polynucleotides are nucleic acid molecules that encode an active PRO21074 polypeptide and which are capable of hybridizing, preferably under stringent hybridization and wash conditions, to nucleotide sequences encoding the full-length PRO21074 polypeptide shown in Figure 2 (SEQ ID NO: 2). PRO21074 variant polypeptides may be those that are encoded by a PRO21074 variant polynucleotide.

"Isolated," when used to describe the various polypeptides disclosed herein, means polypeptide that has been identified and separated and/or recovered from a component of its natural environment. Preferably, the isolated polypeptide is free of association with all components with which it is naturally associated. Contaminant components of its natural environment are materials that would typically interfere with diagnostic or therapeutic uses for the polypeptide, and may include enzymes, hormones, and other proteinaceous or nonproteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells; since at least one component of the PRO21074 natural environment will not be present. Ordinarily, however, isolated polypeptide will be prepared by at least one purification step.

An "isolated" nucleic acid molecule encoding a PRO21074 polypeptide is a nucleic acid molecule that is identified and separated from at least one contaminant nucleic acid molecule with which it is ordinarily associated in the natural source of the PRO21074-encoding nucleic acid. Preferably, the isolated nucleic is free of association with all components with which it is naturally associated. An isolated PRO21074-encoding nucleic acid molecule is other than in the form or setting in which it is found in nature. Isolated nucleic acid molecules therefore are distinguished from the PRO21074-encoding nucleic acid molecule as it exists in natural cells. However, an isolated nucleic acid molecule encoding a PRO21074 polypeptide includes PRO21074-encoding nucleic acid molecules contained in cells that ordinarily express PRO21074 where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO21074 monoclonal antibodies (including agonist, antagonist, and neutralizing antibodies), anti-PRO21074 antibody compositions with polyepitopic specificity, single chain anti-PRO21074 antibodies, and fragments of anti-PRO21074 antibodies (see below). The term "monoclonal antibody" as used herein refers to an antibody obtained from a population of substantially homogeneous antibodies, *i.e.*, the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel *et al., Current Protocols in Molecular Biology,* Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook *et al., Molecular Cloning: A Laboratory Manual,* New York: Cold Spring Harbor Press, 1989, and include the use of washing solution and hybridization conditions (*e.g.*, temperature, ionic strength and %SDS) less stringent that those described above. An example of moderately stringent conditions is overnight incubation at 37 °C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO21074 polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i.e.*, is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of PRO21074 which retain a biological and/or an immunological activity of native or naturally-occurring PRO21074, wherein "biological" activity refers to a biological function (either inhibitory or stimulatory) caused by a native or naturally-occurring PRO21074 other than the ability to induce the production of an antibody against an antigenic epitope possessed by a native or naturally-occurring PRO21074 and an "immunological" activity refers to the ability to induce the production of an antibody against an antigenic epitope possessed by a native or naturally-occurring PRO21074. Preferred biological activities may include, for example, regulation of peptide degradation and regulation of the binding of matrix proteins.

The term "antagonist" is used in the broadest sense, and includes any molecule that partially or fully blocks, inhibits, or neutralizes a biological activity of a native PRO21074 polypeptide disclosed herein. In a similar manner, the term "agonist" is used in the broadest sense and includes any molecule that mimics a biological activity of a native PRO21074 polypeptide disclosed herein. Suitable agonist or antagonist molecules specifically include agonist or antagonist antibodies or antibody fragments, fragments or amino acid sequence variants of native PRO21074 polypeptides, peptides, small organic-molecules, etc. Methods for identifying agonists or antagonists of a PRO21074 polypeptide may comprise contacting a PRO21074 polypeptide with a candidate agonist or antagonist molecule and measuring a detectable change in one or more biological activities normally associated with the PRO21074 polypeptide.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibody fragments" comprise a portion of an intact antibody, preferably the antigen binding or variable region of the intact antibody. Examples of antibody fragments include Fab, Fab', $F(ab')_2$, and Fv fragments; diabodies; linear antibodies (Zapata *et al., Protein Eng.* 8(10): 1057-1062 [1995]); single-chain antibody molecules; and multispecific antibodies formed from antibody fragments.

Papain digestion of antibodies produces two identical antigen-binding fragments; called "Fab" fragments, each with a single antigen-binding site, and a residual "Fc" fragment, a designation reflecting the ability to crystallize readily. Pepsin treatment yields an $F(ab')_2$ fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), *e.g.,* IgG1, IgG2, IgG3, IgG4, IgA, and IgA2.

"Single-chain Fv" or "sFv" antibody fragments comprise the VH and VL domains of antibody, wherein these domains are present in a single polypeptide chain. Preferably, the Fv polypeptide further comprises a polypeptide linker between the VH and VL domains which enables the sFv to form the desired structure for antigen binding. For a review of sFv, see Pluckthun in *The Pharmacology of Monoclonal Antibodies,* vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH - VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger *et al., Proc. Natl. Acad. Sci. USA*, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody the "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The word "label" when used herein refers to a detectable compound or composition which is conjugated directly or indirectly to the antibody so as to generate a "labeled" antibody. The label may be detectable by itself (*e.g.*, radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (*e.g.*, controlled pore glass), polysaccharides (*e.g.*, agarose), polyacrylamides, polystyrene, polyvinyl alcohol and silicones. In certain embodiments, depending on the context, the solid phase can comprise the well of an assay plate; in others it is a purification column (*e.g.*, an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO21074 polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

The term "cartilaginous disorder(s)" refers to cartilage which manifests at least one pathological condition such as metabolic derangement, increased matrix proteoglycan breakdown and/or reduced proteoglycan matrix synthesis, which occurs as a result of disease or injury. Included within the scope of "cartilaginous disorders" is "degenerative cartilaginous disorders" - a collection of disorders characterized, at least in part, by degeneration or metabolic derangement of the cartilaginous connective tissues of the body, including not only the joints or related structures, including muscles, bursae (synovial membrane), tendons and fibrous tissue, but also the growth plate. In one embodiment, the term includes "articular cartilage disorders" which are characterized by disruption of the smooth articular cartilage surface and degradation of the cartilage matrix. In a mammal, "articular cartilage disorders" are further manifested by symptoms of pain, stiffness and/or limitation of motion of the affected body parts.

Included within the scope of "articular cartilage disorders" are osteoarthritis (OA) and rheumatoid arthritis (RA). OA defines not a single disorder, but the final common pathway of joint destruction resulting from multiple processes. OA is characterized by localized asymmetric destruction of the cartilage commensurate with palpable bony enlargements at the joint margins. OA typically affects the interphalangeal joints of the hands, the first carpometacarpal joint, the hips, the knees, the spine, and some joints in the midfoot, while large joints, such as the ankles, elbows and shoulders tend to be spared. OA can be associated with metabolic diseases such as hemochromatosis and alkaptonuria, developmental abnormalities such as developmental dysplasia of the hips (congenital dislocation of the hips), limb-length discrepancies, including trauma and inflammatory arthritides such as gout, septic arthritis, neuropathic arthritis. OA may also develop after extended biomechanical instability, such as results from a sports injury or obesity.

Rheumatoid arthritis (RA) is a systemic, chronic, autoimmune disorder characterized by symmetrical synovitis of the joint and typically affects small and large diarthroid joints alike. As RA progresses, symptoms may include fever, weight loss, thinning of the skin, multiorgan involvement, scleritis, corneal ulcers, the formation of subcutaneous or subperiosteal nodules and even premature death. The symptoms of RA often appears during youth and can include vasculitis, atrophy of the skin and muscle, subcutaneous nodules, lymphadenopathy, splenomegaly, leukopaenia and chronic anaemia.

Furthermore, the term "degenerative cartilaginous disorder" may include systemic lupus erythematosus and gout, amyloidosis or Felty's syndrome. Additionally, the term covers the cartilage degradation and destruction associated with psoriatic arthritis, acute inflammation (*e.g.*, yersinia arthritis, pyrophosphate arthritis, gout arthritis (arthritis urica), septic arthritis), arthritis associated with trauma, inflammatory bowel disease (*e.g.*, ulcerative colitis, Crohn's disease, regional enteritis, distal ileitis, granulomatous enteritis, regional ileitis, terminal ileitis), multiple sclerosis, diabetes (*e.g.*, insulin-dependent and non-insulin dependent), obesity, giant cell arthritis and Sjögren's syndrome.

Examples of other immune and inflammatory diseases, at least some of which may be treatable by the methods of the invention include, juvenile chronic arthritis, spondyloarthropathies, systemic sclerosis (scleroderma), idiopathic inflammatory myopathies (dermatomyositis), systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis) autoimmune inflammatory diseases (*e.g.*, allergic encephalomyelitis, multiple sclerosis, insulin-dependent diabetes mellitus, autoimmune uveoretinitis, thyrotoxicosis, autoimmune thyroid disease, pernicious anemia, autograft rejection, diabetes mellitus, immune-mediated renal disease (glomerulonephritis, tubulointerstitial nephritis)), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonia, idiopathic pulmonary fibrosis and hypersensitivity pneumonitis, transplantation associated disease including graft rejection and graft-versus-host-disease. Infectious diseases including viral diseases such as AIDS (HIV infection), hepatitis, herpes, etc., bacterial infections, fungal infections, protozoal infections, parasitic infections and respiratory syncytial virus, human imunodeficiency virus, *etc.*) and allergic disorders, such as anaphylactic hypersensitivity, asthma, allergic rhinitis, atopic dermatitis, vernal conjunctivitis, eczema, urticaria and food allergies, *etc.*

"Treatment" is an intervention performed with the intention of preventing the development or altering the pathology of a disorder. Accordingly, "treatment" refers to both therapeutic treatment and prophylactic or preventative measures, wherein the object is to prevent or slow down the progression of or lessen the severity of the targeted pathological condition or disorder. Those in need of treatment include those already with the disorder as well as those in which the disorder is to be prevented. In the treatment of a cartilaginous disorder, a therapeutic agent may directly decrease or increase the magnitude of response of a pathological component of the disorder, or render the disease more susceptible to treatment by other therapeutic agents, *e.g.,* antibodies, antifungals, anti-inflammatory agents, chemotherapeutics, *etc.*

The term "effective amount" is at least the minimum concentration of PRO21074 or antagonist thereof which causes, induces or results in either a detectable improvement or repair in damaged cartilage or provides a measurable degree of protection from continued or induced cartilage destruction (*e.g.*, retention of proteoglycans in the matrix, inhibition of proteoglycan release from the matrix, stimulation of proteoglycan synthesis). Alternatively, biological activity may be quantitated by measuring the effect of PRO21074 or antagonists thereof on cartilage cell culture and comparing cellular and tissue physiology (*e.g.*, cell growth, survival, attachment, matrix assembly, *etc.*) compared to untreated controls. Furthermore, a "therapeutically effective amount" is at least the minimum concentration (amount) of PRO21074 polypeptide or antagonist administered to a mammal which would be effective in at least attenuating a pathological symptom (*e.g.*, causing, inducing or resulting in either a detectable improvement or repair in damaged articular cartilage or causing, inducing or resulting in a measurable protection from the continued or initial cartilage destruction, improvement in range of motion, reduction in pain, *etc.*) which occurs as a result of injury or a cartilaginous disorder.

"Cartilage agent" may be a growth factor, cytokine, small molecule, antibody, piece of RNA or DNA, virus particle, peptide, or chemical having a beneficial effect upon cartilage, including peptide growth factors, catabolite antagonists, anti-inflammatory factors, and those which affect bone and/or synovium. Alternatively, "cartilage agent" may be a peptide growth factor - such as any of the fibroblast growth factors (*e.g.*, FGF-1, FGF-2,... FGF-21, *etc.*), IGFs, (I and II), TGF-βs (1-3), BMPs (1-7), or members of the epidermal growth factor family such as EGF, HB-EGF, TGF-α- which could enhance the intrinsic reparative response of cartilage, for example by altering proliferation, differentiation, migration, adhesion, or matrix production by chondrocytes. Alternatively, a "cartilage agent" may be a factor which antagonizes the catabolism of cartilage (*e.g.*; IL-1 receptor antagonist (IL-1ra), NO inhibitors, IL-1β convertase (ICE) inhibitors, factors which inhibit the activity of IL-6, IL-8, LIF, IFN-γ, TNF-α activity, tetracyclines and variants thereof, inhibitors of apoptosis, MMP inhibitors, aggrecanase inhibitors, inhibitors of serine and cysteine proteases such as cathepsins, and urokinase- or tissue-plasminogen activator (uPA or tPA). Alternatively still, "cartilage agent" includes factors which act indirectly on cartilage by affecting the underlying bone (*i.e.*, osteofactors, *e.g.*, bisphosphonates, osteoprotegerin), or the surrounding synovium (*i.e.*, synovial factors) or anti-inflammatory factors (*e.g.*, anti-TNF-α, IL1ra, IL-10, NSAIDs). For review of cartilage agent examples, please see Martel-Pelletier *et al.*, *Front. Biosci.* 4: d694-703 (1999); Hering, T.M., *Front. Biosci.* 4:d743-761 (1999).

"Standard surgical techniques" are surgical procedures which are commonly employed for therapeutic manipulations of cartilage, including: cartilage shaving, abrasion chondroplasty, laser repair, debridement, chondroplasty, microfracture with or without subchondral bone penetration, mosaicplasty, cartilage cell allografts, stem cell autografts, costal cartilage grafts, chemical stimulation, electrical stimulation, perichondral autografts, periosteal autografts, cartilage scaffolds, shee (osteoarticular) autografts or allografts, or osteotomy. These techniques are reviewed and described in better detail in Frenkel *et al.*, *Front. Bioscience* 4: d671-685 (1999).

"Chronic" adminstration refers to administration in a continuous mode as opposed to an acute mode, so as to maintain the initial therapeutic effect (activity) for an extended period of time. "Intermittent" administration is treatment that is done not consecutively without interruption, but rather is cyclic in nature.

The "pathology" of a cartilaginous disorder includes any physiological phenomena that compromise the well-being of the afflicted entity. This includes, without limitation, cartilage destruction, diminished cartilage repair, abnormal or uncontrollable cell growth or differentiation, antibody production, auto-antibody production, complement production and activation, interference with the normal functioning of neighboring cells, production of cytokines or other secretory products at abnormal levels, suppression or aggravation of any inflammatory or immunological response, infiltration of inflammatory cells (neutrophilic, eosinophilic, monocytic, lymphocytic) into tissue spaces, induction of pain, or any tissue effect which results in impairment of joint function or mobility.

"Biological activity" for the purposes herein refers to the ability of PRO21074 polypeptide or antagonists thereof to promote the regeneration of and/or prevent the destruction of cartilage. Optionally, the cartilage is articular cartilage and the regeneration and/or destruction of the cartilage is associated with an injury or a cartilaginous disorder. For example, biological activity may be quantified by measuring the effect of PRO21074 on cartilage cell culture and comparing cellular and tissue physiology (*e.g.*, cell growth, survival, attachment, matrix assembly, *etc.*) compared to untreated controls. Alternatively, biological activity may be quantified by the inhibition of proteoglycan (PG) release from cartilage, the increase in PG synthesis in cartilage, the inhibition of the production of nitric oxide (NO), *etc.*

The term "modulate" means to affect (*e.g.*, either upregulate, downregulate or otherwise control) the response of a signaling pathway. Cellular processes under the control of signal transduction include, but are not limited to, transcription of specific genes, normal cellular functions, such as metabolism, proliferation, differentiation, adhesion, apoptosis, and survival, as well as abnormal processes, such as transformation, de-differentiation and metastasis.

The term "cells in and around the joints" includes cells which could affect, cause or play a role in the formation, repair, regeneration or support of cartilage tissue. Included within the scope of this term are chondrocytes, synoviocytes, fibroblasts, cells within tendons or ligaments, osteoblasts or myoblasts.

Tables 1A-D show hypothetical exemplifications for using the below described method to determine % amino acid sequence identity (Tables 1A-B) and % nucleic acid sequence identity (Tables 1C-D) using the ALIGN-2 sequence comparison computer program, wherein "PRO" represents the amino acid sequence of a hypothetical PRO21074 polypeptide of interest, "Comparison Protein" represents the amino acid sequence of a polypeptide against which the "PRO" polypeptide of interest is being compared, "PRO-DNA" represents a hypothetical PRO21074-encoding nucleic acid sequence of interest, "Comparison DNA" represents the nucleotide sequence of a nucleic acid molecule against which the "PRO-DNA" nucleic acid molecule of interest is being compared, "X, "Y" and "Z" each represent different hypothetical amino acid residues and "N", "L" and "V" each represent different hypothetical nucleotides.

Table 1 A

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity =
(the number of identically matching amino acid residues between the two polypeptide sequences as determined by ALIGN-2) divided by (the total number of amino acid residues of the PRO polypeptide)
5 divided by 15 = 33.3%

Table 1B

| | | |
|---|---|---|
| PRO | XXXXXXXXXX | (Length =10 amino acids) |
| Comparison Protein | XXXXXYYYYYYZZYZ | (Length = 15 amino acids) |

% amino acid sequence identity =
(the number of identically matching amino acid residues between the two polypeptide sequences as determined by ALIGN-2) divided by (the total number of amino acid residues of the PRO polypeptide)
5 divided by 10 = 50%

Table 1C

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |

% nucleic acid sequence identity =
(the number of identically matching nucleotides between the two nucleic acid sequences as determined by ALIGN-2) divided by (the total number of nucleotides of the PRO-DNA nucleic acid sequence) =
6 divided by 14 = 42.9%

Table 1D

| | | |
|---|---|---|
| PRO-DNA | NNNNNNNNNNNN | (Length = 12 nucleotides) |
| Comparison DNA | NNNNLLLVV | (Length = 9 nucleotides) |

% nucleic acid sequence identity =
(the number of identically matching nucleotides between the two nucleic acid sequences as determined by ALIGN-2) divided by (the total number of nucleotides of the PRO-DNA nucleic acid sequence) =
4 divided by 12 = 33.3%

Table 2 provides the complete source code for the ALIGN-2 sequence comparison computer program. This source code may be routinely compiled for use on a UNIX operating system to provide the ALIGN-2 sequence comparison computer program.

## Table 2

```
/*
 *
 * C-C increased from 12 to 15
 * Z is average of EQ
 * B is average of ND
 * match with stop is _M; stop-stop = 0; J (joker) match = 0
 */
#define        _M      -8      /* value of a match with a stop */

int            _day[26][26] = {
/*    A B C D E F G H I J K L M N O P Q R S T U V W X Y Z */
/* A */        { 2, 0,-2, 0, 0,-4, 1,-1,-1, 0,-1,-2,-1, 0,_M, 1, 0,-2, 1, 1, 0, 0,-6, 0,-3, 0},
/* B */        { 0, 3,-4, 3, 2,-5, 0, 1,-2, 0, 0,-3,-2, 2,_M,-1, 1, 0, 0, 0, 0,-2,-5, 0,-3, 1},
/* C */        {-2,-4,15,-5,-5,-4,-3,-3,-2, 0,-5,-6,-5,-4,_M,-3,-5,-4, 0,-2, 0,-2,-8, 0, 0,-5},
/* D */        { 0, 3,-5, 4, 3,-6, 1, 1,-2, 0, 0,-4,-3, 2,_M,-1, 2,-1, 0, 0, 0,-2,-7, 0,-4, 2},
/* E */        { 0, 2,-5, 3, 4,-5, 0, 1,-2, 0, 0,-3,-2, 1,_M,-1, 2,-1, 0, 0, 0,-2,-7, 0,-4, 3},
/* F */        {-4,-5,-4,-6,-5, 9,-5,-2, 1, 0,-5, 2, 0,-4,_M,-5,-5,-4,-3,-3, 0,-1, 0, 0, 7,-5},
/* G */        { 1, 0,-3, 1, 0,-5, 5,-2,-3, 0,-2,-4,-3, 0,_M,-1,-1,-3, 1, 0, 0,-1,-7, 0,-5, 0},
/* H */        {-1, 1,-3, 1, 1,-2,-2, 6,-2, 0, 0,-2,-2, 2,_M, 0, 3, 2,-1,-1, 0,-2,-3, 0, 0, 2},
/* I */        {-1,-2,-2,-2,-2, 1,-3,-2, 5, 0,-2, 2, 2,-2,_M,-2,-2,-2,-1, 0, 0, 4,-5, 0,-1,-2},
/* J */        { 0, 0, 0, 0, 0, 0, 0, 0, 0, 0, 0, 0, 0, 0,_M, 0, 0, 0, 0, 0, 0, 0, 0, 0, 0, 0},
/* K */        {-1, 0,-5, 0, 0,-5,-2, 0,-2, 0, 5,-3, 0, 1,_M,-1, 1, 3, 0, 0, 0,-2,-3, 0,-4, 0},
/* L */        {-2,-3,-6,-4,-3, 2,-4,-2, 2, 0,-3, 6, 4,-3,_M,-3,-2,-3,-3,-1, 0, 2,-2, 0,-1,-2},
/* M */        {-1,-2,-5,-3,-2, 0,-3,-2, 2, 0, 0, 4, 6,-2,_M,-2,-1, 0,-2,-1, 0, 2,-4, 0,-2,-1},
/* N */        { 0, 2,-4, 2, 1,-4, 0, 2,-2, 0, 1,-3,-2, 2,_M,-1, 1, 0, 1, 0, 0,-2,-4, 0,-2, 1},
/* O */        {_M,_M,_M,_M,_M,_M,_M,_M,_M,_M,_M,_M,_M,_M, 0,_M,_M,_M,_M,_M,_M,_M,_M,_M,_M,_M},
/* P */        { 1,-1,-3,-1,-1,-5,-1, 0,-2, 0,-1,-3,-2,-1,_M, 6, 0, 0, 1, 0, 0,-1,-6, 0,-5, 0},
/* Q */        { 0, 1,-5, 2, 2,-5,-1, 3,-2, 0, 1,-2,-1, 1,_M, 0, 4, 1,-1,-1, 0,-2,-5, 0,-4, 3},
/* R */        {-2, 0,-4,-1,-1,-4,-3, 2,-2, 0, 3,-3, 0, 0,_M, 0, 1, 6, 0,-1, 0,-2, 2, 0,-4, 0},
/* S */        { 1, 0, 0, 0, 0,-3, 1,-1,-1, 0, 0,-3,-2, 1,_M, 1,-1, 0, 2, 1, 0,-1,-2, 0,-3, 0},
/* T */        { 1, 0,-2, 0, 0,-3, 0,-1, 0, 0, 0,-1,-1, 0,_M, 0,-1;-1, 1, 3, 0, 0,-5, 0,-3, 0},
/* U */        { 0, 0, 0, 0, 0, 0, 0, 0, 0, 0, 0, 0, 0, 0,_M, 0, 0, 0, 0, 0, 0, 0, 0, 0, 0, 0},
/* V */        { 0,-2,-2,-2,-2,-1,-1,-2, 4, 0,-2, 2, 2,-2,_M,-1,-2,-2,-1, 0, 0, 4,-6, 0,-2,-2},
/* W */        {-6,-5,-8,-7,-7, 0,-7,-3,-5, 0,-3,-2,-4,-4,_M,-6,-5, 2,-2,-5, 0,-6,17, 0, 0,-6},
/* X */        { 0, 0, 0, 0, 0, 0, 0, 0, 0, 0, 0, 0, 0, 0,_M, 0, 0, 0, 0, 0, 0, 0, 0, 0, 0, 0},
/* Y */        {-3,-3, 0,-4,-4, 7,-5, 0,-1, 0,-4,-1,-2,-2,_M,-5,-4,-4,-3,-3, 0,-2, 0, 0,10,-4},
/* Z */        { 0, 1,-5, 2, 3,-5, 0, 2,-2, 0, 0,-2,-1, 1,_M, 0, 3, 0, 0, 0, 0,-2,-6, 0,-4, 4}
};
```

## Table 2 (cont.')

```
/*
 */
#include <stdio.h>
#include <ctype.h>

#define     MAXJMP 16      /* max jumps in a diag */
#define     MAXGAP 24      /* don't continue to penalize gaps larger than this */
#define     JMPS           1024    /* max jmps in an path */
#define     MX             4       /* save if there's at least MX-1 bases since last jmp */

#define     DMAT           3       /* value of matching bases */
#define     DMIS           0       /* penalty for mismatched bases */
#define     DINS0          8       /* penalty for a gap */
#define     DINS1          1       /* penalty per base */
#define     PINS0          8       /* penalty for a gap */
#define     PINS1          4       /* penalty per residue */

struct jmp {
            short           n[MAXJMP];      /* size of jmp (neg for dely) */
            unsigned short  x[MAXJMP];      /* base no. of jmp in seq x */
                                    /* limits seq to 2^16 -1 */
};

struct diag {
            int             score;          /* score at last jmp */
            long            offset;         /* offset of prev block */
            short           ijmp;           /* current jmp index */
            struct jmp jp;          /* list of jmps */
};

struct path {
            int     spc;            /* number of leading spaces */
            short   n[JMPS]; /* size of jmp (gap) */
            int     x[JMPS]; /* loc of jmp (last elem before gap) */
};

char        *ofile;                 /* output file name */
char        *namex[2];              /* seq names: getseqs() */
char        *prog;                  /* prog name for err msgs */
char        *seqx[2];               /* seqs: getseqs() */
int         dmax;                   /* best diag: nw() */
int         dmax0;                  /* final diag */
int         dna;                    /* set if dna: main() */
int         endgaps;                /* set if penalizing end gaps */
int         gapx, gapy;             /* total gaps in seqs */
int         len0, len1;             /* seq lens */
int         ngapx, ngapy;           /* total size of gaps */
int         smax;                   /* max score: nw() */
int         *xbm;                   /* bitmap for matching */
long        offset;                 /* current offset in jmp file */
struct      diag    *dx;                    /* holds diagonals */
struct      path    pp[2];                  /* holds path for seqs */

char        *calloc(), *malloc(), *index(), *strcpy();
char        *getseq(), *g_calloc();
```

## Table 2 (cont.')

```
/* Needleman-Wunsch alignment program
 *
 * usage: progs file1 file2
 *   where file1 and file2 are two dna or two protein sequences.
 *   The sequences can be in upper- or lower-case an may contain ambiguity
 *   Any lines beginning with ';', '>' or '<' are ignored
 *   Max file length is 65535 (limited by unsigned short x in the jmp struct)
 *   A sequence with 1/3 or more of its elements ACGTU is assumed to be DNA
 *   Output is in the file "align.out"
 *
 * The program may create a tmp file in /tmp to hold info about traceback.
 * Original version developed under BSD 4.3 on a vax 8650
 */
#include "nw.h"
#include "day.h"

static          _dbval[26] = {
                1,14,2,13,0,0,4,11,0,0,12,0,3,15,0,0,0,5,6,8,8,7,9,0,10,0
};

static          _pbval[26] = {
                1, 2|(1<<('D'-'A'))|(1<<('N'-'A')), 4, 8, 16, 32, 64,
                128, 256, 0xFFFFFFF, 1<<10, 1<<11, 1<<12, 1<<13, 1<<14,
                1<<15, 1<<16, 1<<17, 1<<18, 1<<19, 1<<20, 1<<21, 1<<22,
                1<<23, 1<<24, 1<<25|(1<<('E'-'A'))|(1<<('Q'-'A'))
};

main(ac, av)                                                          main
                int       ac;
                char      *av[];
{
                prog = av[0];
                if (ac != 3) {
                  fprintf(stderr,"usage: %s file1 file2\n", prog);
                  fprintf(stderr,"where file1 and file2 are two dna or two protein sequences.\n");
                  fprintf(stderr,"The sequences can be in upper- or lower-case\n");
                  fprintf(stderr,"Any lines beginning with ';' or '<' are ignored\n");
                  fprintf(stderr,"Output is in the file \"align.out\"\n");
                  exit(1);
                }
                namex[0] = av[1];
                namex[1] = av[2];
                seqx[0] = getseq(namex[0], &len0);
                seqx[1] = getseq(namex[1], &len1);
                xbm = (dna)? _dbval : _pbval;

                endgaps = 0;                    /* 1 to penalize endgaps */
                ofile = "align.out";            /* output file */

                nw();           /* fill in the matrix, get the possible jmps */
                readjmps();     /* get the actual jmps */
                print();        /* print stats, alignment */

                cleanup(0);     /* unlink any tmp files */
}
```

## Table 2 (cont.')

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()                                                              nw
{
        char            *px, *py;       /* seqs and ptrs */
        int             *ndely, *dely;  /* keep track of dely */
        int             ndelx, delx;    /* keep track of delx */
        int             *tmp;           /* for swapping row0, row1 */
        int             mis;            /* score for each type */
        int             ins0, ins1;     /* insertion penalties */
        register        id;             /* diagonal index */
        register        ij;             /* jmp index */
        register        *col0, *col1;   /* score for curr, last row */
        register        xx, yy;         /* index into seqs */

        dx = (struct diag *)g_calloc("to get diags", len0+len1+1, sizeof(struct diag));

        ndely = (int *)g_calloc("to get ndely", len1+1, sizeof(int));
        dely = (int *)g_calloc("to get dely", len1+1, sizeof(int));
        col0 = (int *)g_calloc("to get col0", len1+1, sizeof(int));
        col1 = (int *)g_calloc("to get col1", len1+1, sizeof(int));
        ins0 = (dna)? DINS0 : PINS0;
        ins1 = (dna)? DINS1 : PINS1;

        smax = -10000;
        if (endgaps) {
         for (col0[0] = dely[0] = -ins0, yy = 1; yy <= len1; yy++) {
                col0[yy] = dely[yy] = col0[yy-1] - ins1;
                ndely[yy] = yy;
         }
         col0[0] = 0;           /* Waterman Bull Math Biol 84 */
        }
        else
         for (yy = 1; yy <= len1; yy++)
                dely[yy] = -ins0;

        /* fill in match matrix
         */
        for (px = seqx[0], xx = 1; xx <= len0; px++, xx++) {
         /* initialize first entry in col
          */
         if (endgaps) {
                if (xx == 1)
                        col1[0] = delx = -(ins0+ins1);
                else
                        col1[0] = delx = col0[0] - ins1;
                ndelx = xx;
         }
         else {
                col1[0] = 0;
                delx = -ins0;
                ndelx = 0;
         }
```

## Table 2 (cont.')

```
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];

        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
                } else
                        ndely[yy]++;
        }

        /* update penalty for del in y seq;
         * favor new del over ongong del
         */
        if (endgaps || ndelx < MAXGAP) {
                if (col1[yy-1] - ins0 >= delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) >= delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

**Table 2 (cont.')**

```
                        id = xx - yy + len1 - 1;
                        if (mis >= delx && mis >= dely[yy])
                                col1[yy] = mis;
                        else if (delx >= dely[yy]) {
                                col1[yy] = delx;
                                ij = dx[id].ijmp;
                                if (dx[id].jp.n[0] && (!dna || (ndelx >= MAXJMP
                                && xx > dx[id].jp.x[ij]+MX) || mis > dx[id].score+DINS0)) {
                                        dx[id].ijmp++;
                                        if (++ij >= MAXJMP) {
                                                writejmps(id);
                                                ij = dx[id].ijmp = 0;
                                                dx[id].offset = offset;
                                                offset += sizeof(struct jmp) + sizeof(offset);
                                        }
                                }
                                dx[id].jp.n[ij] = ndelx;
                                dx[id].jp.x[ij] = xx;
                                dx[id].score = delx;
                        }
                        else {
                                col1[yy] = dely[yy];
                                ij = dx[id].ijmp;
                if (dx[id].jp.n[0] && (!dna || (ndely[yy] >= MAXJMP
                                && xx > dx[id].jp.x[ij]+MX) || mis > dx[id].score+DINS0)) {
                                        dx[id].ijmp++;
                                        if (++ij >= MAXJMP) {
                                                writejmps(id);
                                                ij = dx[id].ijmp = 0;
                                                dx[id].offset = offset;
                                                offset += sizeof(struct jmp) + sizeof(offset);
                                        }
                                }
                                dx[id].jp.n[ij] = -ndely[yy];
                                dx[id].jp.x[ij] = xx;
                                dx[id].score = dely[yy];
                        }
                        if (xx == len0 && yy < len1) {
                                /* last col
                                 */
                                if (endgaps)
                                        col1[yy] -= ins0+ins1*(len1-yy);
                                if (col1[yy] > smax) {
                                        smax = col1[yy];
                                        dmax = id;
                                }
                        }
                }
                if (endgaps && xx < len0)
                        col1[yy-1] -= ins0+ins1*(len0-xx);
                if (col1[yy-1] > smax) {
                        smax = col1[yy-1];
                        dmax = id;
                }
                tmp = col0; col0 = col1; col1 = tmp;
        }
        (void) free((char *)ndely);
        (void) free((char *)dely);
        (void) free((char *)col0);
        (void) free((char *)col1);
}
```

## Table 2 (cont.')

```
/*
 *
 * print() -- only routine visible outside this module
 *
 * static:
 * getmat() -- trace back best path, count matches: print()
 * pr_align() -- print alignment of described in array p[]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */

#include "nw.h"

#define SPC      3
#define P_LINE   256        /* maximum output line */
#define P_SPC    3          /* space between name or num and seq */

extern          _day[26][26];
int             olen;             /* set output line length */
FILE            *fx;              /* output file */

print()                                                              print
{
                int      lx, ly, firstgap, lastgap;    /* overlap */

                if ((fx = fopen(ofile, "w")) == 0) {
                  fprintf(stderr,"%s: can't write %s\n", prog, ofile);
                  cleanup(1);
                }
                fprintf(fx, "<first sequence: %s (length = %d)\n", namex[0], len0);
                fprintf(fx, "<second sequence: %s (length = %d)\n", namex[1], len1);
                olen = 60;
                lx = len0;
                ly = len1;
                firstgap = lastgap = 0;
                if (dmax < len1 - 1) {          /* leading gap in x */
                  pp[0].spc = firstgap = len1 - dmax - 1;
                  ly -= pp[0].spc;
                }
                else if (dmax > len1 - 1) {     /* leading gap in y */
                  pp[1].spc = firstgap = dmax - (len1 - 1);
                  lx -= pp[1].spc;
                }
                if (dmax0 < len0 - 1) {         /* trailing gap in x */
                  lastgap = len0 - dmax0 -1;
                  lx -= lastgap;
                }
                else if (dmax0 > len0 - 1) {    /* trailing gap in y */
                  lastgap = dmax0 - (len0 - 1);
                  ly -= lastgap;
                }
                getmat(lx, ly, firstgap, lastgap);
                pr_align();
}
```

## Table 2 (cont.')

getmat

```
/*
 * trace back the best path, count matches
 */
static
getmat(lx, ly, firstgap, lastgap)
                    int       lx, ly;                  /* "core" (minus endgaps) */
                    int       firstgap, lastgap;       /* leading trailing overlap */
{
                    int              nm, i0, i1, siz0, siz1;
                    char             outx[32];
                    double           pct;
                    register         n0, n1;
                    register char    *p0, *p1;

                    /* get total matches, score
                     */
                    i0 = i1 = siz0 = siz1 = 0;
                    p0 = seqx[0] + pp[1].spc;
                    p1 = seqx[1] + pp[0].spc;
                    n0 = pp[1].spc + 1;
                    n1 = pp[0].spc + 1;

                    nm = 0;
                    while ( *p0 && *p1 ) {
                     if (siz0) {
                              p1++;
                              n1++;
                              siz0--;
                     }
                     else if (siz1) {
                              p0++;
                              n0++;
                              siz1--;
                     }
                     else {
                              if (xbm[*p0-'A']&xbm[*p1-'A'])
                                        nm++;
                              if (n0++ == pp[0].x[i0])
                                        siz0 = pp[0].n[i0++];
                              if (n1++ == pp[1].x[i1])
                                        siz1 = pp[1].n[i1++];
                              p0++;
                              p1++;
                     }
                    }

                    /* pct homology:
                     * if penalizing endgaps, base is the shorter seq
                     * else, knock off overhangs and take shorter core
                     */
                    if (endgaps)
                     lx = (len0 < len1)? len0 : len1;
                    else
                     lx = (lx < ly)? lx : ly;
                    pct = 100.*(double)nm/(double)lx;
                    fprintf(fx, "\n");
                    fprintf(fx, "<%d match%s in an overlap of %d: %.2f percent similarity\n",
                     nm, (nm == 1)? "" : "es", lx, pct);
```

**Table 2 (cont.')**

```
fprintf(fx, "<gaps in first sequence: %d", gapx);
if (gapx) {
 (void) sprintf(outx, " (%d %s%s)",
          ngapx, (dna)? "base":"residue", (ngapx == 1)? "":"s");
 fprintf(fx,"%s", outx);
fprintf(fx, ", gaps in second sequence: %d", gapy);
if (gapy) {
 (void) sprintf(outx, " (%d %s%s)",
          ngapy, (dna)? "base":"residue", (ngapy == 1)? "":"s");
 fprintf(fx,"%s", outx);
}
if (dna)
 fprintf(fx,
 "\n<score: %d (match = %d, mismatch = %d, gap penalty = %d + %d per base)\n",
 smax, DMAT, DMIS, DINS0, DINS1);
else
 fprintf(fx,
 "\n<score: %d (Dayhoff PAM 250 matrix, gap penalty = %d + %d per residue)\n",
 smax, PINS0, PINS1);
if (endgaps)
 fprintf(fx,
 "<endgaps penalized. left endgap: %d %s%s, right endgap: %d %s%s\n",
 firstgap, (dna)? "base" : "residue", (firstgap == 1)? "" : "s",
 lastgap, (dna)? "base" : "residue", (lastgap == 1)? "" : "s");
else
 fprintf(fx, "<endgaps not penalized\n");
}

static        nm;              /* matches in core -- for checking */
static        lmax;            /* lengths of stripped file names */
static        ij[2];           /* jmp index for a path */
static        nc[2];           /* number at start of current line */
static        ni[2];           /* current elem number -- for gapping */
static        siz[2];
static char   *ps[2];          /* ptr to current element */
static char   *po[2];          /* ptr to next output char slot */
static char   out[2][P_LINE];  /* output line */
static char   star[P_LINE];    /* set by stars() */

/*
 * print alignment of described in struct path pp[]
 */
static
pr_align()                                                  pr_align
{
        int           nn;       /* char count */
        int           more;
        register      i;

        for (i = 0, lmax = 0; i < 2; i++) {
         nn = stripname(namex[i]);
         if (nn > lmax)
                  lmax = nn;

         nc[i] = 1;
         ni[i] = 1;
         siz[i] = ij[i] = 0;
         ps[i] = seqx[i];
         po[i] = out[i];
        }
```

## Table 2 (cont.')

```
for (nn = nm = 0, more = 1; more; ) {
 for (i = more = 0; i < 2; i++) {
	/*
	 * do we have more of this sequence?
	 */
	if (!*ps[i])
		continue;

	more++;

	if (pp[i].spc) {	/* leading space */
		*po[i]++ = ' ';
		pp[i].spc--;
	}
	else if (siz[i]) {	/* in a gap */
		*po[i]++ = '-';
		siz[i]--;
	}
	else {			/* we're putting a seq element
				 */
		*po[i] = *ps[i];
		if (islower(*ps[i]))
			*ps[i] = toupper(*ps[i]);
		po[i]++;
		ps[i]++;

		/*
		 * are we at next gap for this seq?
		 */
		if (ni[i] == pp[i].x[ij[i]]) {
			/*
			 * we need to merge all gaps
			 * at this location
			 */
			siz[i] = pp[i].n[ij[i]++];
			while (ni[i] == pp[i].x[ij[i]])
				siz[i] += pp[i].n[ij[i]++];
		}
		ni[i]++;
	}
 }
 if (++nn == olen || !more && nn) {
	dumpblock();
	for (i = 0; i < 2; i++)
		po[i] = out[i];
	nn = 0;
 }
}
}
/*
 * dump a block of lines, including numbers, stars: pr_align()
 */
static
dumpblock()
{
	register	i;

	for (i = 0; i < 2; i++)
	 *po[i]-- = '\0';
```

**dumpblock**

## Table 2 (cont.')

```
                (void) putc('\n', fx);
                for (i = 0; i < 2; i++) {
                 if (*out[i] && (*out[i] != ' ' || *(po[i]) != ' ')) {
                        if (i == 0)
                                nums(i);
                        if (i == 0 && *out[1])
                                stars();
                        putline(i);
                        if (i == 0 && *out[1])
                                fprintf(fx, star);
                        if (i == 1)
                                nums(i);
                 }
                }
}
/*
 * put out a number line: dumpblock()
 */
static
nums(ix)                                                                nums
                int             ix;             /* index in out[] holding seq line */
{
                char                    nline[P_LINE];
                register                i, j;
                register char           *pn, *px, *py;

                for (pn = nline, i = 0; i < lmax+P_SPC; i++, pn++)
                 *pn = ' ';
                for (i = nc[ix], py = out[ix]; *py; py++, pn++) {
                 if (*py == ' ' || *py == '-')
                        *pn = ' ';
                 else {
                        if (i%10 == 0 || (i == 1 && nc[ix] != 1)) {
                                j = (i < 0)? -i : i;
                                for (px = pn; j; j /= 10, px--)
                                        *px = j%10 + '0';
                                if (i < 0)
                                        *px = '-';
                        }
                        else
                                *pn = ' ';
                        i++;
                 }
                }
                *pn = '\0';
                nc[ix] = i;
                for (pn = nline; *pn; pn++)
                 (void) putc(*pn, fx);
                (void) putc('\n', fx);
}

/*
 * put out a line (name, [num], seq, [num]): dumpblock()
 */
static
putline(ix)                                                             putline
                int             ix;
{
```

## Table 2 (cont.')

```
            int                 i;
            register char       *px;

            for (px = namex[ix], i = 0; *px && *px != ':'; px++, i++)
             (void) putc(*px, fx);
            for (; i < lmax+P_SPC; i++)
             (void) putc(' ', fx);

            /* these count from 1:
             * ni[] is current element (from 1)
             * nc[] is number at start of current line
             */
            for (px = out[ix]; *px; px++)
             (void) putc(*px&0x7F, fx);
            (void) putc('\n', fx);
}


/*
 * put a line of stars (seqs always in out[0], out[1]): dumpblock()
 */
static
stars()                                                              stars
{
            int                 i;
            register char       *p0, *p1, cx, *px;

            if (!*out[0] || (*out[0] == ' ' && *(po[0]) == ' ') ||
               !*out[1] || (*out[1] == ' ' && *(po[1]) == ' '))
             return;
            px = star;
            for (i = lmax+P_SPC; i; i--)
             *px++ = ' ';

            for (p0 = out[0], p1 = out[1]; *p0 && *p1; p0++, p1++) {
             if (isalpha(*p0) && isalpha(*p1)) {

                      if (xbm[*p0-'A']&xbm[*p1-'A']) {
                               cx = '*';
                               nm++;
                      }
                      else if (!dna && _day[*p0-'A'][*p1-'A'] > 0)
                               cx = '.';
                      else
                               cx = ' ';
             }
             else
                      cx = ' ';
             *px++ = cx;
            }
            *px++ = '\n';
            *px = '\0';
}
```

## Table 2 (cont.')

**stripname**

```
/*
 * strip path or prefix from pn, return len: pr_align()
 */
static
stripname(pn)
                char        *pn;        /* file name (may be path) */
{
                register char       *px, *py;

                py = 0;
                for (px = pn; *px; px++)
                  if (*px == '/')
                          py = px + 1;
                if (py)
                  (void) strcpy(pn, py);
                return(strlen(pn));
}
```

## Table 2 (cont.')

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>

char        *jname = "/tmp/homgXXXXXX";        /* tmp file for jmps */
FILE        *fj;

int         cleanup();                         /* cleanup tmp file */
long        lseek();

/*
 * remove any tmp file if we blow
 */
cleanup(i)                                                          cleanup
            int     i;
{
            if (fj)
             (void) unlink(jname);
            exit(i);
}

/*
 * read, return ptr to seq, set dna, len, maxlen
 * skip lines starting with ';', '<', or '>'
 * seq in upper or lower case
 */
char        *
getseq(file, len)                                                   getseq
            char    *file;    /* file name */
            int     *len;     /* seq len */
{
            char              line[1024], *pseq;
            register char     *px, *py;
            int               natgc, tlen;
            FILE              *fp;

            if ((fp = fopen(file,"r")) == 0) {
             fprintf(stderr,"%s: can't read %s\n", prog, file);
             exit(1);
            }
            tlen = natgc = 0;
            while (fgets(line, 1024, fp)) {
             if (*line == ';' || *line == '<' || *line == '>')
                    continue;
             for (px = line; *px != '\n'; px++)
                    if (isupper(*px) || islower(*px))
                           tlen++;
            }
            if ((pseq = malloc((unsigned)(tlen+6))) == 0) {
             fprintf(stderr,"%s: malloc() failed to get %d bytes for %s\n", prog, tlen+6, file);
             exit(1);
            }
            pseq[0] = pseq[1] = pseq[2] = pseq[3] = '\0';
```

## Table 2 (cont.')

```
		py = pseq + 4;
		*len = tlen;
		rewind(fp);

		while (fgets(line, 1024, fp)) {
		 if (*line == ';' || *line == '<' || *line == '>')
				continue;
		 for (px = line; *px != '\n'; px++) {
				if (isupper(*px))
					*py++ = *px;
				else if (islower(*px))
					*py++ = toupper(*px);
				if (index("ATGCU",*(py-1)))
					natgc++;
		 }
		}
		*py++ = '\0';
		*py = '\0';
		(void) fclose(fp);
		dna = natgc > (tlen/3);
		return(pseq+4);
}

char		*
g_calloc(msg, nx, sz)
		char	*msg;		/* program, calling routine */
		int	nx, sz;		/* number and size of elements */
{
		char		*px, *calloc();

		if ((px = calloc((unsigned)nx, (unsigned)sz)) == 0) {
		 if (*msg) {
				fprintf(stderr, "%s: g_calloc() failed %s (n=%d, sz=%d)\n", prog, msg, nx, sz);
				exit(1);
		 }
		}
		return(px);
}

/*
 * get final jmps from dx[] or tmp file, set pp[], reset dmax: main()
 */
readjmps()
{
		int		fd = -1;
		int		siz, i0, i1;
		register	i, j, xx;

		if (fj) {
		 (void) fclose(fj);
		 if ((fd = open(jname, O_RDONLY, 0)) < 0) {
				fprintf(stderr, "%s: can't open() %s\n", prog, jname);
				cleanup(1);
		 }
		}
		for (i = i0 = i1 = 0, dmax0 = dmax, xx = len0; ; i++) {
		 while (1) {
				for (j = dx[dmax].ijmp; j >= 0 && dx[dmax].jp.x[j] >= xx; j--)
					;
```

**g_calloc**

**readjmps**

## Table 2 (cont.')

```
                    if (j < 0 && dx[dmax].offset && fj) {
                            (void) lseek(fd, dx[dmax].offset, 0);
                            (void) read(fd, (char *)&dx[dmax].jp, sizeof(struct jmp));
                            (void) read(fd, (char *)&dx[dmax].offset, sizeof(dx[dmax].offset));
                            dx[dmax].ijmp = MAXJMP-1;
                    }
                    else
                            break;
            }
            if (i >= JMPS) {
                    fprintf(stderr, "%s: too many gaps in alignment\n", prog);
                    cleanup(1);
            }
            if (j >= 0) {
                    siz = dx[dmax].jp.n[j];
                    xx = dx[dmax].jp.x[j];
                    dmax += siz;
                    if (siz < 0) {                  /* gap in second seq */
                            pp[1].n[i1] = -siz;
                            xx += siz;
                            /* id = xx - yy + len1 - 1
                             */
                            pp[1].x[i1] = xx - dmax + len1 - 1;
                            gapy++;
                            ngapy -= siz;
/* ignore MAXGAP when doing endgaps */
                            siz = (-siz < MAXGAP || endgaps)? -siz : MAXGAP;
                            i1++;
                    }
                    else if (siz > 0) {     /* gap in first seq */
                            pp[0].n[i0] = siz;
                            pp[0].x[i0] = xx;
                            gapx++;
                            ngapx += siz;
/* ignore MAXGAP when doing endgaps */
                            siz = (siz < MAXGAP || endgaps)? siz : MAXGAP;
                            i0++;
                    }
            }
            else
                    break;
    }
    /* reverse the order of jmps
     */
    for (j = 0, i0--; j < i0; j++, i0--) {
      i = pp[0].n[j]; pp[0].n[j] = pp[0].n[i0]; pp[0].n[i0] = i;
      i = pp[0].x[j]; pp[0].x[j] = pp[0].x[i0]; pp[0].x[i0] = i;
    }
    for (j = 0, i1--; j < i1; j++, i1--) {
      i = pp[1].n[j]; pp[1].n[j] = pp[1].n[i1]; pp[1].n[i1] = i;
      i = pp[1].x[j]; pp[1].x[j] = pp[1].x[i1]; pp[1].x[i1] = i;
    }
    if (fd >= 0)
      (void) close(fd);
    if (fj) {
      (void) unlink(jname);
      fj = 0;
      offset = 0;
    }
}
```

Table 2 (cont.')

```
/*
 * write a filled jmp struct offset of the prev one (if any): nw()
 */
writejmps(ix)                                                    writejmps
        int     ix;
{
        char    *mktemp();

        if (!fj) {
         if (mktemp(jname) < 0) {
                fprintf(stderr, "%s: can't mktemp() %s\n", prog, jname);
                cleanup(1);
         }
         if ((fj = fopen(jname, "w")) == 0) {
                fprintf(stderr, "%s: can't write %s\n", prog, jname);
                exit(1);
         }
        }
        (void) fwrite((char *)&dx[ix].jp, sizeof(struct jmp), 1, fj);
        (void) fwrite((char *)&dx[ix].offset, sizeof(dx[ix].offset), 1, fj);
}
```

## II. Detailed Description of the Invention

*Osteoarthris v. Rheumatoid arthritis:*

Rheumatoid arthritis (RA) is a systemic, autoimmune, degenerative disease that causes disruptions in the synovium of both large and small diarthroidal joints alike. As the disease progresses, symptoms of RA may include fever, weight loss, thinning of the skin, multiorgan involvement, scleritis, corneal ulcers, the formation of subcutaneous or subperiosteal nodules and premature death. In contrast to OA, RA symptoms appear during youth, extra-articular manifestations can affect any organ system, and joint destruction is symmetrical and occurs in both large and small joints alike. Extra-articular symptoms can include vasculitis, atrophy of the skin and muscle, subcutaneous nodules, lymphadenopathy, splenomegaly, leukopaenia and chronic anaemia. Furthermore, RA is heterogeneous in nature with a variable disease expression and is associated with the formation of serum rheumatoid factor in 90% of patients sometime during the course of the illness.

Interestingly, patients with RA also have a hyperactive immune system. The great majority of people with RA have a genetic susceptibility associated with increased activation of class II major histocompatibility complex molecules on monocytes and macrophages. These histocompatibility complex molecules are involved in the presentation of antigen to activated T cells bearing receptors for these class II molecules. The genetic predisposition to RA is supported by the prevalence of the highly conserved leukocyte antigen DR subtype Dw4, Dw14 and Dw15 in human patients with very severe disease.

The activated monocytes and macrophages, in interacting with the appropriate T cells, stimulate a cascade of events including further activation of additional monocytes and macrophages, T cells, B cells and endothelial cells. With the upregulation of adhesion molecules, additional mononuclear cells and polymorphonuclear cells are attracted to the inflamed joint. This influx stimulates secretion of additional chemotactic cytokines, thereby enhancing the influx of inflammatory cells into the synovium and synovial fluid.

Osteoarthritis (OA) is a localized degenerative disease that affects articular cartilage and bone and results in pain and diminished joint function. OA may be classified into two types: primary and secondary. Primary OA refers to the spectrum of degenerative joint diseases for which no underlying etiology has been determined. Typically, the joint affected by primary OA are the interphalangeal joints of the hands, the first carpometacarpal joints, the hips, the knees, the spine, and some joints in the midfoot. Interestingly, it appears that large joints, such as the ankles, elbows and shoulders tend to be spared in primary OA. In contrast, secondary OA often occurs as a result of defined injury or trauma. Secondary arthritis can also be found in individuals with metabolic diseases such as hemochromatosis and alkaptonuria, developmental abnormalities such as developmental dysplasia of the hips (congenital dislocation of the hips) and limb-length discrepancies, obesity, inflammatory arthritis such as rheumatoid arthritis or gout, septic arthritis, and neuropathic arthritis.

OA is a progressive, degenerative disorder. The degradation associated with OA initially appears as fraying and fibrillation of the articular cartilage surface as proteoglycans are lost from the matrix. With continued joint use, surface fibrillation progresses, defects penetrate deeper into the cartilage, and pieces of cartilage tissue are lost. In addition, bone underlying the cartilage (subchondral bone) thickens, and, as cartilage is lost, bone becomes slowly exposed. With asymmetric cartilage destruction, disfigurement can occur. Bony nodules, called osteophytes, often form at the periphery of the cartilage surface and occasionally grow over the adjacent eroded areas. If the surface of these bony outgrowths is permeated, vascular outgrowth may occur and cause the formation of tissue plugs containing fibrocartilage.

Since cartilage is avascular, damage which occurs to the cartilage layer but does not penetrate to the subchondral bone, leaves the job of repair to the resident chondrocytes, which have little intrinsic potential for replication. However, when the subchondral bone is penetrated, its vascular supply allows a triphasic repair process to take place. The suboptimal cartilage which is synthesized in response to this type of damage, termed herein "fibrocartilage" because of its fibrous matrix, has suboptimal biochemical and mechanical properties, and is thus subject to further wear and destruction. In a diseased or damaged joint, increased release of metalloproteinases (MMPs) such as collagenases, gelatinases, stromelysins, aggrecanases, and other proteases, leads to further thinning and loss of cartilage. *In vitro* studies have shown that cytokines such as IL-1α, IL-1β, TNF-α, PDGF, GM-CSF, IFN-γ, TGF-β, LIF, IL-2 and IL-6, IL-8 can alter the activity of synovial fibroblast-like cells, macrophage, T cells, and/or osteoclasts, and these cytokines may thus have indirect effects on cartilage matrix turnover *in vivo.* As such, any of these cytokines could amplify and perpetuate the destructive cycle of joint degeneration *in vivo.* In fact, inhibition of IL-1 or TNF-α activity in arthritic animals and humans has been shown to be an effective way in which to at least slow the progression of arthritis. While the initiating events in RA and OA are clearly different, subsequent cartilage and bone loss in these two degenerative disorders appears to involve many of the same cytokines and proteinases.

The mechanical properties of cartilage are determined by its biochemical composition. While the collagen architecture contributes to the tensile strength and stiffness of cartilage, the compressibility (or elasticity) is due to its proteoglycan component. In healthy articular cartilage, type II collagen predominates (comprising about 90-95%), however, smaller amounts of types V, VI, IX, and XI collagen are also present. Cartilage proteoglycans (PG) include hydrodynamically large, aggregating PG, with covalently linked sulfated glycosaminoglycans, as well as hydrodynamically smaller nonaggregating PG such as decorin, biglycan and lumican.

*Types of injuries to cartilage*

Injuries to cartilage fall into three categories: (1) microdamage or blunt trauma, (2) chondral fractures, and (3) osteochondral fractures.

Microdamage to chondrocytes and cartilage matrix may be caused by a single impact, through repetitive blunt trauma, or with continuous use of a biomechanically unstable joint. In fact, metabolic and biochemical changes such as those found in the early stages of degenerative arthritis can be replicated in animal models by repetitive loading of articular cartilage. Radin *et al., Clin. Orthop. Relat. Res.* 131: 288-93 (1978). Such experiments, along with the distinct pattern of cartilage loss found in arthritic joints, highlight the role that biomechanical loading plays in the loss of homeostasis and integrity of articular cartilage in disease. Radin *et al., J. Orthop. Res.* 2: 221-234 (1984); Radin *et al., Semin. Arthritis. Rheum.* (suppl. 2) 21:12-21 (1991); Wei *et al., Acta Orthop. Scand.* 69: 351-357 (1998). While chondrocytes may initially be able to replenish cartilage matrix with proteoglycans at a basal rate, concurrent damage to the collagen network may increase the rate of loss and result in irreversible degeneration. Buckwalter *et al., J. Am. Acad. Orthop. Surg.* 2: 192-201 (1994).

Chondral fractures are characterized by disruption of the articular surface without violation of the subchondral plate. Chondrocyte necrosis at the injury site occurs, followed by increased mitotic and metabolic activity of the surviving chondrocytes bordering the injury which leads to lining of the clefts of the articular surface with fibrous tissue. The increase in chondrocyte activity is transitory, and the repair response results in insufficient amount and quality of new matrix components.

Osteochondral fractures, the most serious of the three types of injuries, are lesions crossing the tidemark into the underlying subchondral plate. In this type of injury, the presence of subchondral vasculature elicits the three-phase response typically encountered in vascular tissues: (1) necrosis, (2) inflammation, and (3) repair. Initially the lesion fills with blood and clots. The resulting fibrin clot activates an inflammatory response and becomes vascularized repair tissue, and the various cellular components release growth factors and cytokines including transforming growth factor beta (TGF-beta), platelet-derived growth factor (PDGF), bone morphogenic proteins, and insulin-like growth factors I and II. Buckwalter *et al., J. Am. Acad. Orthop. Surg.* 2:191-201 (1994).

The initial repair response associated with osteochondral fractures is characterized by recruitment, proliferation and differentiation of precursors into chondrocytes. Mesenchymal stem cells are deposited in the fibrin network, which eventually becomes a fibrocartilaginous zone. F. Shapiro *et al., J. Bone Joint Surg.* 75:532-53 (1993); N. Mitchell and N. Shepard, *J. Bone Joint Surg.* 58:230-33 (1976). These stem cells, which are believed to come from the underlying bone marrow rather than the adjacent articular surface, progressively differentiate into chondrocytes. At six to eight weeks after injury, the repair tissue contains chondrocyte-like cells in a matrix of proteoglycans and predominantly type II collagen, with some type I collagen. T. Furukawa *et al., J. Bone Joint Surg.* 62: 79-89 (1980); J. Cheung *et al., Arthritis Rheum.* 23: 211-19 (1980); S.O. Hjertquist & R. Lemperg, *Calc. Tissue Res.* 8: 54-72 (1971). However, this newly deposited matrix degenerates, and the chondroid tissue is replaced by more fibrous tissue and fibrocartilage and a shift in the synthesis of collagen from type II to type I. H.S. Cheung *et al., J. Bone Joint Surg.* 60: 1076-81 (1978); D. Hamerman, "Prospects for medical intervention in cartilage repair," *Joint cartilage degradation: Basic and clinical aspects,* Eds. Woessner JF *et al.*, (1993); Shapiro *et al.*, *J. Bone Joint Surg.* 75: 532-53 (1993); N. Mitchell & N. Shepard, *J. Bone Joint Surg.* 58: 230-33 (1976); S.O. Hjertquist & R. Lemperg, *Calc. Tissue Res.* 8: 54-72 (1971). Early degenerative changes include surface fibrillation, depletion of proteoglycans, chondrocyte cloning and death, and vertical fissuring from the superficial to deep layers. At one year post-injury, the repair tissue is a mixture of fibrocartilage and hyaline cartilage, with a substantial amount of type I collagen, which is not found in appreciable amounts in normal articular cartilage. T. Furukawa, *et al., J. Bone Joint Surg.* 62: 79-89 (1980).

From a clinical viewpoint, the fibrocartilaginous repair tissue may function satisfactorily for a certain length of time. However, fibrocartilage has inferior biomechanical properties relative to that of normal hyaline cartilage. Collagen fibers are arrayed in a random orientation with a lower elastic modulus than in normal hyaline cartilage. J. Colletti *et al., J. Bone Joint Surg.* 54:147-60 (1972). The permeability of the repair tissue is also elevated, thus reducing the fluid-pressure load-carrying capacity of the tissue. H. Mankin *et al.*, "Form and Function of Articular Cartilage", *Orthopaedic Basic Science,* Ed: Simon & Schuster, American Academy of Orthopeadic Surgeons, Rosemont, IL (1994). These changes result in increased viscoelastic deformation, making the repair tissue less able to withstand repetitive loading than normal articular cartilage. Glycosaminoglycan (GAG) levels in the cartilage adjacent to osteochondral defects have been reported to be reduced by 42% of normal values, indicating that injury leads to degeneration beyond the initial defect. *Osteoarthritis Cartilage* 3:61-70 (1995).

*Chondrocyte transplantation and survival:*

The transplantation of chondrocytes, the cells responsible for secreting cartilage matrix, has also been suggested as a means of effecting cartilage repair. However, the disadvantages of allografts, *e.g.* the possibility of the host's immunogenic response as well as the transmission of viral and other infectious diseases, has effectively limited the scope of allogenic chondrocyte transplantation. Although these risks can be minimized by using the patient's own tissue or cells, this procedure requires further surgery, creation of a new lesion in the patient's cartilage, and expensive culturing and growing of patient-specific cells.

When cultured as monolayers on tissue culture dishes, isolated chondrocytes will de-differentiate, and with time in culture, come to resemble fibroblasts. For example, collagen production will switch from predominantly type II to type I, and cells will synthesize an increased proportion of hyaluronic acid relative to the total glycosaminoglycan (GAG) content. W. Green, *Clin. Orthop. Relat. Res.* 124: 237-50 (1977). However, chondrocytes grown in collagen gels or as aggregate cultures will maintain normal morphology, proteoglycan and type II collagen synthesis as well as retain their ability to accumulate metachromatic matrix *in vitro.* Thus, under these conditions, chondocytes will remain relatively differentiated and phenotypically stable for up to several weeks *in vitro.* T. Kimura *et al., Clin. Orthop. Relat. Res.* 186: 231-39 (1984).

*Tissue engineering:*

The difficulties and expense associated with the culturing of chondrocytes has led to the design of chondrocyte-seeded or cell-free implants for articular cartilage repair using a variety of biomaterials, including: demineralized or enzymatically treated bone, L. Dahlberg *et al., J. Orthop. Res.* 9: 11-19 (1991); B.C. Toolan *et al., J. Biomed. Mat. Res.* 41: 244-50 (1998); polylactic acid, C.R. Chu *et al., J. Biomed Mat. Res.* 29: 1147-54 (1995); polyglycolic acid, C.A. Vacanti *et al., Mat. Res. Soc. Symp. Proc.* 252: 367-74 (1992); hydroxyapaptite/Dacron composites, K. Messner & J. Gillquist, *Biomaterials* 14: 513-21 (1993); fibrin, D.A. Hendrickson *et al., J. Orthop. Res.* 12: 485-97 (1994); collagen gels, D. Grande *et al., J. Orthop. Res.* 7: 208-18 (1989), S. Wakitani *et al., J. Bone Joint Surg.* 71: 74-80 (1989), S. Wakitani *et al., J. Bone Joint Surg.* 76: 579-92 (1994); and collagen fibers, J.M. Pachence *et al.*, "Development of a tissue analog for cartilage repair," Tissue inducing biomaterials, Eds, L. Cima & E. Ron, Materials Research Soc. Press, Pittsburgh, PA (1992); B.C. Toolan *et al., J. Biomed. Mat. Res.* 31: 273-80 (1996). Alternative tissues employed include synovial tissue, A.G. Rothwell, Orthopedics 13: 433-42 (1990); or tissues rich in mesenchymal stem cells (*e.g.*, bone marrow or periosteal tissue), K. Messner & J. Gillquist, *Mat. Res. Soc. Symp. Proc.* 252: 367-74 (1992).

*Standard cartilage surgical techniques*:

The present method may also be administered in combination with any standard cartilage surgical technique. Standard surgical techniques are surgical procedures which are commonly employed for therapeutic manipulations of cartilage, including: cartilage shaving, abrasion chondroplasty, laser repair, debridement, chondroplasty, microfracture with or without subchondral bone penetration, mosaicplasty, cartilage cell allografts, stem cell autografts, costal cartilage grafts, chemical stimulation, electrical stimulation, perichondral autografts, periosteal autografts, cartilage scaffolds, shell (osteoarticular) autografts or allografts, or osteotomy, These techniques are described and discussed in greater detail in Frenkel *et al.*, *Front. Bioscience* 4: d671-685 (1999).

*Cartilage Agents:*

In combination with or in lieu of tissue engineering, the administration of cartilage agents (*e.g.*, peptide growth factors) has been considered as a way to augment cartilage repair. Peptide growth factors are very significant regulators of cartilage cell differentiation, migration, adhesion, and metabolism. F. S. Chen *et al., Am J. Orthop.* 26: 396-406 (1997). Because cartilage agents are soluble proteins of relative small molecular mass and are rapidly absorbed and/or degraded, a great challenge exists in making them available to cells in sufficient quantity and for sufficient duration. Secreted proteins may thus need to be incorporated into engineered, implantable devices for maximum effectiveness. The ideal delivery vehicle is biocompatible, resorbable, has the appropriate mechanical properties, and degrades into non-toxic by-products.

Several secreted peptides have the potential to induce host cartilage repair without transplantation of cells. Insulin-like growth factor (IGF-1) stimulates both matrix synthesis and cell proliferation in culture, K. Osborn. *J. Orthop. Res.* 7: 35-42 (1989), and insufficiency of IGF-1 may have an etiologic role in the development of osteoarthritis. R.D. Coutts, *et al.*, Instructional Course Lect. 47: 487-94, *Amer. Acad. Orthop. Surg*. Rosemont, IL (1997). Some studies indicate that serum IGF-1 concentrations are lower in osteoarthritic patients than control groups, while other studies have found no difference. Nevertheless, both serum IGF-1 levels and chondrocyte responsiveness to IGF-1 decrease with age. J.R. Florini & S.B. Roberts, *J. Gerontol.* 35: 23-30 (1980). Thus, both the decreased availability of IGF-1 as well as diminished chondrocyte responsiveness to IGF-1 may contribute to cartilage homeostasis and lead to degeneration with advancing age.

IGF-1 has been proposed for the treatment or prevention of osteoarthritis. In fact, intra-articular administration of IGF-1 in combination with sodium pentosan polysulfate (a chondrocyte catabolic activity inhibitor) caused improved histological appearance, and near-normal levels of degradative enzymes (neutral metalloproteinases and collagenase), tissue inhibitors of metalloproteinase and matrix collagen. R.A. Rogachefsky, *et al., Ann. N. Y. Acad. Sci.* 732: 889-95 (1994). The use of IGF-1 either alone or as an adjuvant with other growth factors to stimulate cartilage regeneration has been described in WO 91/19510, WO 92/13565, US 5,444,047, EP 434,652.

Bone morphogenetic proteins (BMPs) are members of the large transforming growth factor beta (TGF-β) family of growth factors. *In vitro* and *in vivo* studies have shown that BMP induces the differentiation of mesenchymal cells into chondrocytes. K. Sato & M. Urist, *Clin. Orthop. Relat. Res.* 183: 180-87 (1984). Furthermore, skeletal growth factor and cartilage-derived growth factors have synergistic effects with BMP, as the combination of these growth factors with BMP and growth hormone initiates mesenchymal cell differentiation. Subsequent proliferation of the differentiated cells are stimulated by other factors. D.J. Hill & A. Logan, *Prog. Growth Fac. Res.* 4: 45-68 (1992).

Transforming growth factor beta (TGF-β) is produced by osteoblasts, chondrocytes, platelets, activated lymphocytes, and other cells. R.D. Coutts *et al., supra.* TGF-β can have both stimulatory and inhibitory properties on matrix synthesis and cell proliferation depending on the target cell, dosage, and cell culture conditions. P. Guerne *et al., J. Cell Physiol.* 158: 476-84 (1994); H. Van Beuningen *et al.*, *Ann. Rheum. Dis.* 52: 185-91 (1993); P. Van der Kraan *et al.*, *Ann. Rheum. Dis.* 51: 643-47 (1992). Furthermore, as with IGF-1, TGF-β responsiveness is decreased with age. P. Guerne *et al.*, *J. Cell Physiol.* 158: 476-84 (1994). However, TGF-β is a more potent stimulator of chondrocyte proliferation than other growth factors, including platelet-derived growth factor (PDGF), bFGF, and IGF-1 (Guerne *et al., supra*), and can stimulate proteoglycan production by chondrocytes. TGF-β also down-regulates the effects of cytokines which stimulate chondrocyte catabolism. Van der Kraan *et al., supra. In vivo,* TGF-β induces proliferation and differentiation of mesenchymal cells into chondrocytes and enhances repair of partial-thickness defects in rabbit articular cartilage. E.B. Hunziker & L. Rosenberg, *Trans. Orthopaed. Res. Soc.* 19: 236 (1994).

*Antagonism of cartilage catabolism*

Cartilage matrix degradation is believed to be due to cleavage of matrix molecules (proteoglycans and collagens) by proteases (reviewed in Woessner JF Jr., "Proteases of the extracellular matrix", in Mow, V., Ratcliffe, A. (eds): Structure and Function of Articular Cartilage. Boca Raton, FL, CRC Press, 1994 and Smith R.L., *Front. In Biosci.* 4:

d704-712. While the key enzymes involved in matrix breakdown have not yet been clearly identified, matrix metalloproteinases (MMPs) and "aggrecanases" appear to play key roles in joint destruction. In addition, members of the serine and cysteine family of proteinases, for example the cathepsins and urokinase or tissue plasminogen activator (uPA and tPA) may also be involved. Plasmin, urokinase plasminogen activator (uPA) and tissue plasminogen activator (tPA) may play an important role in the activation pathway of the metalloproteinases. Evidence connects the closely related group of cathepsin B, L and S to matrix breakdown, and these cathepsins are somewhat increased in OA. Many cytokines, including IL-1, TNF-$\alpha$ and LIF induce MMP expression in chondrocytes. Induction of MMPs can be antagonized by TGF-$\beta$ and is potentiated, at least in rabbits, by FGF and PDGF. As shown by animal studies, inhibitors of these proteases (MMPs and aggrecanases) may at least partially protect joint tissue from damage *in vivo.*

Other methods of stimulating cartilage repair include blocking the effects of molecules which are associated with cartilage destruction. For example, both IL-1 (-$\alpha$ and -$\beta$) and nitric oxide are substances with known catabolic effects on cartilage. The cytokine IL-1 causes cartilage breakdown, including the generation of synovial inflammation and up-regulation of matrix metalloproteinases and aggrecanases. V. Baragi, *et al., J. Clin. Invest.* 96: 2454-60 (1995); V.M. Baragi *et al., Osteoarthritis Cartilage* 5: 275-82 (1997); C.H. Evans *et al., J. Leukoc. Biol.* 64: 55-61 (1998); C.H Evans and P.D. Robbins, *J. Rheumatol.* 24: 2061-63 (1997); R. Kang *et al., Biochem. Soc. Trans.* 25: 533-37 (1997); R. Kang *et al., Osteoarthritis Cartilage* 5: 139-43 (1997). Because high levels of IL-1 are found in diseased joints and IL-1 is believed to play a pivotal role in initiation and development of arthritis, inhibition of IL-1 activity may prove to be a successful therapy. In mammals only one protease, named interleukin 1 $\beta$-convertase (ICE), can specifically generate mature, active IL-1$\beta$. Inhibition of ICE has been shown to block IL-1$\beta$ production and may slow arthritic degeneration (reviewed in Martel-Pelletier J. *et al., Front. Biosci.* 4: d694-703). The soluble IL-1 receptor antagonist (IL-1ra), a naturally occurring protein that can inhibit the effects of IL-1 by preventing IL-1 from interacting with chondrocytes, has also been shown to be effective in animal models of arthritis and is currently being tested in humans for its ability to prevent incidence or progression of arthritis.

Nitric oxide (NO) has been implicated to play a role in the destruction of cartilage. Attur *et al., Arthritis & Rheum.* 40: 1050-1053 (1997); Ashok *et al., Curr. Opin. Rheum.* 10: 263-268 (1998). Unlike normal cartilage which does not produce NO unless stimulated with cytokines such as IL-1$\alpha$, cartilage obtained from osteoarthritic joints produces large amounts of nitric oxide for over 3 days in culture despite the absence of added stimuli. Moreover, inhibition of NO production has been shown to prevent IL-1$\alpha$ mediated cartilage destruction and chondrocyte death as well as progression of osteoarthritis in animal models. Moreover, tissue explants from such patients spontaneously release high levels of nitrite in the absence of stimulation with cytokines such as IL-1. Amin *et al., Cur. Opin. Rheum.* 10: 263-268 (1998). While a conclusive determination of the positive or negative role of NO in the progression of joint determination has not yet been made, the inhibition of NO can attenuate the effects of IL-1$\alpha$ on matrix metalloproteinase production, aggrecan synthesis, and lactate production by chondrocytes - thus, inhibition of NO may be one way to prevent cartilage destruction.

As with IL-1$\alpha$ and $\beta$, TNF-$\alpha$ is synthesized by chondrocytes, induces matrix breakdown, inhibits matrix synthesis, and is found at high levels in arthritic joints. TNF-$\alpha$ also synergizes with IL-1 in terms of cartilage destruction. Inhibition of TNF-$\alpha$ activity, in arthritic animals and humans has been shown to inhibit progression of arthritis.

Leukemia inhibitory factor (LIF), which is synthesized by both cartilage and synovium, is present in human synovial fluids. Because LIF induces the synthesis of matrix metalloproteinases (MMPs) by chondrocytes, it may be involved in the breakdown of the cartilaginous matrix.

Interferon-gamma (IFN-$\gamma$) inhibits proteoglycan synthesis by human chondrocytes without enhancing its breakdown. Indeed, IFN-$\gamma$ may suppress proteoglycan loss by inhibiting the induction of MMPs.

Interleukin 8, a potent chemotactic cytokine for polymorphonuclear neutrophils (PMN), is synthesized by a variety of cells including monocytes/macrophages, chondrocytes and fibroblasts and is induced by TNF-$\alpha$. In OA patients, IL-1$\beta$, IL-6, TNF-$\alpha$ and IL-8 are all found in the synovial fluid. IL-8 can enhance the release of inflammatory cytokines in human mononuclear cells, including that of IL-1$\beta$, IL-6 and TNF-$\alpha$, which may further modulate the inflammatory reaction (reviewed in Martel-Pelletier J. *et al., Front. Biosci.* 4: d694-703).

IL-6 has also been proposed as a contributor to the OA pathological process by increasing inflammatory cells in the synovial tissue and by stimulating the proliferation of chondrocytes. In addition, IL-6 can amplify the effects of IL-1 on MMP synthesis and inhibition of proteoglycan production (reviewed in Martel-Pelletier J. *et al., Front. Biosci.* 4: d694-703).

Interleukin 17 upregulates production of IL-1$\beta$, TNF-$\alpha$, IL-6 and MMPs in human macrophages. IL-17 also induces NO production in chondrocytes, and is expressed in arthritic, but not normal joints (reviewed in Martel-Pelletier J. *et al., Front. Biosci.* 4: d694-703).

Basic fibroblast growth factor (bFGF), which is synthesized by chondrocytes, can induce articular chondrocyte replication. B. C. Toolan *et al., J. Biomed. Mat. Res.* 41: 244-50 (1998). In explants taken from young animals, bFGF in small amounts (*e.g.,* 3 ng/ml) stimulates synthesis and inhibits breakdown of proteoglycans, while higher levels (*e.g.*, 30-300 ng/ml) has exactly the opposite effect (*i.e.*, synthesis inhibition and enhanced breakdown). In adult tissues, higher doses of FGF stimulated proteoglycan, protein and collagen synthesis with no cell proliferation. R.L. Sah *et al., Arch.*

*Biochem. Biophys.* 308: 137-47 (1994). bFGF also regulates cartilage homeostasis by inducing the autocrine release from chondrocytes of interleukin 1 (IL-1), a potent stimulator of catabolic behavior in cartilage. bFGF further enhances IL-1-mediated protease release, perhaps through its ability to upregulate IL-1 receptors on chondrocytes. J.E. *Chin et al., Arthritis Rheum*. 34: 314-24 (1991). Similarly, platelet-derived growth factor (PDGF) can potentiate the catabolic effects of IL-1 and presumably of TNF-$\alpha$. However, some evidence suggests that in human cartilage bFGF and PDGF may have an anticatabolic effect; whether this phenomenon is species-specific or an effect of age remains to be determined.

While inflammation does not appear to be the initiating event in osteoarthritis, inflammation does occur in osteoarthritic joints. The inflammatory cells (*i.e*., monocytes, macrophages, and neutrophils) which invade the synovial lining after injury and during inflammation produce metalloproteinases as well as catabolic cyokines which can contribute to further release of degradative enzymes. Although inflammation and joint destruction do not show perfect correlation in all animal models of arthritis, agents which inhibit inflammation (*e.g.*, IL-4, IL-13, IL-10) also decrease cartilage and bone pathology in arthritic animals (reviewed in Martel-Pelletier J. *et al., Front. Biosci.* 4: d694-703). Application of agents which inhibit inflammatory cytokines may slow OA progression by countering the local synovitis which occurs in OA patients.

Numerous studies show that members of the tetracycline family of antibiotics are effective in inhibiting collagenase and gelatinase activity. Oral administration of one of these, doxycycline, proved to decrease both collagenase and gelatinase activity in cartilage from endstage hip osteoarthritis. These data suggest that an effective oral dose of doxycycline may slow down the progression of osteoarthritis. Smith R.L., *Front. Biosci*. 4: d704-712.

The pathology of OA involves not only the degeneration of articular cartilage leading to eburnation of bone, but also extensive remodeling of subchondral bone resulting in the so-called sclerosis of this tissue. These bony changes are often accompanied by the formation of subchondral cysts as a result of focal resorption. Agents which inhibit bone resorption, *i.e.* osteoprotegerin or bisphosphonates have shown promising results in animal models of arthritis, and therefore show promise in treating cartilaginous disorders. Kong *et al. Nature* 402: 304-308.

DNA153576 appears to be expressed at high levels specifically in cartilage (Figs. 4 & 5). No expression of DNA153576 was detected in libraries prepared from the spleen, heart, thymus, placenta, kidney, testes, uterus, skin, liver, lung, esophagus, thyroid (Fig. 4) nor in RNA prepared from colon, kidney, lung, small bowel, lymph node, duodenum and artery (Fig. 5).

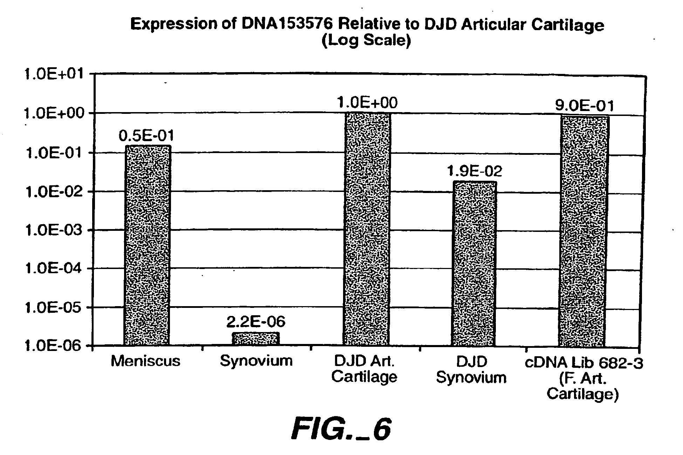

The level of expression of DNA 153576 in cartilage is similar to that of actin, indicating that the gene is highly expressed. Furthermore, levels in adult cartilage appear to be similar to that found in fetal cartilage (Figs. 5, 6), suggesting that this molecule plays an important role in chondrocyte biology. Interestingly, -ten-fold lower levels of expression were found in the meniscus, which is fibrocartilage, in contrast to articular cartilage, which is hyaline cartilage (Fig. 6). Furthermore, though expressed at very low levels (~100x lower than in articular cartilage) in diseased synovium, expression in normal synovium was even lower (10,000x) than that in diseased synovium (Fig. 6). Thus, upregulation of expression of DNA153576 in the synovium may be part of the disease process in joints.

The native sequence PR021074 protein encoded by DNA153576 shows sequence homology to inter-alpha-trypsin inhibitor (ITI), a polypeptide which appears to bind to hyaluronan (HA); ITI may thus be involved in assembly of, and adherence of cells to, the cartilage matrix. Based on its homology to ITI, PRO21074 likely also plays a significant role in chondrocyte attachment and cartilage matrix assembly. As such, native sequence PRO21074 will likely be useful as a therapeutic agent to stimulate the repair of cartilage tissue, especially at the interface of the "new" and "old" cartilage. Since the matrix can provide signals back to chondrocyctes, PRO21074 may also affect chondrocyte proliferation and differentiation. Such activites would also be beneficial to cartilage repair.

Given the high level of sequence homology between ITI and the protein encoded by DNA153576 (native sequence PRO21074), we expect native sequence PRO21074 to bind hyaluronan and to be retained within the extracellular matrix. Since arthritis is characterized by degeneration of cartilage and release of cartilage matrix molecules, changes in the levels of native sequence PRO21074, or fragments thereof (*e.g.*, in the synovial fluid, plasma/serum, or urine) may be indicative of distinct stages of joint degeneration.

As mentioned in the previous paragraph, the presence of native sequence PRO21074 (including agonists thereof) is likely to benefit or be therapeutic to the repair or regeneration of cartilage. However, it is also possible that excessively high levels of native sequence PRO21074, or fragments thereof, (*e.g.*, within the joint or in the synovial fluid) may also be detrimental. In such a situation, therapeutic benefit would lie in the adminstration of antagonists of PRO21074.

## III. Compositions and Methods of the Invention

### A. Full-length PRO21074 Polypeptide

The present invention provides newly identified and isolated nucleotide sequences encoding polypeptides referred to in the present application as PRO21074 (or also UNQ6369). In particular, cDNA encoding a PRO21074 polypeptide has been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by DNA 153576-2925 as well as all further native homologues and variants included in the foregoing definition of PRO21074, will be referred to as "PRO21074", regardless of their origin or mode of preparation.

As disclosed in the Examples below, a cDNA clone designated herein as DNA153576-2925 has been deposited with the ATCC. The actual nucleotide sequence of the clone can readily be determined by the skilled artisan by sequencing of the deposited clone using routine methods in the art. The predicted amino acid sequence can be determined from the nucleotide sequence using routine skill. For the PRO21074 polypeptide and encoding nucleic acid described herein, Applicants have identified what is believed to be the reading frame best identifiable with the sequence information available at the time.

Using the ALIGN-2 sequence alignment computer program referenced above, it has been found that the full-length native sequence PRO21074 (shown in Figure 2 and SEQ ID NO: 2) has certain amino acid sequence identity with sequence from a piece of genomic DNA (Dayhoff No. HS1409_1). However, this genomic clone does not contain the complete coding sequence of PRO21074. Accordingly, it is presently believed that the PR021074 polypeptide disclosed in the present application is a newly identified member of the inter-alpha-trypsin inhibitor protein family and may possess one or more biological, enzymatic, and/or immunological activities or properties of members of that protein family.

B. <u>PRO21074 Variants</u>

In addition to the full-length native sequence PRO21074 polypeptides described herein, it is contemplated that PRO21074 variants can be prepared. PRO21074 variants can be prepared by introducing appropriate nucleotide changes into the PRO21074 DNA, and/or by synthesis of the desired PRO21074 polypeptide. Those skilled in the art will appreciate that amino acid changes may alter post-translational processes of the PRO21074, such as changing the number or position of glycosylation sites or altering the membrane anchoring characteristics.

Variations in the native full-length sequence PRO21074 or in various domains of the PRO21074 described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO21074 that results in a change in the amino acid sequence of the PRO21074 as compared with the native sequence PRO21074. Optionally the variation is by substitution of at least one amino acid with any other amino acid in one or more of the domains of the PRO21074. Guidance in determining which amino acid residue may be inserted, substituted or deleted without adversely affecting the desired activity may be found by comparing the sequence of the PRO21074 with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO21074 polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO21074 polypeptide.

PRO21074 fragments may be prepared by any of a number of conventional techniques. Desired peptide fragments may be chemically synthesized. An alternative approach involves generating PRO21074 fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO21074 polypeptide fragments share at least one biological and/or immunological activity with the native PRO21074 polypeptide shown in Figure 2 (SEQ ID NO: 2).

In particular embodiments, conservative substitutions of interest are shown in Table 3 under the heading of preferred substitutions. If such substitutions result in a change in biological activity, then more substantial changes, denominated exemplary substitutions in Table 3, or as further described below in reference to amino acid classes, are introduced and the products screened.

Table 3

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO21074 polypeptide are accomplished by selecting.substitutions.that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter *et al., Nucl. Acids Res.,* 13: 4331 (1986); Zoller *et al., Nucl. Acids Res.,* 10:6487 (1987)], cassette mutagenesis [Wells *et al., Gene,* 34:315 (1985)], restriction selection mutagenesis [Wells *et al., Philos. Trans. R. Soc. London SerA,* 317:415 (1986)] or other known techniques can be performed on the cloned DNA to produce the PR021074 variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, *Science,* 244: 1081-1085 (1989)]. Alanine is also typically preferred because it is the most common amino acid. Further, it is frequently found in both buried and exposed positions [Creighton, *The Proteins,* (W.H. Freeman & Co., N.Y.); Chothia, *J. Mol. Biol.*, 150:1 (1976)]. If alanine substitution does not yield adequate amounts of variant, an isoteric amino acid can be used.

C. Modifications of PRO21074

Covalent modifications of PRO21074 are included within the scope of this invention. One type of covalent modification includes reacting targeted amino acid residues of a PRO21074 polypeptide with an organic derivatizing agent that is capable of reacting with selected side chains or the N- or C- terminal residues of the PRO21074. Derivatization with bifunctional agents is useful, for instance, for crosslinking PRO21074 to a water-insoluble support matrix or surface for use in the method for purifying anti-PRO21074 antibodies, and vice-versa. Commonly used crosslinking agents include, *e.g.*, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\alpha$-amino groups of lysine, arginine, and histidine side chains [T.E. Creighton, *Proteins: Structure and Molecular Properties,* W.H. Freeman & Co., San Francisco, pp. 79-86 (1983)], acetylation of the N-terminal amine, and amidation of any C-terminal carboxyl group.

Another type of covalent modification of the PRO21074 polypeptide included within the scope of this invention comprises altering the native glycosylation pattern of the polypeptide. "Altering the native glycosylation pattern" is intended for purposes herein to mean deleting one or more carbohydrate moieties found in native sequence PRO21074 (either by removing the underlying glycosylation site or by deleting the glycosylation by chemical and/or enzymatic means), and/or adding one or more glycosylation sites that are not present in the native sequence PRO21074. In addition, the phrase includes qualitative changes in the glycosylation of the native proteins, involving a change in the nature and proportions of the various carbohydrate moieties present.

Addition of glycosylation sites to the PRO21074 polypeptide may be accomplished by altering the amino acid sequence. The alteration may be made, for example, by the addition of, or substitution by, one or more serine or threonine residues to the native sequence PRO21074 (for O-linked glycosylation sites). The PRO21074 amino acid sequence may optionally be altered through changes at the DNA level, particularly by mutating the DNA encoding the PRO21074 polypeptide at preselected bases such that codons are generated that will translate into the desired amino acids.

Another means of increasing the number of carbohydrate moieties on the PRO21074 polypeptide is by chemical or enzymatic coupling of glycosides to the polypeptide. Such methods are described in the art, e.g., in WO 87/05330 published 11 September 1987, and in Aplin and Wriston, *CRC Crit. Rev. Biochem.,* pp. 259-306 (1981).

Removal of carbohydrate moieties present on the PR021074 polypeptide may be accomplished chemically or enzymatically or by mutational substitution of codons encoding for amino acid residues that serve as targets for glycosylation. Chemical deglycosylation techniques are known in the art and described, for instance, by Hakimuddin, *et al., Arch. Biochem. Biophys.,* 259:52 (1987) and by Edge *et al., Anal. Biochem.,* 118:131 (1981). Enzymatic cleavage of carbohydrate moieties on polypeptides can be achieved by the use of a variety of endo- and exo-glycosidases as described by Thotakura *et al., Meth. Enzymol.,* 138:350 (1987).

Another type of covalent modification of PRO21074 comprises linking the PR021074 polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO21074 of the present invention may also be modified in a way to form a chimeric molecule comprising PRO21074 fused to another, heterologous polypeptide or amino acid sequence.

In one embodiment, such a chimeric molecule comprises a fusion of the PRO21074 with a tag polypeptide which provides an epitope to which an anti-tag antibody can selectively bind. The epitope tag is generally placed at the amino- or carboxyl- terminus of the PRO21074. The presence of such epitope-tagged forms of the PRO21074 can be detected using an antibody against the tag polypeptide. Also, provision of the epitope tag enables the PRO21074 to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field *et al., Mol. Cell. Biol.,* 8:2159-2165 (1988)]; the c-myc tag and the 8F9, 3C7, 6E10, G4, B7 and 9E10 antibodies thereto [Evan *et al., Molecular and Cellular Biology,* 5:3610-3616 (1985)]; and the Herpes Simplex virus glycoprotein D (gD) tag and its antibody [Paborsky *et al., Protein Engineering*, 3(6):547-553 (1990)]. Other tag polypeptides include the Flag-peptide [Hopp *et al., BioTechnology,* 6:1204-1210 (1988)]; the KT3 epitope peptide [Martin *et al., Science,* 255:192-194 (1992)]; an $\alpha$-tubulin epitope peptide [Skinner *et al., J. Biol. Chem.*, 266:15163-15166 (1991)]; and the T7 gene 10 protein peptide tag [Lutz-Freyermuth *et al., Proc. Natl. Acad. Sci. USA*, 87:6393-6397 (1990)].

In an alternative embodiment, the chimeric molecule may comprise a fusion of the PRO21074 with an immunoglobulin or a particular region of an immunoglobulin. For a bivalent form of the chimeric molecule (also referred to as an "immunoadhesin"), such a fusion could be to the Fe region of an IgG molecule. The Ig fusions preferably include the substitution of a soluble (transmembrane domain deleted or inactivated) form of a PR021074 polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

D. Preparation of PRO21074

The description below relates primarily to production of PRO21074 by culturing cells transformed or transfected with a vector containing PRO21074 nucleic acid and purification of the resulting protein. It is, of course, contemplated that alternative methods, which are well known in the art, may be employed to prepare PRO21074. For instance, the PRO21074 sequence, or portions thereof, may be produced by direct peptide synthesis using solid-phase techniques [see, e.g., Stewart *et al., Solid-Phase Peptide Synthesis,* W.H. Freeman Co., San Francisco, CA (1969); Merrifield, *J. Am. Chem. Soc.,* 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO21074 may be chemically synthesized separately and combined using chemical or enzymatic methods to produce the full-length PR021074.

1. Isolation of DNA Encoding PRO21074

DNA encoding PRO21074 may be obtained from a cDNA library prepared from tissue believed to possess the PRO21074 mRNA and to express it at a detectable level. Accordingly, human PRO21074 DNA can be conveniently obtained from a cDNA library prepared from human tissue, such as described in the Examples. The PRO21074-encoding gene may also be obtained from a genomic library or by known synthetic procedures (*e.g.*, automated nucleic acid synthesis).

Libraries can be screened with probes (such as antibodies to the PR021074 or oligonucleotides of at least about 20-80 bases) designed to identify the gene of interest or the protein encoded by it. Screening the cDNA or genomic library with the selected probe may be conducted using standard procedures, such as described in Sambrook *et al., Molecular Cloning: A Laboratory Manual* (New York: Cold Spring Harbor Laboratory Press, 1989). An alternative means to isolate the gene encoding PRO21074 is to use PCR methodology [Sambrook *et al*., *supra*; Dieffenbach *et al., PCR Primer: A Laboratory Manual* (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like $^{32}P$-labeled ATP, biotinylation or enzyme labeling. Hybridization conditions, including moderate stringency and high stringency, are provided in Sambrook *et al., supra*.

Sequences identified in such library screening methods can be compared and aligned to other known sequences deposited and available in public databases such as Genbank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook *et al*., supra, to detect precursors and processing intermediates of mRNA that may not have been reverse-transcribed into cDNA.

2. Selection and Transformation of Host Cells

Host cells are transfected or transformed with expression or cloning vectors described herein for PRO21074 production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in *Mammalian Cell Biotechnology: A Practical Approach,* M. Butler, ed. (IRL Press, 1991) and Sambrook *et al*., *supra.*

Methods of eukaryotic cell transfection and prokaryotic cell transformation are known to the ordinarily skilled artisan, for example, $CaCl_2$, $CaPO_4$, liposome-mediated and electroporation. Depending on the host cell used, transformation is performed using standard techniques appropriate to such cells. The calcium treatment employing calcium chloride, as described in Sambrook *et al., supra,* or electroporation is generally used for prokaryotes. Infection with *Agrobacterium tumefaciens* is used for transformation of certain plant cells, as described by Shaw *et al., Gene*, 23:315 (1983) and WO 89/05859 published 29 June 1989. For mammalian cells without such cell walls, the calcium phosphate precipitation method of Graham and van der Eb, *Virology,* 52:456-457 (1978) can be employed. General aspects of mammalian cell host system transfections have been described in U.S. Patent No. 4,399,216. Transformations into yeast are typically carried out according to the method of Van Solingen *et al., J. Bact.,* 130:946 (1977) and Hsiao *et al., Proc. Natl. Acad Sci. (USA),* 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown *et al., Methods in Enzymology*, 185:527-537 (1990) and Mansour *et al., Nature,* 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli*. Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella*, e.g., *Salmonella typhimurium, Serratia,* e.g., *Serratia marcescans,* and *Shigella,* as well as *Bacilli* such as *B. subtilis* and *B. licheniformis* (*e.g., B. licheniformis* 41P disclosed in DD 266,710 published 12 April 1989), *Pseudomonas* such as *P. aeruginosa,* and *Streptomyces*. These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3*; *E. coli* W3110 strain 27C7 (ATCC 55,244), which has the complete genotype *tonA ptr3 phoA E15 (argF-lac)169 degP ompT kan$^r$*; *E. coli* W3110 strain 37D6, which has the complete genotype *tonA ptr3 phoA E15 (argF-lac)169 degP ompT rbs7 ilvG kan$^r$; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, *e.g.,* PCR or other nucleic acid polymerase reactions, are suitable.

In addition to prokaryotes, eukaryotic microbes such as filamentous fungi or yeast are suitable cloning or expression hosts for PRO21074-encoding vectors. *Saccharomyces cerevisiae* is a commonly used lower eukaryotic host microorganism. Others include *Schizosaccharomyces pombe* (Beach and Nurse, *Nature,* 290:140 [1981]; EP 139,383 published 2 May 1985); *Kluyveromyces* hosts (U.S. Patent No. 4,943,529; Fleer *et al., Bio/Technology*, 9: 968-975 (1991)) such as, e.g., *K. lactis* (MW98-8C, CBS683, CBS4574; Louvencourt *et al., J. Bacteriol.*, 154(2): 737-42 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg *et al., Bio/Technology,* 8:135 (1990)), *K. thermotolerans,* and *K. marxianus; yarrowia* (EP 402,226); *Pichia pastoris* (EP 183,070; Sreekrishna *et al., J. Basic Microbiol.*, 28:265-278 [1988]); *Candida; Trichoderma reesia* (EP 244,234); *Neurospora crassa* (Case *et al., Proc. Natl. Acad. Sci. USA,* 76:5259-5263 [1979]); *Schwanniomyces* such as *Schwanniomyces occidentalis* (EP 394,538 published 31 October 1990); and filamentous fungi such as, *e.g., Neurospora, Penicillium, Tolypocladiuni* (WO 91/00357 published 10 January 1991), and *Aspergillus* hosts such as *A. nidulans* (Ballance *et al., Biochem. Biophys. Res. Commun.*, 112:284-289 [1983]; Tilburn *et al., Gene,* 26:205-221 [1983]; Yelton *et al., Proc. Natl. Acad. Sci. USA,* 81: 1470-1474 [1984]) and *A. niger* (Kelly and Hynes, *EMBO J.,* 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula*. A list of specific species that are exemplary of this class of yeasts may be found in C. Anthony, *The Biochemistry of Methylotrophs*, 269 (1982).

Suitable host cells for the expression of glycosylated PRO21074 are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham *et al., J. Gen Virol.*, 36:59 (1977)); Chinese hamster ovary cells/-DHFR (CHO, Urlaub and Chasin, *Proc. Natl. Acad Sci. USA*, 77:4216 (1980)); mouse sertoli cells (TM4, Mather, *Biol. Reprod.*, 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

3. Selection and Use of a Replicable Vector

The nucleic acid (*e.g.*, cDNA or genomic DNA) encoding PR021074 may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PR021074 may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO21074-encoding DNA that is inserted into the vector. The signal sequence may be a prokaryotic signal sequence selected, for example, from the group of the alkaline phosphatase, penicillinase, 1pp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.,* ampicillin, neomycin, methotrexate, or tetracycline, (b) complement auxotrophic deficiencies, or (c) supply critical nutrients not available from complex media, *e.g.,* the gene encoding D-alanine racemase for *Bacilli.*

An example of suitable selectable markers for mammalian cells are those that enable the identification of cells competent to take up the PRO21074-encoding nucleic acid, such as DHFR or thymidine kinase. An appropriate host cell when wild-type DHFR is employed is the CHO cell line deficient in DHFR activity, prepared and propagated as described by Urlaub *et al., Proc. Natl. Acad. Sci. USA,* 77:4216 (1980). A suitable selection gene for use in yeast is the *trp*1 gene present in the yeast plasmid YRp7 [Stinchcomb *et al., Nature,* 282:39 (1979); Kingsman *et al.*, Gene, 7:141 (1979); Tschemper *et al., Gene,* 10:157 (1980)]. The *trp*1 gene provides a selection marker for a mutant strain of yeast lacking the ability to grow in tryptophan, for example, ATCC No. 44076 or PEP4-1 [Jones, *Genetics,* 85:12 (1977)].

Expression and cloning vectors usually contain a promoter operably linked to the PRO21074-encoding nucleic acid sequence to direct mRNA synthesis. Promoters recognized by a variety of potential host cells are well known. Promoters suitable for use with prokaryotic hosts include the β-lactamase and lactose promoter systems [Chang *et al., Nature*, 275:615 (1978); Goeddel *et al., Nature*, 281:544 (1979)], alkaline phosphatase, a tryptophan (trp) promoter system [Goeddel, *Nucleic Acids Res.,* 8:4057 (1980); EP 36,776], and hybrid promoters such as the tac promoter [deBoer *et al., Proc. Natl. Acad. Sci. USA,* 80:21-25 (1983)]. Promoters for use in bacterial systems also will contain a Shine-Dalgarno (S.D.) sequence operably linked to the DNA encoding PRO21074.

Examples of suitable promoting sequences for use with yeast hosts include the promoters for 3-phosphoglycerate kinase [Hitzeman *et al., J. Biol. Chem.*, 255:2073 (1980)] or other glycolytic enzymes [Hess *et al.*, *J. Adv. Enzyme Reg.,* 7:149 (1968); Holland, *Biochemistry*, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO21074 transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g.*, the actin promoter or an immunoglobulin promoter, and from heat-shock promoters, provided such promoters are compatible with the host cell systems.

Transcription of a DNA encoding the PRO21074 by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus.

Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO21074 coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO21074.

Still other methods, vectors, and host cells suitable for adaptation to the synthesis of PRO21074 in recombinant vertebrate cell culture are described in Gething *et al., Nature,* 293:620-625 (1981); Mantei *et al., Nature,* 281:40-46 (1979); EP 117,060; and EP 117,058.

4. Detecting Gene Amplification/Expression

Gene amplification and/or expression may be measured in a sample directly, for example, by conventional Southern blotting, Northern blotting to quantitate the transcription of mRNA [Thomas, *Proc. Natl. Acad. Sci. USA,* 77: 5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO21074 polypeptide or against a synthetic peptide based on the DNA sequences provided herein or against exogenous sequence fused to PRO21074 DNA and encoding a specific antibody epitope.

5. Purification of Polypeptide

Forms of PRO21074 may be recovered from culture medium or from host cell lysates. If membrane-bound, it can be released from the membrane using a suitable detergent solution (*e.g.* Triton-X 100) or by enzymatic cleavage. Cells employed in expression of PRO21074 can be disrupted by various physical or chemical means, such as freeze-thaw cycling, sonication, mechanical disruption, or cell lysing agents.

It may be desired to purify PRO21074 from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO21074. Various methods of protein purification may be employed and such methods are known in the art and described for example in Deutscher, *Methods in Enzymology*, 182 (1990); Scopes, *Protein Purification: Principles and Practice,* Springer-Verlag, New York (1982). The purification step(s) selected will depend, for example, on the nature of the production process used and the particular PRO21074 produced.

E. Uses for PRO21074

Nucleotide sequences (or their complement) encoding PRO21074 have various applications in the art of molecular biology, including uses as hybridization probes, in chromosome and gene mapping and in the generation of antisense RNA and DNA. PRO21074 nucleic acid will also be useful for the preparation of PRO21074 polypeptides by the recombinant techniques described herein.

The full-length native sequence PRO21074 gene (SEQ ID NO: 1), or portions thereof, may be used as hybridization probes for a cDNA library to isolate the full-length PRO21074 cDNA or to isolate still other cDNAs (for instance, those encoding naturally-occurring variants of PRO21074 or PR021074 from other species) which have a desired sequence identity to the PRO21074 sequence disclosed in Figure 1 (SEQ ID NO: 1). Optionally, the length of the probes will be about 20 to about 50 bases. The hybridization probes may be derived from at least partially novel regions of the nucleotide sequence of SEQ ID NO: 1 wherein those regions may be determined without undue experimentation or from genomic sequences including promoters, enhancer elements and introns of native sequence PRO21074. By way of example, a screening method will comprise isolating the coding region or the untranslated region of the PRO21074 gene using the known DNA sequence to synthesize a selected probe of about 40 bases. Hybridization probes may be labeled by a variety of labels, including radionucleotides such as $^{32}P$ or $^{35}S$, of enzymatic labels such as alkaline phosphatase coupled to the probe via avidin/biotin coupling systems. Labeled probes having a sequence complementary to that of the PRO21074 gene of the present invention can be used to screen libraries of human cDNA, genomic DNA or mRNA to determine which members of such libraries the probe hybridizes to. Hybridization techniques are described in further detail in the Examples below.

Any EST sequences disclosed in the present application may similarly be employed as probes, using the methods disclosed herein.

Other useful fragments of the PRO21074 nucleic acids include antisense or sense oligonucleotides comprising a singe-stranded nucleic acid sequence (either RNA or DNA) capable of binding to target PRO21074 mRNA (sense) or PRO21074 DNA (antisense) sequences. Antisense or sense oligonucleotides, according to the present invention, comprise a fragment of the coding region or the untranslated region of PRO21074 DNA. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (*Cancer Res*. 48:2659, 1988) and van der Krol *et al.* (*BioTechniques* 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. The antisense oligonucleotides thus may be used to block expression of PRO21074 proteins. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (*i.e.*, capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, $CaPO_4$-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either *in vivo* or *ex vivo.* Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

The probes may also be employed in PCR techniques to generate a pool of sequences for identification of closely related PRO21074 coding sequences.

Nucleotide sequences encoding a PRO21074 can also be used to construct hybridization probes for: determining sites of expression of DNA 153576 in tissues (*in situ* hybridization), mapping the gene which encodes PR021074, and for the genetic analysis of individuals with particular diseases. The nucleotide sequences provided herein may be mapped to a chromosome and specific regions of a chromosome using known techniques, such as *in situ* hybridization, linkage analysis against known chromosomal markers, and hybridization screening with libraries.

When the coding sequences for PRO21074 encode a protein which binds to another protein (example, where the PRO21074 is a receptor), the PRO21074 can be used in assays to identify the other proteins or molecules involved in the binding interaction. By such methods, inhibitors of the receptor/ligand binding interaction can be identified. Proteins involved in such binding interactions can also be used to screen for peptide or small molecule inhibitors or agonists of the binding interaction. Also, the receptor PRO21074 can be used to isolate correlative ligand(s). Screening assays can be designed to find lead compounds that mimic the biological activity of a native PRO21074 or a receptor for PRO21074.

Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode PRO21074 or its modified forms can also be used to generate either transgenic animals or "knock out" animals which, in turn, are useful in the development and screening of therapeutically useful reagents. A transgenic animal (*e.g.,* a mouse or rat) is an animal having cells that contain a transgene, which transgene was introduced into the animal or an ancestor of the animal at a prenatal, *e.g.*, an embryonic stage. A transgene is a DNA which is integrated into the genome of a cell from which a transgenic animal develops. In one embodiment, cDNA encoding PRO21074 can be used to clone genomic DNA encoding PRO21074 in accordance with established techniques and the genomic sequences used to generate transgenic animals that contain cells which express DNA encoding PRO21074. Methods for generating transgenic animals, particularly animals such as mice or rats, have become conventional in the art and are described, for example, in U.S. Patent Nos. 4,736,866 and 4,870,009. Typically, particular cells would be targeted for PRO21074 transgene incorporation with tissue-specific enhancers. Transgenic animals that include a copy of a transgene encoding PRO21074 introduced into the germ line of the animal at an embryonic stage can be used to examine the effect of increased expression of DNA encoding PRO21074. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Alternatively, non-human homologues of PRO21074 can be used to construct a PRO21074 "knock out" animal which has a defective or altered gene encoding PRO21074 as a result of homologous recombination between the endogenous gene encoding PRO21074 and altered genomic DNA encoding PRO21074 introduced into an embryonic stem cell of the animal. For example, cDNA encoding PRO21074 can be used to clone genomic DNA encoding PRO21074 in accordance with established techniques. A portion of the genomic DNA encoding PRO21074 can be deleted or replaced with another gene, such as a gene encoding a selectable marker which can be used to monitor integration. Typically, several kilobases of unaltered flanking DNA (both at the 5' and 3' ends) are included in the vector [see *e.g.*, Thomas and Capecchi, *Cell,* 51:503 (1987) for a description of homologous recombination vectors]. The vector is introduced into an embryonic stem cell line (*e.g.*, by electroporation) and cells in which the introduced DNA has homologously recombined with the endogenous DNA are selected [see *e.g.,* Li *et al., Cell, 69*:915 (1992)]. The selected cells are then injected into a blastocyst of an animal (*e.g.,* a mouse or rat) to form aggregation chimeras [see *e.g.,* Bradley, in *Teratocarcinomas and Embryonic Stem Cells: A Practical Approach,* E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the PRO21074 polypeptide.

Nucleic acid encoding the PRO21074 polypeptides may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo.* It has already been shown that short antisense oligonucleotides can be imported into cells where they act as inhibitors, despite their low intracellular concentrations caused by their restricted uptake by the cell membrane. (Zamecnik *et al., Proc. Natl. Acad. Sci. USA* 83: 4143-4146 [1986]). The oligonucleotides can be modified to enhance their uptake, e.g. by substituting their negatively charged phosphodiester groups by uncharged groups.

There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau *et al., Trends in Bioteclanology* 11, 205-210 [1993]). In some situations it is desirable to provide the nucleic acid source with an agent that targets the target cells, such as an antibody specific for a cell surface membrane protein or the target cell, a ligand for a receptor on the target cell, *etc.* Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life.

The technique of receptor-mediated endocytosis is described, for example, by Wu *et al., J. Biol. Chem.* 262, 4429-4432 (1987); and Wagner *et al., Proc. Natl. Acad. Sci. USA* 87, 3410-3414 (1990). For review of gene marking and gene therapy protocols see Anderson *et al., Science* 256, 808-813 (1992).

The PRO21074 polypeptides described herein may also be employed as molecular weight markers for protein electrophoresis purposes.

The nucleic acid molecules encoding the PRO21074 polypeptides or fragments thereof described herein are useful for chromosome identification. In this regard, there exists an ongoing need to identify new chromosome markers, since relatively few chromosome marking reagents, based upon actual sequence data are presently available. Each PRO21074 nucleic acid molecule of the present invention can be used as a chromosome marker.

The PRO21074 polypeptides and nucleic acid molecules of the present invention may also be used for tissue typing, wherein the PRO21074 polypeptides of the present invention may be differentially expressed in one tissue as compared to another. PRO21074 nucleic acid molecules will find use for generating probes for PCR, Northern analysis, Southern analysis and Western analysis.

The PRO21074 polypeptides and nucleic acid molecules of the invention may also be useful in therapeutic applications related to the skin, *e.g.*, skin grafts, burns, skin healing, reduction in scarring, *etc.* The structural similarity between cartilage and skin as well as the importance of the extracellular matrix in skin cell biology suggests that the PRO21074 polypeptides and nucleic acid molecules disclosed herein would potentially also cause or result in skin repair.

The PRO21074 polypeptides described herein may also be employed as therapeutic agents. The PRO21074 polypeptides of the present invention can be formulated according to known methods to prepare pharmaceutically useful compositions, whereby the PRO21074 product hereof is combined in admixture with a pharmaceutically acceptable carrier vehicle. Therapeutic formulations are prepared for storage by mixing the active ingredient having the desired degree of purity with optional physiologically acceptable carriers, excipients or stabilizers (*Remington's Pharmaceutical Sciences* 16th edition, Osol, A. Ed. (1980)), in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as TWEEN™, PLURONICS™ or PEG.

The formulations to be used for *in vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

The route of administration is in accord with known methods, *e.g.* injection or infusion by intravenous, intraperitoneal, intracerebral, intramuscular, intraocular, intraarterial or intralesional routes, topical administration, or by sustained release systems.

Dosages and desired drug concentrations of pharmaceutical compositions of the present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" In Toxicokinetics and New Drug Development, Yacobi *et al*., Eds., Pergamon Press, New York 1989, pp. 42-96.

When *in vivo* administration of a PR021074 polypeptide or agonist or antagonist thereof is employed, normal dosage amounts may vary from about 10 ng/kg to up to 100 mg/kg of mammal body weight or more per day, preferably about 1 μg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

Where sustained-release administration of a PRO21074 polypeptide is desired in a formulation with release characteristics suitable for the treatment of any disease or disorder requiring administration of the PRO21074 polypeptide, microencapsulation of the PRO21074 polypeptide is contemplated. Microencapsulation of recombinant proteins for sustained release has been successfully performed with human growth hormone (rhGH), interferon- (rhIFN-), interleukin-2, and MN rgp120. Johnson *et al., Nat. Med.,* 2:795-799 (1996); Yasuda, *Biomed. Ther.,* 27:1221-1223 (1993); Hora *et al.*, *Bio/Technology,* 8:755-758 (1990); Cleland, "Design and Production of Single Immunization Vaccines Using Polylactide Polyglycolide Microsphere Systems," in *Vaccine Design: The Subunit and Adjuvant Approach,* Powell and Newman, eds, (Plenum Press: New York, 1995), pp. 439-462; WO 97/03692, WO 96/40072, WO 96/07399; and U.S.

Pat. No. 5,654,010.

The sustained-release formulations of these proteins were developed using poly-lactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. Lewis, "Controlled release of bioactive agents from lactide/glycolide polymer," in: M. Chasin and R. Langer (Eds.), *Biodegradable Polymers as Drug Delivery Systems* (Marcel Dekker: New York, 1990), pp. 1-41.

This invention encompasses methods of screening compounds to identify those that mimic the PRO21074 polypeptide (agonists) or prevent the effect of the PRO21074 polypeptide (antagonists). Screening assays for antagonist drug candidates are designed to identify compounds that bind or complex with the PRO21074 polypeptides encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a PRO21074 polypeptide encoded by a nucleic acid identified herein under conditions and for a time sufficient to allow these two components to interact.

In binding assays, the interaction is binding and the complex formed can be isolated or detected in the reaction mixture. In a particular embodiment, the PRO21074 polypeptide encoded by the gene identified herein or the drug candidate is immobilized on a solid phase, *e.g.*, on a microtiter plate, by covalent or non-covalent attachments. Non-covalent attachment generally is accomplished by coating the solid surface with a solution of the PRO21074 polypeptide and drying. Alternatively, an immobilized antibody, *e.g.,* a monoclonal antibody, specific for the PRO21074 polypeptide to be immobilized can be used to anchor it to a solid surface. The assay is performed by adding the non-immobilized component, which may be labeled by a detectable label, to the immobilized component, *e.g.*, the coated surface containing the anchored component. When the reaction is complete, the non-reacted components are removed, *e.g.*, by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular PRO21074 polypeptide encoded by a gene identified herein, its interaction with that polypeptide can be assayed by methods well known for detecting protein-protein interactions. Such assays include traditional approaches, such as, *e.g.*, cross-linking, co-immunoprecipitation, and co-purification through gradients or chromatographic columns. In addition, protein-protein interactions can be monitored by using a yeast-based genetic system described by Fields and co-workers (Fields and Song, *Nature (London)*, 340:245-246 (1989); Chien *et al., Proc. Natl. Acad Sci. USA,* 88:9578-9582 (1991)) as disclosed by Chevray and Nathans, *Proc. Natl. Acad Sci. USA,* 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a gene encoding a PRO21074 polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the PRO21074 polypeptide may be added to a cell along with the compound to be screened for a particular activity and the ability of the compound to inhibit the activity of interest in the presence of the PRO21074 polypeptide indicates that the compound is an antagonist to the PRO21074 polypeptide. Alternatively, antagonists may be detected by combining the PRO21074 polypeptide and a potential antagonist with membrane-bound PRO21074 polypeptide receptors or recombinant receptors under appropriate conditions for a competitive inhibition assay. The PRO21074 polypeptide can be labeled, such as by radioactivity, such that the number of PRO21074 polypeptide molecules bound to the receptor can be used to determine the effectiveness of the potential antagonist. The gene encoding the receptor can be identified by numerous methods known to those of skill in the art, for example, ligand panning and FACS sorting. Coligan *et al.*, *Current Protocols in Immun.,* 1(2): Chapter 5 (1991). Preferably, expression cloning is employed wherein polyadenylated RNA is prepared from a cell responsive to the PRO21074 polypeptide and a cDNA library created from this RNA is divided into pools and used to transfect COS cells or other cells that are not responsive to the PRO21074 polypeptide. Transfected cells that are grown on glass slides are exposed to labeled PRO21074 polypeptide. The PRO21074 polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, labeled PRO21074 polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor.

In another assay for antagonists, mammalian cells or a membrane preparation expressing the receptor would be incubated with labeled PRO21074 polypeptide in the presence of the candidate compound. The ability of the compound to enhance or block this interaction could then be measured.

More specific examples of potential antagonists include an oligonucleotide that binds to the fusions of immunoglobulin with PRO21074 polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the PRO21074 polypeptide that recognizes the receptor but imparts no effect, thereby competitively inhibiting the action of the PRO21074 polypeptide.

Another potential PRO21074 polypeptide antagonist is an antisense RNA or DNA construct prepared using antisense technology, where, *e.g.*, an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature PRO21074 polypeptides herein, is used to design an antisense RNA oligonucleotide of from about 10 to 40 base pairs in length. A DNA oligonucleotide is designed to be complementary to a region of the gene involved in transcription (triple helix - see Lee *et al., Nucl. Acids Res.,* 6:3073 (1979); Cooney *et al., Science,* 241: 456 (1988); Dervan *et al., Science,* 251:1360 (1991)), thereby preventing transcription and the production of the PRO21074 polypeptide. The antisense RNA oligonucleotide hybridizes to the mRNA *in vivo* and blocks translation of the mRNA molecule into the PRO21074 polypeptide (antisense - Okano, *Neurochem.*, 56:560 (1991); *Oligodeoxynucleotides as Antisense Inhibitors of Gene Expression* (CRC Press: Boca Raton, FL, 1988). The oligonucleotides described above can also be delivered to cells such that the antisense RNA or DNA may be expressed *in vivo* to inhibit production of the PRO21074 polypeptide. When antisense DNA is used, oligodeoxyribonucleotides derived from the translation-initiation site, *e.g.*, between about -10 and +10 positions of the target gene nucleotide sequence, are preferred.

Potential antagonists include small molecules that bind to the active site, the receptor binding site, or growth factor or other relevant binding site of the PRO21074 polypeptide, thereby blocking the normal biological activity of the PRO21074 polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e.g.,* Rossi, *Current Biology,* 4:469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, *e.g.*, PCT publication No. WO 97/33551, *supra*.

These small molecules can be identified by any one or more of the screening assays discussed hereinabove and/or by any other screening techniques well known for those skilled in the art. Based upon its sequence identity to other known polypeptides, potential uses for the PRO21074 polypeptide include use as a regulatory agent in peptide degradation, or in the regulation of matrix protein binding.

F. Anti-PRO21074 Antibodies

The present invention further provides anti-PRO21074 antibodies. Exemplary antibodies include polyclonal, monoclonal, humanized, bispecific, and heteroconjugate antibodies.

1. Polyclonal Antibodies

The anti-PRO21074 antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO21074 polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

2. Monoclonal Antibodies

The anti-PRO21074 antibodies may, alternatively, be monoclonal antibodies. Monoclonal antibodies may be prepared using hybridoma methods, such as those described by Kohler and Milstein, *Nature,* 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO21074 polypeptide or a fusion protein thereof. Generally, either peripheral blood lymphocytes ("PBLs") are used if cells of human origin are desired, or spleen cells or lymph nodE cells are used if non-human mammalian sources are desired. The lymphocytes are then fused with an immortalized cell line using a suitable fusing agent, such as polyethylene glycol, to form a hybridoma cell [Goding, *Monoclonal Antibodies: Principles and Practice,* Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, *J. Immunol.*, 133:3001 (1984); Brodeur *et al., Monoclonal Antibody Production Techniques and Applications*, Marcel Dekker, Inc., New York, (1987) pp. 51-63].

The culture medium in which the hybridoma cells are cultured can then be assayed for the presence of monoclonal antibodies directed against PRO21074. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, *Anal. Biochem.*, 107:220 (1980).

After the desired hybridoma cells are identified, the clones may be subcloned by limiting dilution procedures and grown by standard methods [Goding, *supra*]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (*e.g.*, by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison *et al., supra*] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

3. Human and Humanized Antibodies

The anti-PRO21074 antibodies of the invention may further comprise humanized antibodies or human antibodies. Humanized forms of non-human (*e.g.*, murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones *et al.*, *Nature,* 321:522-525 (1986); Riechmann *et al., Nature*, 332:323-329 (1988); and Presta, *Curr. Op. Struct. Biol.*, 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones *et al., Nature,* 321: 522-525 (1986); Riechmann *et al.*, *Nature*, 332:323-327 (1988); Verhoeyen *et al.*, *Science,* 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, *J. Mol. Biol.*, 227:381 (1991); Marks *et al.*, *J. Mol. Biol.*, 222:581 (1991)]. The techniques of Cole *et al.* and Boerner *et al.* are also available for the preparation of human monoclonal antibodies (Cole *et al.*, *Monoclonal Antibodies and Cancer Therapy*, Alan R. Liss, p. 77 (1985) and Boerner *et al.*, *J. Immunol.*, 147(1): 86-95 (1991)]. Similarly, human antibodies can be made by introducing of human immunoglobulin loci into transgenic animals, *e.g.*, mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks *et al., Bio/Technology* 10, 779-783 (1992); Lonberg *et al., Nature* 368 856-859 (1994); Morrison, *Nature* 368, 812-13 (1994); Fishwild *et al., Nature Biotechnology* 14, 845-51 (1996); Neuberger, *Nature Biotechnology* 14, 826 (1996); Lonberg and Huszar, *Intern. Rev. Immunol.* 13 65-93 (1995).

4. Bispecific Antibodies

Bispecific antibodies are monoclonal, preferably human or humanized, antibodies that have binding specificities for at least two different antigens. In the present case, one of the binding specificities is for the PRO21074, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, *Nature,* 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker *et al., EMBO J.,* 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh *et al., Methods in Enzymology,* 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (*e.g.* $F(ab')_2$ bispecific antibodies). Techniques for generating bispecific antibodies from antibody fragments have been described in the literature. For example, bispecific antibodies can be prepared can be prepared using chemical linkage. Brennan *et al., Science* 229:81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby *et al., J. Exp. Med.* 175:217-225 (1992) describe the production of a fully humanized bispecific antibody $F(ab')_2$ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny *et al., J. Immunol.* 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger *et al., Proc. Natl. Acad. Sci. USA* 90:6444-6448 (1993) has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See, Gruber *et al., J. Immunol.* 152:5368 (1994).

Antibodies with more than two valencies are contemplated. For example, trispecific antibodies can be prepared. Tutt *et*

*al., J. Immunol.* 147:60 (1991).

Exemplary bispecific antibodies may bind to two different epitopes on a given PRO21074 polypeptide herein. Alternatively, an anti-PRO21074 polypeptide arm may be combined with an arm which binds to a triggering molecule on a leukocyte such as a T-cell receptor molecule (*e.g.*, CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO21074 polypeptide. Bispecific antibodies may also be used to localize cytotoxic agents to cells which express a particular PRO21074 polypeptide. These antibodies possess a PRO21074-binding arm and an arm which binds a cytotoxic agent or a radionuclide chelator, such as EOTUBE, DPTA, DOTA, or TETA. Another bispecific antibody of interest binds the PRO21074 polypeptide and further binds tissue factor (TF).

5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

6. Effector Function Engineering

It may be desirable to modify the antibody of the invention with respect to effector function, so as to enhance, *e.g.*, the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron *et al., J. Exp Med.*, 176: 1191-1195 (1992) and Shopes, *J. Immunol.*, 148: 2918-2922 (1992). Homodimeric antibodies with enhanced antitumor activity may also be prepared using heterobifunctional cross-linkers as described in Wolff *et al. Cancer Research*, 53: 2560-2565 (1993). Alternatively, an antibody can be engineered that has dual Fc regions and may thereby have enhanced complement lysis and ADCC capabilities. See Stevenson *et al., Anti-Cancer Drug Design*, 3: 219-230 (1989).

7. Immunoconjugates

The invention also pertains to immunoconjugates comprising an antibody conjugated to a cytotoxic agent such as a chemotherapeutic agent, toxin (*e.g.*, an enzymatically active toxin of bacterial, fungal, plant, or animal origin, or fragments thereof), or a radioactive isotope (*i.e.*, a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}Bi$, $^{131}I$, $^{131}In$, $^{90}Y$, and $^{186}Re$.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta *et al., Science*, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) that is conjugated to a cytotoxic agent (*e.g.*, a radionucleotide).

8. Immunoliposomes

The antibodies disclosed herein may also be formulated as immunoliposomes. Liposomes containing the antibody are prepared by methods known in the art, such as described in Epstein *et al., Proc. Natl. Acad. Sci. USA,* 82: 3688 (1985); Hwang *et al., Proc. Natl Acad. Sci. USA,* 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin *et al., J. Biol. Chem.*, 257: 286-288 (1982) via a disulfide-interchange reaction. A chemotherapeutic agent (such as Doxorubicin) is optionally contained within the liposome. See Gabizon *et al., J. National Cancer Inst.*, 81(19): 1484 (1989).

9. Pharmaceutical Compositions of Antibodies

Antibodies specifically binding a PRO21074 polypeptide identified herein, as well as other molecules identified by the screening assays disclosed hereinbefore, can be administered for the treatment of various disorders in the form of pharmaceutical compositions.

If the PRO21074 polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e.g.,* Marasco *et al., Proc. Natl. Acad. Sci. USA*, 90: 7889-7893 (1993).

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles, and nanocapsules) or in macroemulsions. Such techniques are disclosed in *Remington's Pharmaceutical Sciences, supra.*

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g.*, films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and $\gamma$ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37 °C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

G. Uses for anti-PRO21074 Antibodies

The anti-PRO21074 antibodies of the invention have various utilities. For example, anti-PRO21074 antibodies may be used in diagnostic assays for PRO21074, *e.g.,* detecting its expression in specific cells, tissues, synovial fluid, plasma/serum, or urine. For example, the level of PRO21074 expression in articular cartilage by an anti-PRO21074 antibody may be used as a marker for the presence and severity of a cartilaginous disorder. Various diagnostic assay techniques known in the art may be used, such as competitive binding assays, direct or indirect sandwich assays and immunoprecipitation assays conducted in either heterogeneous or homogeneous phases [Zola, *Monoclonal Antibodies: A Manual of Techniques,* CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as $^{3}H$, $^{14}C$, $^{32}P$, $^{35}S$, or $^{125}I$, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter *et al., Nature,* 144:945 (1962); David *et al., Biochemistry*, 13:1014 (1974); Pain *et al., J. Immunol. Meth.*, 40:219 (1981); and Nygren, *J. Histochem. and Cytochem.*, 30:407 (1982).

Anti-PRO21074 antibodies also are useful for the affinity purification of PRO21074 from recombinant cell culture or natural sources. In this process, the antibodies against PRO21074 are immobilized on a suitable support, such a Sephadex resin or filter paper, using methods well known in the art. The immobilized antibody then is contacted with a sample containing the PRO21074 to be purified, and thereafter the support is washed with a suitable solvent that will remove substantially all the material in the sample except the PRO21074, which is bound to the immobilized antibody. Finally, the support is washed with another suitable solvent that will release the PRO21074 from the antibody.

H. Pharmaceutical Compositions and Dosages

The PRO21074 polypeptides and antagonists usable with the method of the invention can be adminstered for the treatment of cartilaginous disorders in the form of a pharmaceutical composition. Additionally, lipofections or liposomes can be used to deliver the PRO21074 polypeptide or antagonist.

Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (see, *e.g.*, Marasco *et al*., *Proc. Natl. Acad. Sci. USA* 90: 7889-7893 [1993]).

Therapeutic formulations are prepared for storage by mixing the active ingredient having the desired degree of purity with optional pharmaceutically acceptable carriers, excipients or stabilizers (*Remington's Pharmaceutical Sciences* 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as TWEEN™, PLURONICS™ or polyethylene glycol (PEG).

In order for the formulations to be used for *in vivo* administration, they must be sterile. The formulation may be rendered sterile by filtration through sterile filtration membranes, prior to or following lyophilization and reconstitution. The therapeutic compositions herein generally are placed into a container having a sterile access port, for example, an intravenous solution bag or vial having a stopper pierceable by a hypodermic injection needle.

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The route of adminstration is in accordance with known and accepted methods, *e.g.*, injection or infustion by intravenous, intraperitoneal; intramuscular, intraarterial, intralesional or intraarticular routes, topical administriaton, by sustained release or extended-release means. Optionally, the active compound of formulation is injected directly into the afflicted cartilaginous region or articular joint.

The active agents of the present invention, *e.g.* antibodies, are administered to a mammal, preferably a human, in accord with known methods, such as intravenous administration as a bolus or by continuous infusion over a period of time, by intramuscular, intraperitoneal, intracerebral, intracerobrospinal, subcutaneous, intra-articular, intrasynovial, intrathecal, intraoccular, intralesional, oral, topical, inhalation or through sustained release.

Dosages and desired drug concentration of pharmaceutical compositions of the present invention may vary depending on the particular use envisioned. The determination of the appropriate dosage or route of administration is well within the skill of an ordinary artisan. Animal experiments provide reliable guidance for the determination of effective doses for human therapy. Interspecies scaling of effective doses can be performed following the principles laid down by Mordenti, J. and Chappell, W. "The Use of Interspecies Scaling in Toxicokinetics," In *Toxicokinetics and New Drug Development,* Yacobi *et al.,* Eds, Pergamon Press, New York 1989, pp.42-46.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in *Remington's Pharmaceutical Sciences* 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, *e.g.* films, or microcapsules. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and γ-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. Microencapsulation of recombinant proteins for sustained release has been successfully performed with human growth hormone (rhGH), interferon- (rhIFN-), interleukin-2, and MN rpg 120. Johnson *et al., Nat. Med.* 2: 795-799 (1996); Yasuda *et al.*, *Biomed. Ther.* 27: 1221-1223 (1993); Hora *et al., Bio/Technology* 8: 755-758 (1990); Cleland, "Design and Production of Single Immunization Vaccines Using Polylactide Polyglycolide Microsphere Systems," in *Vaccine Design: The Subunit und Adjuvant Approach*, Powell and Newman, eds., (Penum Press: New York, 1995), pp. 439-462; WO 97/03692; WO 96/40072; WO 96/07399; and U.S. Pat. No. 5,654,010.

The sustained-release formulations of these proteins may be developed using poly lactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. Lewis, "Controlled release of bioactive agents from lactide/glycolide polymer", in *Biodegradable Polymers as Drug Delivery Systems* (Marcel Dekker; New York, 1990), M. Chasin and R. Langer (Eds.) pp. 1-41.

While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

When *in vivo* administration of the PRO21074 or PRO21074 antagonists are used, normal dosage amounts may vary from about 10 ng/kg up to about 100 mg/kg of mammal body weight or more per day, preferably about 1 mg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is within the scope of the invention that different formulations will be effective for different treatments and different disorders, and that adminstration intended to treat a specific organ or tissue may necessitate delivery in a manner different from that to another organ or tissue. Moreover, dosages may be administered by one or more separate administrations, or by continuous infusion. For repeated administrations over several days or longer, depending on the condition, the treatment is sustained until a desired suppression of disease symptoms occurs. However, other dosage regimens may be useful. The progress of this therapy is easily monitored by conventional techniques and assays.

I. Methods of Treatment

For the prevention or treatment of disease, the appropriate dosage of an active agent, will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments.

It is contemplated that the polypeptides, antibodies and other active compounds of the present invention may be used to treat various cartilaginous disorders. Exemplary conditions or disorders to be treated with the polypeptides of the invention, include, but are not limited to systemic lupus erythematosis, rheumatoid arthritis, juvenile chronic arthritis, osteoarthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft-versus-host-disease.

In systemic lupus erythematosus, the central mediator of disease is the production of auto-reactive antibodies to self proteins/tissues and the subsequent generation of immune-mediated inflammation. These antibodies either directly or indirectly mediate tissue injury. Although T lymphocytes have not been shown to be directly involved in tissue damage, T lymphocytes are required for the development of auto-reactive antibodies. The genesis of the disease is thus T lymphocyte dependent. Multiple organs and systems are affected clinically including kidney, lung, musculoskeletal system, mucocutaneous, eye, central nervous system, cardiovascular system, gastrointestinal tract, bone marrow and blood.

Rheumatoid arthritis (RA) is a chronic systemic autoimmune inflammatory disease that affects the synovial membrane of multiple joints and which results in injury to the articular cartilage. The pathogenesis is T lymphocyte dependent and is associated with the production of rheumatoid factors, auto-antibodies directed against endogenous proteins, with the resultant formation of immune complexes that attain high levels in joint fluid and blood. These complexes may induce infiltration by lymphocytes, monocytes, and neutrophils into the synovial compartment. Tissues affected are primarily the joints, often in symmetrical pattern. However, disease outside the joints occurs in two major forms. In one form, typical lesions are pulmonary fibrosis, vasculitis, and cutaneous ulcers. The second form is the so-called Felty's syndrome which occurs late in the RA disease course, sometimes after joint disease has become quiescent, and involves the presence of neutropenia, thrombocytopenia and splenomegaly. This can be accompanied by vasculitis in multiple organs and occurrence of infarcts, skin ulcers and gangrene. Patients often also develop rheumatoid nodules in the subcutis tissue overlying affected joints; in late stages, the nodules have necrotic centers surrounded by a mixed inflammatory cellular infiltrate. Other manifestations which can occur in RA include: pericarditis, pleuritis, coronary arteritis, intestitial pneumonitis with pulmonary fibrosis, keratoconjunctivitis sicca, and rheumatoid nodules.

Juvenile chronic arthritis is a chronic idiopathic inflammatory disease which begins often at less than 16 years of age and which has some similarities to RA. Some patients which are rheumatoid factor positive are classified as juvenile rheumatoid arthritis. The disease is subclassified into three major categories: pauciarticular, polyarticular, and systemic. The arthritis can be severe and leads to joint ankylosis and retarded growth. Other manifestations can include chronic anterior uveitis and systemic amyloidosis.

Spondyloarthropathies are a group of disorders with some common clinical features and the common association with the expression of HLA-B27 gene product. The disorders include: ankylosing sponylitis, Reiter's syndrome (reactive arthritis), arthritis associated with inflammatory bowel disease, spondylitis associated with psoriasis, juvenile onset spondyloarthropathy and undifferentiated spondyloarthropathy. Distinguishing features include sacroileitis with or without spondylitis; inflammatory asymmetric arthritis; association with HLA-B27 (a serologically defined allele of the HLA-B locus of class I MHC); ocular inflammation, and absence of autoantibodies associated with other rheumatoid disease. The cell most implicated as key to induction of the disease is the CD8+ T lymphocyte, a cell which targets antigen presented by class I MHC molecules. CD8+ T cells may react against the class I MHC allele HLA-B27 as if it were a foreign peptide expressed by MHC class I molecules. It has been hypothesized that an epitope of HLA-B27 may mimic a bacterial or other microbial antigenic epitope and thus induce a CD8+ T cells response.

Systemic sclerosis (scleroderma) has an unknown etiology. A hallmark of the disease is induration of the skin which is likely induced by an active inflammatory process. Scleroderma can be localized or systemic. Vascular lesions are common, and endothelial cell injury in the microvasculature is an early and important event in the development of systemic sclerosis. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. Other organs may also, be involved. In the gastrointestinal tract, smooth muscle atrophy and fibrosis can result in abnormal peristalsis/motility. In the kidney, concentric subendothelial intimal proliferation affecting small arcuate and interlobular arteries can result in reduced renal cortical blood flow and thus proteinuria, azotemia and hypertension. In skeletal and cardiac muscle, atrophy, interstitial fibrosis/scarring, and necrosis can occur. Finally, the lung can have interstitial pneumonitis and interstitial fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components involved in protein synthesis.

Sjögren's syndrome is the result of immune-mediated inflammation and subsequent functional destruction of the tear glands and salivary glands. The disease can be associated with or accompanied by inflammatory connective tissue diseases. The disease is associated with autoantibody production against Ro and La antigens, both of which are small RNA-protein complexes. Lesions result in keratoconjunctivitis sicca, xerostomia, with other manifestations or associations including bilary cirrhosis, peripheral or sensory neuropathy, and palpable purpura.

Systemic vasculitis are diseases in which the primary lesion is inflammation and subsequent damage to blood vessels which results in ischemia/necrosis/degeneration to tissues supplied by the affected vessels and eventual end-organ dysfunction in some cases. Vasculitides can also occur as a secondary lesion or sequelae to other immune-inflammatory mediated diseases such as rheumatoid arthritis, systemic sclerosis, etc., particularly in diseases also associated with the formation of immune complexes. Diseases in the primary systemic vasculitis group include: systemic necrotizing vasculitis: polyarteritis nodosa, allergic angiitis and granulomatosis, polyangiitis; Wegener's granulomatosis; lymphomatoid granulomatosis; and giant cell arteritis. Miscellaneous vasculitides include: mucocutaneous lymph node syndrome (MLNS or Kawasaki's disease), isolated CNS vasculitis, Behet's disease, thromboangiitis obliterans (Buerger's disease) and cutaneous necrotizing venulitis. The pathogenic mechanism of most of the types of vasculitis listed is believed to be primarily due to the deposition of immunoglobulin complexes in the vessel wall and subsequent induction of an inflammatory response either via ADCC, complement activation, or both.

Sarcoidosis is a condition of unknown etiology which is characterized by the presence of epithelioid granulomas in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

In autoimmune thrombocytopenia including thrombocytopenic purpura, and immune-mediated thrombocytopenia in other clinical settings, platelet destruction/removal occurs as a result of either antibody or complement attaching to platelets and subsequent removal by complement lysis, ADCC or FC-receptor mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase).

Diabetes mellitus is a genetic disorder of metabolism of carbohydrate, protein and fat associated with a relative or absolute insufficiency of insulin secretion and with various degrees of insulin resistance. In its fully developed clinical expression, it is characterized by fasting hyperglycemia and in the majority of long-standing patients by atherosclerotic and microangiopathic vascular disease and neuropathy. Differences between various forms of the disease are expressed in terms of cause and pathogenesis, natural history, and response to treatment. Thus, diabetes is not a single disease but a syndrome.

Type I, or insulin-dependent diabetes mellitus (IDDM) occurs in approximately 10 per cent of all diabetic patients in the Western world. Type I diabetes mellitus or insulin-dependent diabetes is the autoimmune destruction of pancreatic islet $\beta$-cells; this destruction is mediated by auto-antibodies and auto-reactive T cells. Antibodies to insulin or the insulin receptor can also produce the phenotype of insulin-non-responsiveness.

Classically, this type of disease occurs most commonly in childhood and adolescence; however, it can be recognized and become symptomatic at any age. In the most common type of IDDM (Type IA), it has been postulated that environmental (acquired) factors such as certain viral infections, and possibly chemical agents, superimposed on genetic factors, may lead to cell-mediated autoimmune destruction of $\beta$ cells. Thus, genetically determined abnormal immune, responses (linked to HLA associations) characterized by cell mediated and humoral autoimmunity are thought to play a pathogenetic role after evocation by an environmental factor. A second type of IDDM (Type IB) is believed to be due to primary autoimmunity. These patients have associated autoimmune endocrine diseases such as Hashimoto's thyroiditis, Graves' disease, Addison's disease, primary gonadal failure, and associated nonendocrine autoimmune diseases such as pernicious anemia, connective tissue diseases, celiac disease and myasthenia gravis. Insulin dependency implies that administration of insulin is essential to prevent spontaneous ketosis, coma, and death. However, even with insulin treatment, diabetic patients can still have many of the additional problems associated with diabetes, *i.e.* connective tissue disorders, neuropathy, *etc.*

The second type of diabetes, Type II or non-insulin-dependent diabetes mellitus (NIDDM), present in approximately 90% of all diabetics, also has a genetic basis. Patients with type II diabetes may have a body weight that ranges from normal to excessive. Obesity and pathological insulin resistance are by no means essential in the evolution of NIDDM. In the majority of patients with NIDDM, a diagnosis is made in middle age. Patients with NIDDM are non-insulin-dependent for prevention of ketosis, but they may require insulin for correction of symptomatic or nonsymptomatic persistent fasting hyperglycemia if this cannot bye achieved with the use of diet or oral agents. Thus, therapeutic administration of insulin does not distinguish between IDDM and NIDDM. In some NIDDM families, the insulin secretory responses to glucose are so low that they may resemble those of early Type I diabetes at any point in time. Early in its natural history, the insulin secretory defect and insulin resistance may be reversible by treatment (*i.e.* weight reduction) with normalization of glucose tolerance. The typical chronic complications of diabetes, namely macroangiopathy, microangiopathy, neuropathy, and cataracts seen in IDDM are seen in NIDDM as well.

Other types of diabetes include entities secondary to or associated with certain other conditions or syndromes. Diabetes may be secondary to pancreatic disease or removal of pancreatic tissue; endocrine diseases such as acromegaly, Cushing's syndrome, pheochromocytoma, glucagonoma, somatostatinoma, or primary aldosteronism; the administration of hormones, causing hyperglycemia; and the administration of certain drugs (*i.e.* antihypertensive drugs, thiazide diuretics, preparations containing estrogen, psychoactive drugs, sympathomimetic agents). Diabetes may be associated with a large number of genetic syndromes. Finally, diabetes may be associated with genetic defects of the insulin receptor or due to antibodies to the insulin receptor with or without associated immune disorders.

Immune mediated renal diseases, including glomerulonephritis and tubulointerstitial nephritis, are the result of antibody or T lymphocyte mediated injury to renal tissue either directly as a result of the production of autoreactive antibodies or T cells against renal antigens or indirectly as a result of the deposition of antibodies and/or immune complexes in the kidney that are reactive against other, non-renal antigens. Thus, other immune-mediated diseases that result in the formation of immune-complexes can also induce immune mediated renal disease as an indirect sequelae. Both direct and indirect immune mechanisms result in inflammatory response that produces/induces lesion development in renal tissues with resultant organ function impairment and in some cases progression to renal failure. Both humoral and cellular immune mechanisms can be involved in the pathogenesis of lesions.

Demyelinating diseases of the central and peripheral nervous systems, including multiple sclerosis; idiopathic demyelinating polyneuropathy or Guillain-Barré syndrome; and Chronic Inflammatory Demyelinating Polyneuropathy, are believed to have an autoimmune basis and result in nerve demyelination as a result of damage caused to oligodendrocytes or to myelin directly. In MS there is evidence to suggest that disease induction and progression is dependent on T lymphocytes. Multiple sclerosis is a demyelinating disease that is T lymphocyte-dependent and has either a relapsing-remitting course or a chronic progressive course. The etiology is unknown; however, viral infections, genetic predisposition, environment, and autoimmunity all contribute. Lesions contain infiltrates of predominantly T lymphocyte mediated, microglial cells and infiltrating macrophages; CD4+T lymphocytes are the predominant cell type at lesions. The mechanism of oligodendrocyte cell death and subsequent demyelination is not known but is likely T lymphocyte driven.

Inflammatory and fibrotic lung disease, including eosinophilic pneumonias, idiopathic pulmonary fibrosis, and hypersensitivity pneumonitis may involve a disregulated immune-inflammatory response. Inhibition of that response would be of therapeutic benefit and within the scope of the invention.

Autoimmune or immune-mediated skin disease, including bullous skin diseases, erythema multiforme, and contact dermatitis are mediated by auto-antibodies, the genesis of which is T lymphocyte-dependent.

Psoriasis is a T lymphocyte-mediated inflammatory disease. Lesions contain infiltrates of T lymphocytes, macrophages and antigen processing cells, and some neutrophils.

Transplantation associated diseases, including Graft rejection and Graft-Versus-Host-Disease (GVHD) are T lymphocyte-dependent; inhibition of T lymphocyte function is ameliorative.

Other diseases in which intervention of the immune and/or inflammatory response have benefit are infectious disease including but not limited to viral infection (including but not limited to AIDS, hepatitis A, B, C, D, E and herpes) bacterial infection, fungal infections, and protozoal and parasitic infections (molecules (or derivatives/agonists) which stimulate the MLR can be utilized therapeutically to enhance the immune response to infectious agents), diseases of immunodeficiency (molecules/derivatives/agonists) which stimulate the MLR can be utilized therapeutically to enhance the immune response for conditions of inherited, acquired, infectious induced (as in HIV infection), or iatrogenic (*i.e.* as from chemotherapy) immunodeficiency, and neoplasia.

Additionally, inhibition of molecules with proinflammatory properties may have therapeutic benefit in reperfusion injury; stroke; myocardial infarction; atherosclerosis; acute lung injury; hemorrhagic shock; burn; sepsis/septic shock; acute tubular necrosis; endometriosis; degenerative joint disease and pancreatis.

The compounds of the present invention, *e.g.*, polypeptides or antibodies, are administered to a mammal, preferably a human, in accord with known methods, such as intravenous administration as a bolus or by continuous infusion over a period of time, by intramuscular, intraperitoneal, intracerebrospinal, subcutaneous, intra-articular, intrasynovial, intrathecal, oral, topical, or inhalation (intranasal, intrapulmonary) routes.

It may be desirable to also administer antibodies against other immune disease associated or tumor associated antigens, such as antibodies which bind to CD20, CD11a, CD 40, CD18, ErbB2, EGFR, ErbB3, ErbB4, or vascular endothelial growth factor (VEGF). Alternatively, or in addition, two or more antibodies binding the same or two or more different antigens disclosed herein may be coadministered to the patient. Sometimes, it may be beneficial to also administer one or more cytokines to the patient. In one embodiment, the polypeptides of the invention are coadministered with a growth inhibitory agent. For example, the growth inhibitory agent may be administered first, followed by a polypeptide of the invention. However, simultaneous administration or administration first is also contemplated. Suitable dosages for the growth inhibitory agent are those presently used and may be lowered due to the combined action (synergy) of the growth inhibitory agent and the polypeptide of the invention.

For the treatment or reduction in the severity of immune related disease, the appropriate dosage of an a compound of the invention will depend on the type of disease to be treated, as defined above, the severity and course of the disease, whether the agent is administered for preventive or therapeutic purposes, previous therapy, the patient's clinical history and response to the compound, and the discretion of the attending physician. The compound is suitably administered to the patient at one time or over a series of treatments.

J. Articles of Manufacture

In another embodiment of the invention, an article of manufacture containing materials useful for the diagnosis or treatment of the disorders described above is provided. The article of manufacture comprises a container and an instruction. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is typically a PRO21074 polypeptide or antagonist thereof. The composition can further comprise any or multiple ingredients disclosed herein. The instruction on, or associated with, the container indicates that the composition is used for diagnosing or treating the condition of choice. For example, the instruction could indicate that the composition is effective for the treatment of osteoarthritis, rheumatoid arthritis or any other cartilaginous disorder. The article of manufacture may further comprise a second container comprising a pharmaceutically acceptable buffer, such as phosphate-buffered saline; Ringer's solution and dextrose solution. It may further include other materials desirable from a commercial and user standpoint, including other buffers, diluents, filters, needles, syringes, and package inserts with instructions for use.

The following examples are offered for illustrative purposes only, and are not intended to limit the scope of the present invention in any way.

## EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1

### Isolation of cDNA Clones Encoding a Human PRO21074

The extracellular domain (ECD) sequences (including the secretion signal sequence, if any) from about 950 known secreted proteins from the Swiss-Prot public database were used to search sequence databases. The databases included public databases (*e.g.*, GenBank) In this instance, genomic DNA sequence from GenBank was analyzed using the gene preditiction program GENSCAN, licenced from Stanford University. GENSCAN analysis predicts gene coding regions, creating sequences which can be subjected to the ECD search. The search was performed using the computer program BLAST or BLAST2 [Altschul *et al., Methods in Enzymology,* 266:460-480 (1996)] as a comparison of the ECD protein sequences to a 6 frame translation of the sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington) if necessary.

A consensus DNA sequence was assembled. This consensus sequence is herein designated DNA144306. Based on the DNA144306 consensus sequence, oligonucleotides were synthesized: 1) to identify by PCR a cDNA library that contained the sequence of interest, and 2) for use as probes to isolate a clone of the full-length coding sequence for PRO21074. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel *et al.*, Current Protocols in Molecular Biology, *supra,* with the PCR primer pair. A positive library was then used to isolate clones encoding the gene of interest using the probe oligonucleotide and one of the primer pairs.

PCR primers (forward and reverse) were synthesized:

forward PCR primer 5'-AGAGGCCTTCCACCTATGGAGAAGAATGT-3' (SEQ ID NO:4)
reverse PCR primer 5'-GGGGGCAAAGTAGTGAATGAAATAGTC- 3' (SEQ ID NO:5)

Additionally, a synthetic oligonucleotide hybridization probe was constructed from the consensus DNA144306 sequence which had the following nucleotide sequence:

hybridization probe 5'-GTTATTGACATAAGTGGCTTCATGTTTGGTACCAAGATGAAACAGGA- 3' (SEQ ID NO:6).

A pool of 50 different human cDNA libraries from various tissues was used in cloning. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes *et al., Science,* 253:1278-1280 (1991)) in the unique XhoI and NotI sites.

DNA sequencing of the clones isolated as described above gave the full-length DNA sequence for a full-length PRO21074 polypeptide (designated herein as DNA153576-2925 [Figure 1, SEQ ID NO: 1]) and the derived protein sequence for that PRO21074 polypeptide.

The full length clone identified above contained a single open reading frame with an apparent translational initiation site at nucleotide positions 31-33 and a stop signal at nucleotide positions 3970-3972 (Figure 1, SEQ ID NO: 1). The predicted polypeptide precursor is 1313 amino acids long, has a calculated molecular weight of approximately 143187 daltons and an estimated pI of approximately 9.27. Analysis of the full-length PRO21074 sequence shown in Figure 2 (SEQ ID NO: 2) evidences the presence of a variety of important polypeptide domains as shown in Figure 2, wherein the locations given for those important polypeptide domains are approximate as described above. Clone DNA153576-2925 has been deposited with ATCC on May 23, 2000 and is assigned ATCC Deposit No. 1907-PTA.

An analysis of the Dayhoff database (version 35.45 SwissProt 35), using the ALIGN-2 sequence alignment analysis of the full-length sequence shown in Figure 2 (SEQ ID NO:2), evidenced sequence identity between the PRO21074 amino acid sequence and the following Dayhoff sequences: HS1409_1; P_Y17829; P_Y32169; ITH3_HUMAN; ITH4_HUMAN; ITH2_HUMAN; ITH1_HUMAN; AF119856_1; P_Y48475; HSU70136_1..

EXAMPLE 2

Use of PRO21074 as a hybridization probe

The following method describes use of a nucleotide sequence encoding PRO21074 as a hybridization probe.

DNA comprising all or part of the gene encoding PRO21074 is employed as a probe to screen for homologous DNAs (such as those encoding naturally-occurring variants of PRO21074) in human tissue cDNA libraries or human tissue genomic libraries.

Hybridization and washing of filters containing either library DNAs is performed under the following high stringency conditions. Hybridization of radiolabeled PRO21074-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PRO21074 can then be identified using standard techniques known in the art.

DNA comprising any part of gene encoding PRO21074 can also be used as a probe to detect sites of expression in tissue sections.

EXAMPLE 3

Expression of PRO21074 in *E. coli*

This example illustrates preparation of an unglycosylated form of PRO21074 by recombinant expression in *E. coli.*

The DNA sequence encoding PRO21074 is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli;* see Bolivar *et al., Gene*, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO21074 coding region, lambda transcriptional terminator, and an argU gene. Additionally, the vector may include at least not insignificant portions of the untranslated 5' and 3' sections of the native sequence PRO21074 encoding nucleic acid.

The ligation mixture is then used to transform a selected *E. coli* strain using the methods described in Sambrook *et al., supra*. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO21074 protein can then be purified using a metal chelating column under conditions that allow tight binding of the protein.

PRO21074 may be expressed in *E. coli* in a poly-His tagged form, using the following procedure. The DNA encoding PRO21074 is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) lon galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g $(NH_4)_2SO_4$, 0.71 g sodium citrate•$2H_2O$, 1.07 g KCl, 5.36 g Difco yeast extract, 5.36 g Sheffield hycase SF in 500 mL water, as well as 110 mM MPOS, pH 7.3, 0.55% (w/v) glucose and 7 mM $MgSO_4$) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1% TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO21074 polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

EXAMPLE 4

Expression of PRO21074 in mammalian cells

This example illustrates preparation of a potentially glycosylated form of PRO21074 by recombinant expression in mammalian cells.

The vector, pRK5 (see EP 307,247, published March 15, 1989), is employed as the expression vector. Optionally, the PRO21074 DNA is ligated into pRK5 with selected restriction enzymes to allow insertion of the PRO21074 DNA using ligation methods such as described in Sambrook *et al., supra*. The resulting vector is called pRK5-PRO21074.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 μg pRK5-PRO21074 DNA is mixed with about 1 μg DNA encoding the VA RNA gene [Thimmappaya *et al*., *Cell,* 31:543 (1982)] and dissolved in 500 μl of 1 mM Tris-HCl, 0.1 mM EDTA, 0.227 M $CaCl_2$. To this mixture is added, dropwise, 500 μl of 50 mM HEPES (pH.7.35), 280 mM NaCl, 1.5 mM $NaPO_4$, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 μCi/ml $^{35}$S-cysteine and 200 μCi/ml $^{35}$S-methionine. After a 12 hour incubation, the conditioned medium is collected, concentrated on a spin filter, and loaded onto a 15% SDS gel. The processed gel may be dried and exposed to film for a selected period of time to reveal the presence of PRO21074 polypeptide. The cultures containing transfected cells may undergo further incubation (in serum free medium) and the medium is tested in selected bioassays.

In an alternative technique, PRO21074 may be introduced into 293 cells transiently using the dextran sulfate method described by Somparyrac *et al., Proc. Natl. Acad Sci.*, 12:7575 (1981). 293 cells are grown to maximal density in a spinner flask and 700 μg pRK5-PRO21074 DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 μg/ml bovine insulin and 0.1 μg/ml bovine trassferrin. After about four days, the conditioned media is centrifuged and filtered to remove cells and debris. The sample containing expressed PRO21074 can then be concentrated and purified by any selected method, such as dialysis and/or column chromatography.

In another embodiment, PRO21074 can be expressed in CHO cells. The pRK5-PRO21074 can be transfected into CHO cells using known reagents such as $CaPO_4$ or DEAE-dextran. As described above, the cell cultures can be incubated, and the medium replaced with culture medium (alone) or medium containing a radiolabel such as $^{35}$S-methionine. After determining the presence of PRO21074 polypeptide, the culture medium may be replaced with serum free medium. Preferably, the cultures are incubated for about 6 days, and then the conditioned medium is harvested. The medium containing the expressed PRO21074 can then be concentrated and purified by any selected method.

Epitope-tagged PRO21074 may also be expressed in host CHO cells. The PRO21074 may be subcloned out of the pRK5 vector. The subclone insert can undergo PCR to fuse in frame with a selected epitope tag such as a poly-his tag into a Baculovirus expression vector. The poly-his tagged PRO21074 insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO21074 can then be concentrated and purified by any selected method, such as by $Ni^{2+}$-chelate affinity chromatography.

PRO21074 may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG1 constant region sequence containing the hinge, CH2 and CH2 domains and/or is a poly-His tagged form.

Following PCR amplification, the respective DNAs are subcloned in a CHO expression vector using standard techniques as described in Ausubel *et al*., *Current Protocols of Molecular Biology,* Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas *et al*., *Nucl. Acids Res.* 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect® (Quiagen), Dosper® or Fugene® (Boehringer Mannheim). The cells are grown as described in Lucas *et al., supra.* Approximately 3 x $10^{-7}$ cells are frozen in an ampule for further growth and production as described below.

The ampules containing the plasmid DNA are thawed by placement into water bath and mixed by vortexing. The contents are pipetted into a centrifuge tube containing 10 mLs of media and centrifuged at 1000 rpm for 5 minutes. The supernatant is aspirated and the cells are resuspended in 10 mL of selective media (0.2 μm filtered PS20 with 5% 0.2 μm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x $10^5$ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x $10^6$ cells/mL. On day 0, the cell number and pH is determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (*e.g.*, 35% polydimethylsiloxane emulsion, Dow Corning 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 μm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated at 4°C in 20 mM Hepes, pH 7.4, buffer containing 0.3 M NaCl and 5 mM imidazole at a flow rate of 4-5 ml/min. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 μL of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

EXAMPLE 5

Expression of PRO21074 in Yeast

The following method describes recombinant expression of PRO21074 in yeast.

First, yeast expression vectors are constructed for intracellular production or secretion of PRO21074 from the ADH2/GAPDH promoter. DNA encoding PRO21074 and the promoter is inserted into suitable restriction enzyme sites in the selected plasmid to direct intracellular expression of PRO21074. For secretion, DNA encoding PRO21074 can be cloned into the selected plasmid, together with DNA encoding the ADH2/GAPDH promoter, a native PRO21074 signal peptide or other mammalian signal peptide, or, for example, a yeast alpha-factor or invertase secretory signal/leader sequence, and linker sequences (if needed) for expression of PRO21074.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO21074 can subsequently be isolated and purified by removing the yeast cells from the fermentation medium by centrifugation and then concentrating the medium using selected cartridge filters. The concentrate containing PRO21074 may further be purified using selected column chromatography resins.

EXAMPLE 6

Expression of PRO21074 in Baculovirus-Infected Insect Cells

The following method describes recombinant expression of PRO21074 in Baculovirus-infected insect cells.

The sequence coding for PRO21074 is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO21074 or the desired portion of the coding sequence of PRO21074 such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodoptera frugiperda* ("Sf9") cells (ATCC CRL 1711) using lipofectin (commercially available from GIBCO-BRL). After 4-5 days of incubation at 28°C, the released viruses are harvested and used for further amplifications. Viral infection and protein expression are performed as described by O'Reilley *et al., Baculovirus expression vectors: A Laboratory Manual*, Oxford: Oxford University Press (1994).

Expressed poly-his tagged PRO21074 can then be purified, for example, by $Ni^{2+}$-chelate affinity chromatography as follows. Extracts are prepared from recombinant virus-infected Sf9 cells as described by Rupert *et al., Nature,* 362:175-179 (1993). Briefly, Sf9 cells are washed, resuspended in sonication buffer (25 mL Hepes, pH 7.9; 12.5 mM $MgCl_2$; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 μm filter. A $Ni^{2+-}$NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline again, the column is developed with a 0 to 500 mM Imidazole gradient in the secondary wash buffer. One mL fractions are collected and analyzed by SDS-PAGE and silver staining or Western blot with $Ni^{2+}$-NTA-conjugated to alkaline phosphatase (Qiagen). Fractions containing the eluted $His_{10}$-tagged PR021074 are pooled and dialyzed against loading buffer.

Alternatively, purification of the IgG tagged (or Fc tagged) PR021074 can be performed using known chromatography techniques, including for instance, Protein A or protein G column chromatography.

EXAMPLE 7

Preparation of Antibodies that Bind PR021074

This example illustrates preparation of monoclonal antibodies which can specifically bind PRO21074.

Techniques for producing the monoclonal antibodies are known in the art and are described, for instance, in Goding, *supra.* Immunogens that may be employed include purified PRO21074, fusion proteins containing PRO21074, and cells expressing recombinant PRO21074 on the cell surface. Selection of the immunogen can be made by the skilled artisan without undue experimentation.

Mice, such as Balb/c, are immunized with the PRO21074 immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO21074 antibodies.

After a suitable antibody titer has been detected, the animals "positive" for antibodies can be injected with a final intravenous injection of PRO21074. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO21074. Determination of "positive" hybridoma cells secreting the desired monoclonal antibodies against PRO21074 is within the skill in the art.

The positive hybridoma cells can be injected intraperitoncally into syngeneic Balb/c mice to produce ascites containing the anti-PRO21074 monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

EXAMPLE 8

Purification of PRO21074 Polypeptides Using Specific Antibodies

Native or recombinant PRO21074 polypeptides may be purified by a variety of standard techniques in the art of protein purification. For example, pro-PRO21074 polypeptide, mature PRO21074 polypeptide, or pre-PRO21074 polypeptide is purified by immunoaffinity chromatography using antibodies specific for the PRO21074 polypeptide of interest. In general, an immunoaffinity column is constructed by covalently coupling the anti-PRO21074 polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Pharmacia LKB Biotechnology). The antibody is coupled to the resin, the resin is blocked, and the derivative resin is washed according to the manufacturer's instructions.

Such an immunoaffinity column is utilized in the purification of PRO21074 polypeptide by preparing a fraction from cells containing PRO21074 polypeptide in a soluble form. This preparation is derived by solubilization of the whole cell or of a subcellular fraction obtained via differential centrifugation by the addition of detergent or by other methods well known in the art. Alternatively, soluble PRO21074 polypeptide containing a signal sequence may be secreted in useful quantity into the medium in which the cells are grown.

A soluble PRO21074 polypeptide-containing preparation is passed over the immunoaffinity column, and the column is washed under conditions that allow the preferential absorbance of PRO21074 polypeptide (*e.g.*, high ionic strength buffers in the presence of detergent). Then, the column is eluted under conditions that disrupt antibody/PRO21074 polypeptide binding (*e.g.*, a low pH buffer such as approximately pH 2-3, or a high concentration of a chaotrope such as urea or thiocyanate ion), and PRO21074 polypeptide is collected.

EXAMPLE 9

Drug Screening

This invention is particularly useful for screening compounds by using PRO21074 polypeptides or binding fragment thereof in any of a variety of drug screening techniques. The PRO21074 polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO21074 polypeptide or fragment. Drugs are screened against such transformed cells in competitive binding assays. Such cells, either in viable or fixed form, can be used for standard binding assays. One may measure, for example, the formation of complexes between PRO21074 polypeptide or a fragment and the agent being tested. Alternatively, one can examine the diminution in complex formation between the PRO21074 polypeptide and its target cell or target receptors caused by the agent being tested.

Thus, the present invention provides methods of screening for drugs or any other-agents which can affect a PRO21074 polypeptide-associated disease or disorder. These methods comprise contacting such an agent with an PRO21074 polypeptide or fragment thereof and assaying (i) for the presence of a complex between the agent and the PRO21074 polypeptide or fragment, or (ii) for the presence of a complex between the PRO21074 polypeptide or fragment and the cell, by methods well known in the art. In such competitive binding assays, the PRO21074 polypeptide or fragment is typically labeled. After suitable incubation, free PRO21074 polypeptide or fragment is separated from that present in bound form, and the amount of free or uncomplexed label is a measure of the ability of the particular agent to bind to PRO21074 polypeptide or to interfere with the PRO21074 polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO21074 polypeptide, the peptide test compounds are reacted with PRO21074 polypeptide and washed. Bound PRO21074 polypeptide is detected by methods well known in the art. Purified PRO21074 polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO21074 polypeptide specifically compete with a test compound for binding to PRO21074 polypeptide or fragments thereof. In this manner, the antibodies can be used to detect the presence of any peptide which shares one or more antigenic determinants with PRO21074 polypeptide.

EXAMPLE 10

Rational Drug Design

The goal of rational drug design is to produce structural analogs of biologically active polypeptide of interest (*i.e.*, a PRO21074 polypeptide) or of small molecules with which they interact, *e.g.*, agonists, antagonists, or inhibitors. Any of these examples can be used to fashion drugs which are more active or stable forms of the PRO21074 polypeptide or which enhance or interfere with the function of the PRO21074 polypeptide *in vivo* (*c.f.*, Hodgson, *Bio/Technology*, 9: 19-21 (1991)).

In one approach, the three-dimensional structure of the PRO21074 polypeptide, or of an PRO21074 polypeptide-inhibitor complex, is determined by x-ray crystallography, by computer modeling or, most typically, by a combination of the two approaches. Both the shape and charges of the PRO21074 polypeptide must be ascertained to elucidate the structure and to determine active site(s) of the molecule. Less often, useful information regarding the structure of the PRO21074 polypeptide may be gained by modeling based on the structure of homologous proteins. In both cases, relevant structural information is used to design analogous PRO21074 polypeptide-like molecules or to identify efficient inhibitors. Useful examples of rational drug design may include molecules which have improved activity or stability as shown by Braxton and Wells, *Biochemistry*, 31:7796-7801 (1992) or which act as inhibitors, agonists, or antagonists of native peptides as shown by Athauda *et al., J. Biochem.*, 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO21074 polypeptide may be made available to perform such analytical studies as X-ray crystallography. In addition, knowledge of the PRO21074 polypeptide amino acid sequence provided herein will provide guidance to those employing computer modeling techniques in place of or in addition to x-ray crystallography.

EXAMPLE 11

Tissue Expression Distribution

Oligonucleotide probes were constructed DNA 153576 shown in the accompanying figures for use in quantitative PCR amplification reactions. The oligonucleotide probes were chosen so as to give an approximately 50-300 base pair amplified fragment from the 3' end of its associated template in a standard PCR reaction. The oligonucleotide probes were employed in standard quantitative PCR amplification reactions with cDNA libraries isolated from different human adult and/or fetal tissue sources and analyzed by agarose gel electrophoresis so as to obtain a quantitative determination of the level of expression of the PRO21074 polypeptide-encoding nucleic acid in the various tissues tested. Knowledge of the expression pattern or the differential expression of the PRO21074 polypeptide-encoding nucleic acid in various different human tissue types provides a diagnostic marker useful for tissue typing, with or without other tissue-specific markers, for determining the primary tissue source of a metastatic tumor, and the like. The results of these assays demonstrated that the DNA153576-2925 molecule is highly expressed in cartilage and is expressed at very low levels (100-1000-fold lower than that of cartilage) in bone marrow and prostate. It is not expressed in cDNA libraries prepared from HUVEC, spleen, heart, uterus, colon tumor, substantia nigra, hippocampus, macrophage, dendrocyte or lymphoblast.

EXAMPLE 12

Taq Man Analysis

The cartilage specific expression pattern of DNA153576 was analyzed using Taqman PCR methodology on a variety of RNA's prepared form several human tissues as well as human cDNA libraries. The primers and probe used for the taqman analysis are as follows:

Primers

<153576.tm.f1> 5'-TTCCCTAACTCCTATGGCATATT-3' (SEQ ID NO:7)

<153576.tm.r1> 5'-TGGTCCAGTGGTAGGAGTGA-3' (SEQ ID NO:8)

Probe

<153576.tm.p1> 5'-AAGGCTCTCAGAGTTTCCCTAACAAACCA-3'(SEQ ID NO:9)

Standard Taqman protocols were used for all experiments as provided by PE Applied Biosystems Inc. "User Bulletin #2 ABI Prism 7700 Sequence Detection System, December 11, 1997". Briefly, In a 25 μl reaction 50 ng of cDNA library or 6-50 ng of RNA were used for each sample. All samples were normalized to beta actin expression in order to allow comparisons between samples. Conditions for the Taqman reactions were as follows:

Reverse Transcriptase Reaction (for RNA samples only)

| | |
|---|---|
| 48 °C | 30min |
| 95 °C | 10min |

PCR reaction for 40 cycles (for both cDNA libraries and RNA samples)

| | |
|---|---|
| 95 °C | 30sec |
| 60 °C | 1.5min |

Taqman data was analyzed by the "Comparative CT method" using the program "Sequence Detection Systems Ver. 1.6", Perkin Elmer corporation, Foster City, CA as described in "User Bulletin #2 ABI Prism 7700 Sequence Detection System, December 11, 1997, pg. 11-16".

DEPOSIT OF MATERIAL

The following materials have been deposited with the American Type Culture Collection, 10801 University Blvd., Manassas, VA 20110-2209, USA (ATCC):

| Material | ATCC Dep. No. | Deposit Date |
|---|---|---|
| DNA153576-2925 | 1907-PTA | May 23, 2000 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC §122 and the Commissioner's rules pursuant thereto (including 37 CFR §1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the embodiments described herein in in addition to those shown and described herein will become apparent to those skilled in the art from the foregoing description and fall within the scope of the appended claims.

## Claims

1. An isolated nucleic acid molecule which comprises DNA having at least 80% sequence identity to (a) a DNA molecule encoding a PR021074 polypeptide comprising the sequence of amino acid resides from 1 or 24 to 1313 of Figure 2, or (b) the complement of the DNA molecule of (a).

2. The isolated nucleic acid molecule of Claim 1 comprising the sequence of nucleotide positions from 31 or 100 to 3969 of Figure 1.

3. The isolated nucleic acid molecule of Claim 1 comprising the nucleotide sequence of Figure 1.

4. The isolated nucleic acid molecule of Claim 1 comprising a nucleotide sequence that encodes the sequence of amino acid residues from 1 or 24 to 1313 of Figure 2.

5. An isolated nucleic acid molecule comprising DNA which comprises at least 80% sequence identity to (a) a DNA molecule encoding the same mature polypeptide encoded by the human protein cDNA deposited with the ATCC on May 23, 2000 under ATCC Deposit No. 1907-PTA, or (b) the complement of the DNA molecule of (a).

6. The isolated nucleic acid molecule of Claim 5 comprising DNA encoding the same mature polypeptide encoded by the human protein cDNA deposited with the ATCC on May 23, 2000 under ATCC Deposit No. 1907-PTA.

7. An isolated nucleic acid molecule comprising DNA which comprises at least 80% sequence identity to (a) the full-length polypeptide coding sequence of the human protein cDNA deposited with the ATCC on May 23, 2000 under ATCC Deposit No. 1907-PTA, or (b) the complement of the coding sequence of (a).

8. The isolated nucleic acid molecule of Claim 7 comprising the full-length polypeptide coding sequence of the human protein cDNA deposited with the ATCC on May 23, 2000 under ATCC Deposit No. 1907-PTA.

9. An isolated nucleic acid molecule encoding a PR021074 polypeptide comprising DNA comprising at least 2036 nucleotides that hybridizes under stringent hybridization and wash conditions to the complement of the nucleic acid sequence that encodes amino acids 1 or 24 to 1313 of Figure 2.

10. An isolated nucleic acid molecule comprising at least 2036 nucleotides and which is produced by hybridizing a test DNA molecule under stringent hybridization conditions with (a) a DNA molecule which encodes a PR021074 polypeptide comprising a sequence of amino acid residues from 1 or 24 to 1313 of Figure 2, or (b) the complement of the DNA molecule of (a), and isolating the test DNA molecule.

11. The isolated nucleic acid molecule of Claim 10, which has at least 80% sequence identity to (a) a DNA molecule which encodes a PR021074 polypeptide comprising a sequence of amino acid residues from 1 or 24 to 1313 of Figure 2, or (b) the complement of the DNA molecule of (a).

12. A vector comprising the nucleic acid molecule of any one of Claims 1 to 11.

13. The vector of Claim 12, wherein said nucleic acid molecule is operably linked to control sequences recognized by a host cell transformed with the vector.

14. A nucleic acid molecule deposited with the ATCC under accession number 1907-PTA.

15. An isolated host cell comprising the vector of Claim 12.

16. The host cell of Claim 15, wherein said cell is a CHO cell.

17. The host cell of Claim 15, wherein said cell is an *E. coli*.

18. The host cell of Claim 15, wherein said cell is a yeast cell.

19. A process for producing a PRO21074 polypeptide comprising culturing the host cell of Claim 15 under conditions suitable for expression of said PRO21074 polypeptide and recovering said PRO21074 polypeptide from the cell culture.

**20.** An isolated PRO21074 polypeptide comprising an amino acid sequence comprising at least 80% sequence identity to the sequence of amino acid residues from about 1 or 24 to 1313 of Figure 2.

**21.** The isolated PRO21074 polypeptide of Claim 20 comprising amino acid residues 1 or 24 to 1313 of Figure 2.

**22.** An isolated PRO21074 polypeptide having at least 80% sequence identity to the polypeptide encoded by the cDNA insert of the vector deposited with the ATCC on May 23, 2000 as ATCC Deposit No. 1907-PTA.

**23.** The isolated PRO21074 polypeptide of Claim 22 which is encoded by the cDNA insert of the vector deposited with the ATCC on May 23, 2000 as ATCC Deposit No. 1907-PTA.

**24.** An isolated PRO21074 polypeptide comprising the sequence of amino acid residues from 1 or 24 to 1313 of Figure 2.

**25.** An isolated polypeptide produced by culturing a host cell comprising the isolated nucleic acid molecule of claim 10 under conditions suitable for the expression of said polypeptide, and recovering said polypeptide from the cell culture.

**26.** The isolated polypeptide of Claim 25, wherein said isolated nucleic acid molecule has at least 80% sequence identity to (a) a DNA molecule which encodes a PRO21074 polypeptide comprising a sequence of amino acid residues from 1 or 24 to 1313 of Figure 2, or (b) the complement of the DNA molecule of (a).

**27.** A chimeric molecule comprising a PRO21074 polypeptide according to any one of claims 20 to 24 fused to a heterologous amino acid sequence.

**28.** The chimeric molecule of Claim 27, wherein said heterologous amino acid sequence is an epitope tag sequence.

**29.** The chimeric molecule of Claim 27, wherein said heterologous amino acid sequence is a Fc region of an immunoglobulin.

**30.** An antibody which specifically binds to a PRO21074 polypeptide according to any one of claims 20 to 24.

**31.** The antibody of Claim 30, wherein said antibody is a monoclonal antibody.

**32.** The antibody of Claim 30, wherein said antibody is a humanized antibody.

**33.** The antibody of Claim 30, wherein said antibody is an antibody fragment.

**34.** A composition of matter comprising (a) a PRO21074 polypeptide according to any one of claims 20 to 24, or (b) an antibody according to any one of claims 30 to 33 in admixture with a pharmaceutically acceptable carrier.

## Patentansprüche

**1.** Isoliertes Nucleinsäuremolekül, das DNA umfasst, die zumindest 80 % Sequenzidentität (a) mit einem DNA-Molekül, das für ein PRO21074-Polypeptid kodiert, das die Sequenz der Aminosäurereste 1 oder 24 bis 1313 aus Fig. 2 umfasst, oder (b) mit dem Komplement des DNA-Moleküls unter (a) aufweist.

**2.** Isoliertes Nucleinsäuremolekül nach Anspruch 1, umfassend die Sequenz der Nucleotidpositionen 31 oder 100 bis 3969 aus Fig. 1.

**3.** Isoliertes Nucleinsäuremolekül nach Anspruch 1, umfassend die Nucleotidsequenz aus Fig. 1.

**4.** Isoliertes Nucleinsäuremolekül nach Anspruch 1, umfassend eine Nucleotidsequenz, die für die Sequenz der Aminosäurereste 1 oder 24 bis 1313 aus Fig. 2 kodiert.

**5.** Isoliertes Nucleinsäuremolekül, das DNA umfasst, die zumindest 80 % Sequenzidentität (a) mit einem DNA-Molekül, das für dasselbe reife Polypeptid kodiert, für das die menschliche cDNA kodiert, die bei der ATCC am 23. Mai 2000 unter der ATCC-Hinterlegungs-Nr. 1907-PTA hinterlegt wurde, oder (b) mit dem Komplement des DNA-Moleküls unter (a) aufweist.

6. Isoliertes Nucleinsäuremolekül nach Anspruch 5, umfassend DNA, die für dasselbe reife Polypeptid kodiert, für das die menschliche cDNA kodiert, die bei der ATCC am 23. Mai 2000 unter der ATCC-Hinterlegungs-Nr. 1907-PTA hinterlegt wurde.

7. Isoliertes Nucleinsäuremolekül, umfassend DNA, die zumindest 80 % Sequenzidentität (a) mit der Polypeptid-Kodiersequenz voller Länge der menschlichen Protein-cDNA, die bei der ATCC am 23. Mai 2000 unter der ATCC-Hinterlegungs-Nr. 1907-PTA hinterlegt wurde, oder (b) mit dem Komplement der Kodiersequenz unter (a) aufweist.

8. Isoliertes Nucleinsäuremolekül nach Anspruch 7, umfassend die Polypeptid-Kodiersequenz voller Länge der menschlichen Protein-cDNA, die bei der ATCC am 23. Mai 2000 unter der ATCC-Hinterlegungs-Nr. 1907-PTA hinterlegt wurde.

9. Isoliertes Nucleinsäuremolekül, das für ein PRO21074-Polypeptid kodiert und DNA mit zumindest 2036 Nucleotiden umfasst, die unter stringenten Hybridisierungs- und Waschbedingungen an das Komplement der Nucleinsäuresequenz hybridisiert, die für die Aminosäuren 1 oder 24 bis 1313 aus Fig. 2 kodiert.

10. Isoliertes Nucleinsäuremolekül, das zumindest 2036 Nucleotide umfasst und durch Hybridisieren eines Test-DNA-Moleküls unter stringenten Hybridisierungsbedingungen (a) mit einem DNA-Molekül, das für ein PRO21074-Polypeptid kodiert, das eine Sequenz der Aminosäurereste 1 oder 24 bis 1313 aus Fig. 2 umfasst, oder (b) mit dem Komplement des DNA-Moleküls unter (a) sowie Isolieren des Test-DNA-Moleküls erzeugt wird.

11. Isoliertes Nucleinsäuremolekül nach Anspruch 10, das zumindest 80 % Sequenzidentität (a) mit einem DNA-Molekül, das für ein PRO21074-Polypeptid kodiert, das eine Sequenz der Aminosäurereste 1 oder 24 bis 1313 aus Fig. 2 umfasst, oder (b) mit dem Komplement des DNA-Moleküls unter (a) aufweist.

12. Vektor, umfassend ein Nucleinsäuremolekül nach einem der Ansprüche 1 bis 11.

13. Vektor nach Anspruch 12, worin das Nucleinsäuremolekül operabel an Kontrollsequenzen gebunden ist, die von einer mit dem Vektor transformierten Wirtszelle erkannt werden.

14. Nucleinsäuremolekül, das bei der ATCC unter der ATCC-Hinterlegungs-Nr. 1907-PTA hinterlegt ist.

15. Isolierte Wirtszelle, die einen Vektor nach Anspruch 12 umfasst.

16. Wirtszelle nach Anspruch 15, worin die Zelle eine CHO-Zelle ist.

17. Wirtszelle nach Anspruch 15, worin die Zelle eine E.coli-Zelle ist.

18. Wirtszelle nach Anspruch 15, worin die Zelle eine Hefezelle ist.

19. Verfahren zur Herstellung eines PRO21074-Polypeptids, umfassend das Kultivieren einer Wirtszelle nach Anspruch 15 unter Bedingungen, die für die Expression von PRO21074-Polypeptid geeignet sind, sowie das Gewinnen des PRO21074-Polypeptids aus der Zellkultur.

20. Isoliertes PRO21074-Polypeptid, umfassend eine Aminosäuresequenz, die zumindest 80 % Sequenzidentität mit der Sequenz der Aminosäurereste von etwa 1 oder 24 bis 1313 aus Fig. 2 aufweist.

21. Isoliertes PRO21074-Polypeptid nach Anspruch 20, umfassend die Aminosäurereste 1 oder 24 bis 1313 aus Fig. 2.

22. Isoliertes PRO21074-Polypeptid mit zumindest 80 % Sequenzidentität mit dem Polypeptid, für das das cDNA-Insert des Vektors kodiert, der bei der ATCC am 23. Mai 2000 unter der ATCC-Hinterlegungs-Nr. 1907-PTA hinterlegt wurde.

23. Isoliertes PRO21074-Polypeptid nach Anspruch 22, für das das cDNA-Insert des Vektors kodiert, der bei der ATCC am 23. Mai 2000 unter der ATCC-Hinterlegungs-Nr. 1907-PTA hinterlegt wurde.

**24.** Isoliertes PRO21074-Polypeptid, umfassend die Sequenz der Aminosäurereste 1 oder 24 bis 1313 aus Fig. 2.

**25.** Isoliertes Polypeptid, das durch Kultivieren einer Wirtszelle, die ein isoliertes Nucleinsäuremolekül nach Anspruch 10 umfasst, unter Bedingungen, die für die Expression dieses Polypeptids geeignet sind, sowie Gewinnen des Polypeptids aus der Zellkultur erzeugt wird.

**26.** Isoliertes Polypeptid nach Anspruch 25, worin das isolierte Nucleinsäuremolekül zumindest 80 % Sequenzidentität (a) mit einem DNA-Molekül, das für ein PRO-21074-Polypeptid kodiert, das eine Sequenz der Aminosäurereste 1 oder 24 bis 1313 aus Fig. 2 umfasst, oder (b) mit dem Komplement des DNA-Moleküls unter (a) aufweist.

**27.** Chimäres Molekül, umfassend ein PRO21074-Polypeptid nach einem der Ansprüche 20 bis 24, das an eine heterologe Aminosäuresequenz fusioniert ist.

**28.** Chimäres Molekül nach Anspruch 27, worin die heterologe Aminosäuresequenz eine Epitopmarkierungs-Sequenz ist.

**29.** Chimäres Molekül nach Anspruch 27, worin die heterologe Aminosäuresequenz eine Fc-Region eines Immunglobulins ist.

**30.** Antikörper, der sich spezifisch an ein PRO21074-Polypeptid nach einem der Ansprüche 20 bis 24 bindet.

**31.** Antikörper nach Anspruch 30, worin der Antikörper ein monoklonaler Antikörper ist.

**32.** Antikörper nach Anspruch 30, worin der Antikörper ein humanisierter Antikörper ist.

**33.** Antikörper nach Anspruch 30, worin der Antikörper ein Antikörperfragment ist.

**34.** Substanzzusammensetzung, umfassend (a) ein PRO21074-Polypeptid nach einem der Ansprüche 20 bis 24 oder (b) einen Antikörper nach einem der Ansprüche 30 bis 33 im Gemisch mit einem pharmazeutisch annehmbaren Träger.

**Revendications**

**1.** Molécule d'acide nucléique isolée qui comprend un ADN ayant une identité de séquence d'au moins 80 % avec (a) une molécule d'ADN codant pour un polypeptide PRO21074 comprenant la séquence de résidus d'aminoacides de 1 ou 24 à 1313 de la figure 2, ou (b) le complément de la molécule d'ADN de (a).

**2.** Molécule d'acide nucléique isolée suivant la revendication 1, comprenant la séquence de nucléotides de la position 31 ou 100 à la position 3969 de la figure 1.

**3.** Molécule d'acide nucléique isolée suivant la revendication 1, comprenant la séquence de nucléotides de la figure 1.

**4.** Molécule d'acide nucléique isolée suivant la revendication 1, comprenant une séquence de nucléotides qui code pour la séquence de résidus d'aminoacides de 1 ou 24 à 1313 de la figure 2.

**5.** Molécule d'acide nucléique isolée comprenant un ADN qui possède une identité de séquence d'au moins 80 % avec (a) une molécule d'ADN codant pour le même polypeptide mature codé par l'ADNc de protéine humaine déposé dans l'ATCC le 23 mai 2000 sous le numéro de dépôt ATCC 1907-PTA ou (b) le complément de la molécule d'ADN de (a).

**6.** Molécule d'acide nucléique isolée suivant la revendication 5, comprenant l'ADN codant pour le même polypeptide mature codé par l'ADNc de protéine humaine déposé dans l'ATCC le 23 mai 2000 sous le numéro de dépôt ATCC 1907-PTA.

**7.** Molécule d'acide nucléique isolée comprenant un ADN qui présente une identité de séquence d'au moins 80 % avec (a) la séquence codant pour le polypeptide complet de l'ADNc de protéine humaine déposé dans l'ATCC le 23 mai 2000 sous le numéro de dépôt ATCC 1907-PTA, ou (b) le complément de la séquence codante de (a).

**8.** Molécule d'acide nucléique isolée suivant la revendication 7, comprenant la séquence codant pour le polypeptide complet de l'ADNc de protéine humaine déposé dans l'ATCC le 23 mai 2000 sous le numéro de dépôt ATCC 1907-PTA.

**9.** Molécule d'acide nucléique isolée codant pour un polypeptide PRO21074, comprenant un ADN comprenant au moins 2036 nucléotides qui s'hybride dans des conditions drastiques d'hybridation et de lavage avec le complément de la séquence d'acide nucléique qui code pour les aminoacides 1 ou 24 à 1313 de la figure 2.

**10.** Molécule d'acide nucléique isolée comprenant au moins 2036 nucléotides et qui est produite en hybridant une molécule d'ADN d'essai dans des conditions d'hybridation drastiques avec (a) une molécule d'ADN qui code pour un polypeptide PRO21074 comprenant une séquence de résidus d'aminoacides de 1 ou 24 à 1313 de la figure 2 ou (b) le complément de la molécule d'ADN de (a), et en isolant la molécule d'ADN d'essai.

**11.** Molécule d'acide nucléique isolée suivant la revendication 10, qui a une identité de séquence d'au moins 80 % avec (a) une molécule d'ADN qui code pour un polypeptide PRO21074 comprenant une séquence de résidus d'aminoacides de 1 ou 24 à 1313 de la figure 2 ou (b) le complément de la molécule d'ADN de (a).

**12.** Vecteur comprenant la molécule d'acide nucléique de l'une quelconque des revendications 1 à 11.

**13.** Vecteur suivant la revendication 12, dans lequel ladite molécule d'acide nucléique est liée de manière fonctionnelle à des séquences de régulation reconnues par une cellule hôte transformée avec le vecteur.

**14.** Molécule d'acide nucléique déposée dans l'ATCC sous le numéro de dépôt 1907-PTA.

**15.** Cellule hôte isolée comprenant le vecteur de la revendication 12.

**16.** Cellule hôte suivant la revendication 15, ladite cellule étant une cellule CHO.

**17.** Cellule hôte suivant la revendication 15, ladite cellule étant une cellule de *E. coli.*

**18.** Cellule hôte suivant la revendication 15, ladite cellule étant une cellule de levure.

**19.** Procédé pour la production d'un polypeptide PRO21074, comprenant, les étapes consistant à cultiver la cellule hôte de la revendication 15 dans des conditions convenables pour l'expression dudit polypeptide PRO21074 et à recueillir ledit polypeptide PRO2174 à partir de la culture cellulaire.

**20.** Polypeptide PRO21074 isolé comprenant une séquence d'aminoacides présentant une identité de séquence d'au moins 80 % avec la séquence de résidus d'aminoacides d'environ 1 ou 24 à 1313 de la figure 2.

**21.** Polypeptide PRO21074 isolé suivant la revendication 20, comprenant les résidus d'aminoacides 1 ou 24 à 1313 de la figure 2.

**22.** Polypeptide PRO21074 isolé présentant une identité de séquence d'au moins 80 % avec le polypeptide codé par le segment d'ADNc d'insertion du vecteur déposé dans l'ATCC le 23 mai 2000 sous le numéro de dépôt ATCC 1907-PTA.

**23.** Polypeptide PRO21074 isolé suivant la revendication 22, qui est codé par le segment d'ADNc d'insertion du vecteur déposé dans l'ATCC le 23 mai 2000 sous le numéro de dépôt ATCC 1907-PTA.

**24.** Polypeptide PRO21074 isolé comprenant la séquence de résidus d'aminoacides de 1 ou 24 à 1313 de la figure 2.

**25.** Polypeptide isolé produit en cultivant une cellule hôte comprenant la molécule d'acide nucléique isolée de la revendication 10 dans des conditions convenables pour l'expression dudit polypeptide et en recueillant ledit polypeptide à partir de la culture cellulaire.

**26.** Polypeptide isolé suivant la revendication 25, dans lequel ladite molécule d'acide nucléique isolée présente une identité de séquence d'au moins 80 % avec (a) une molécule d'ADN qui code pour un polypeptide PRO21074 comprenant une séquence de résidus d'aminoacides de 1 ou 24 à 1313 de la figure 2 ou (b) le complément de la molécule d'ADN de (a).

**27.** Molécule chimère comprenant un polypeptide PRO2174 suivant l'une quelconque des revendications 20 à 24 fusionné à une séquence d'aminoacides hétérologue.

**28.** Molécule chimère suivant la revendication 27, dans laquelle ladite séquence d'aminoacides hétérologue est une séquence de marqueur d'épitope.

**29.** Molécule chimère suivant la revendication 27, dans laquelle ladite séquence d'aminoacides hétérologue est une région Fc d'une immunoglobuline.

**30.** Anticorps qui se lie spécifiquement au polypeptide PRO21074 suivant l'une quelconque des revendications 20 à 24.

**31.** Anticorps suivant la revendication 30, ledit anticorps étant un anticorps monoclonal.

**32.** Anticorps suivant la revendication 30, ledit anticorps étant un anticorps humanisé.

**33.** Anticorps suivant la revendication 30, ledit anticorps étant un fragment d'anticorps.

**34.** Composition comprenant (a) un polypeptide PRO2174 suivant l'une quelconque des revendications 20 à 24 ou (b) un anticorps suivant l'une quelconque des revendications 30 à 33 en mélange avec un support pharmaceutiquement acceptable.

GAACAACAGATCCAGGCCAGAGGTGGCATC**ATG**TCTGGGTGGAGGTACCTCATCTGTGTC
AGCTTTTTGCTGACCATTCTTCTTGAACTGACATACCAGGGACCCCCTGTCCCCGCTTCA
TCAAGCACAAAGTTGTTAATGACAAGCTATTCTATGCGCTCCACGGTGGTGTCTCGCTAT
GCCCACACCTTGGTCACCTCTGTCCTGTTTAATCCACATGCTGAAGCCCATGAAGCCATC
TTTGACCTGGATCTGCCTCATCTTGCCTTTATCTCCAATTTCACTATGACCATCAACAAT
AAAGTCTACATTGCAGAAGTCAAAGAGAAGCACCAGGCAAAGAAAATCTATGAAGAAGCC
CATCAGCAGGGAAAGACAGCTGCTCATGTAGGCATCAGGGACCGGGAATCAGAGAAGTTC
CGCATCTCCACCAGCCTGGCAGCAGGCACAGAGGTGACTTTTTCCCTGGCCTATGAGGAA
CTGCTTCAGCGGCACCAGGGCCAGTACCAGCTGGTGGTGAGCCTGAGGCCTGGCCAATTG
GTGAAGAGGCTGAGCATAGAGGTTACAGTGTCAGAAAGGACAGGCATCTCCTATGTGCAC
ATACCACCCCTGAGGACCGGCCGTCTGCGCACCAATGCCCATGCAAGTGAGGTGGATTCA
CCCCCATCCACCAGGATCGAGAGGGGAGAGACCTGTGTCCGAATCACCTACTGCCCGACA
TTGCAAGACCAGTCGTCCATCTCTGGGTCAGGCATCATGGCTGACTTCCTGGTTCAGTAC
GATGTGGTCATGGAGGACATCATTGGAGACGTGCAGATTTACGATGACTATTTCATTCAC
TACTTTGCCCCCAGAGGCCTTCCACCTATGGAGAAGAATGTGGTTTTTGTTATTGACGTA
AGCAGCTCCATGTTTGGTACCAAGATGGAACAGACTAAAACGGCCATGAATGTGATCCTC
AGTGACCTTCAAGCCAATGACTACTTCAACATCATCTCCTTTTCTGACACAGTTAATGTT
TGGAAAGCTGGAGGCTCAATCCAGGCCACCATCCAGAATGTCCACAGTGCCAAGGACTAC
CTGCATTGCATGGAAGCCGATGGCTGGACAGACGTCAACTCAGCTCTGCTGGCAGCTGCT
TCAGTGCTGAACCATAGCAACCAGGAGCCTGGGAGGGGCCCCAGTGTGGGGAGGATCCCT
CTTATCATCTTCCTGACGGATGGGGAGCCCACGGCCGGCGTGACGACCCCCAGTGTGATC
CTCTCCAATGTCCGTCAGGCGCTAGGCCACAGGGTATCCCTTTTCAGCTTGGCCTTTGGG
GATGATGCTGACTTTACACTGCTGCGCCGCCTGTCCCTGGAAAACCGGGGAATAGCCCGG
CGCATATATGAGGACACTGATGCGGCCCTACAGCTGAAGGGCCTCTATGAGGAGATCTCC
ATGCCTCTGCTGGCAGATGTGCGTCTGAACTACCTGGGTGGCTTGGTTGGGGCCTCCCCT
TGGGCCGTTTTCCCCAACTACTTTGGTGGCTCTGAGCTGGTGGTGGCAGGACAGGTGCAG
CCAGGCAAACAGGAACTGGGCATCCACCTGGCAGCCCGTGGCCCCAAGGATCAGCTTCTT
GTGGCCCACCACAGTGAAGGGGCCACCAACAACAGCCAGAAGGCCTTTGGTTGCCCAGGG
GAGCCAGCCCCCAATGTGGCCCACTTCATCCGCCGCCTCTGGGCCTATGTCACCATTGGA
GAACTGCTGGATGCACACTTCCAAGCTCGTGACACCACCACTCGCCACCTGCTGGCTGCC
AAAGTCCTCAACCTGTCCCTTGAATACAACTTTGTCACACCTCTGACTTCACTGGTCATG
GTGCAACCCAAACAGGCCAGTGAGGAGACCAGGAGACAGACTTCCACCTCTGCTGGGCCA
GACACCATCATGCCCTCATCCAGCAGCAGGCATGGCCTAGGGGTAAGCACAGCTCAGCCA
GCCTTGGTGCCCAAGGTCATCTCCCCCAAATCAAGGCCTGTGAAACCAAAGTTCTACTTA
TCCTCAACTACTACTGCCTCTACCAAGAAGATGCTAAGTTCCAAAGAGCTGGAGCCATTG
GGAGAGAGCCCTCATACCCTGTCAATGCCCACATACCCAAAGGCCAAAATTCCAGCACAA

***FIG._1A-1***

CAGGATTCTGGCACCTTGGCCCAGCCAACTCTCAGGACAAAACCTACCATTCTTGTGCCC
TCAAATTCTGGTACTCTGTTGCCTCTGAAGCCCGGCTCTCTATCACACCAGAATCCTGAT
ATATTACCCACGAACTCCAGGACACAAGTCCCACCTGTGAAACCTGGCATCCCAGCCTCG
CCCAAAGCTGACACTGTGAAATGTGTTACTCCACTGCATTCCAAACCTGGTGCTCCATCG
CACCCCCAACTTGGGGCACTCACATCACAGGCACCTAAAGGCCTGCCACAGTCAAGACCT
GGAGTCTCTACACTTCAGGTTCCCAAGTACCCACTACACACCAGACCTAGGGTTCCTGCT
CCCAAGACCCGAAACAACATGCCACACCTGGGGCCTGGAATCCTCTTGTCCAAGACCCCT
AAAATCTTATTATCTCTTAAACCGAGTGCCCCACCACACCAAATTTCCACAAGCATATCA
CTTTCCAAGCCT

*FIG._1A-2*

GAGACCCCAAACCCCCATATGCCTCAAACCCCACTACCTCCTAGACCTGACAGACCAAGG
CCCCCACTTCCTGAGAGCCTAAGCACATTCCCAAATACAATCTCAAGTTCCACAGGTCCC
AGCAGTACCACAACCACCTCTGTCCTTGGAGAACCCCTCCCCATGCCCTTTACTCCCACT
CTGCCCCCTGGAAGGTTCTGGCATCAGTATGACCTCCTCCCGGGTCCCCAGAGGACCAGG
CAAGTTCTGGGACCATCTAGGCCAGGAGTTCCAACAATGAGCCTACTCAACAGCTCCAGG
CCTACACCAGAAGGCAGCCCTCCAAACCTGCCAATCTTGCTGCCTTCTAGCATCCTCCCT
GAGGCCATCAGTCTGCTCCTTCTCCCTGAGGAGCTAGAGCTGCTGTCTGAATCAATGGTG
GAATCCAAGTTCGTGGAGTCCTTGAACCCACCAGCTTTCTATACCTTCCTCACTCCTGAT
GAAGATGGAAGTCCAAACTGGGATGGCAATTCTGAGGAGATCCTGGGAGGAGCTGGAGGC
AGCATGGAATCTCAAGGAAGTTCTGTGGGGTTAGCAAAAGGCACATTGCCTAGCATCTTC
ACCTTCTCCTCCTCAGTGGACGGGGACCCCCACTTTGTGATCCAAATCCCACACTCAGAA
GAGAAGATCTGCTTCACACTGAATGGGCACCCTGGGGACTTGCTGCAGCTCATAGAGGAC
CCAAAGGCAGGGCTGCATGTGAGTGGGAAGCTGCTTGGCGCACCACCAAGGCCGGGCCAC
AAGGACCAGACTCGCACCTACTTCCAGATCATCACAGTCACTACAGACAAACCCCGGGCC
TATACTATCACCATCAGCCGCAGTTCTATATCTTTGCGAGGCGAGGGTACCTTGCGCCTG
TCCTGGGACCAACCTGCCCTGCTGAAGAGGCCCCAGCTGGAGCTCTATGTGGCTGCTGCA
GCCCGCCTTACCCTCCGCCTTGGGCCCTACCTTGAGTTCCTAGTCCTCCGACACCGCTAC
AGGCATCCCAGTACCCTGCAACTACCCCACCTGGGGTTCTACGTGGCCAATGGCTCAGGC
CTCAGCCCCTCAGCCCGTGGCCTGATAGGGCAGTTCCAGCACGCAGACATCCGACTGGTG
ACAGGACCTATGGGGCCATGCTTACGAAGGCACCATGGCCCAGATGTGCCTGTGATTCTA
GGCAAGAGGCTGCTGAAGGACTCACCAAGGCTGCTGCCCCGCTGGGCTTCCTGCTGGCTG
GTGAAGCGCTCTCATGTAGAGCTGCTTCTGGGCCACCCCTACCTCTCCTATGTCCTG**TGA**
TGGCTTCTGAATTCCAGAGCCAGGAGACCTGAATTTGGGAGATCCAGAAACCAGGGCATG
GGGTGAGCCAGGGACAGAGACCCAAGGACATGGAAGCACACACAGGGACACACAGACTCA
CGCATGTTCTAAGGAACACATATACAAGGGTATACAAACACATAGACATGCAGAGACATA
GAGTCAGGGACTCCTGCATCTTCATGGTCCTTCACATCTCCAGTCTCACCCTCTCCAAAT
CCATGCTCCTCACCAGCCCTACAAGCCACTTTTGTAAAACGAAAAAATGATCAGTAGTTC
TGCTCACAGCTTTCCCTAACTCCTATGGCATATTCAAGGCTCTCAGAGTTTCCCTAACAA
ACCATGCTCACTCCTACCACTGGACCATTGCATGTGCTTTTCCTTCTGCATGAAACACTC
TTCCTTTGTTCCTGTAGGTGAGTATAATTCATCCTTCAAATCCTAGTTGTTACCTCTTCT
AGGAGGCCTTTCCTGATGCCTGCCCTAGATTGAGTTTGGTCCTGTCCTCTGGGTTCCTAT
AGCCCACCCCCAACCCTCCACGCTCACAGTATTTATACTGTGTAGTAATCATCTGTTTAC
TTGTCTGGATCCCTGACTTAGACTGTGAGATCTTTGAGGGCAAGGACCTTTTCTGAATCA
TCTATGTATCCCCAGTGCCTCTCACATAATAGGTGCTTAGTAAATATTTGTT

*FIG._1B*

MSGWRYLICVSFLLTILLELTYQGPPVPASSSTKLLMTSYSMRSTVVSRYAHTLVTSVLF
NPHAEAHEAIFDLDLPHLAFISNFTMTINNKVYIAEVKEKHQAKKIYEEAHQQGKTAAHV
GIRDRESEKFRISTSLAAGTEVTFSLAYEELLQRHQGQYQLVVSLRPGQLVKRLSIEVTV
SERTGISYVHIPPLRTGRLRTNAHASEVDSPPSTRIERGETCVRITYCPTLQDQSSISGS
GIMADFLVQYDVVMEDIIGDVQIYDDYFIHYFAPRGLPPMEKNVVFVIDVSSSMFGTKME
QTKTAMNVILSDLQANDYFNIISFSDTVNVWKAGGSIQATIQNVHSAKDYLHCMEADGWT
DVNSALLAAASVLNHSNQEPGRGPSVGRIPLIIFLTDGEPTAGVTTPSVILSNVRQALGH
RVSLFSLAFGDDADFTLLRRLSLENRGIARRIYEDTDAALQLKGLYEEISMPLLADVRLN
YLGGLVGASPWAVFPNYFGGSELVVAGQVQPGKQELGIHLAARGPKDQLLVAHHSEGATN
NSQKAFGCPGEPAPNVAHFIRRLWAYVTIGELLDAHFQARDTTTRHLLAAKVLNLSLEYN
FVTPLTSLVMVQPKQASEETRRQTSTSAGPDTIMPSSSSRHGLGVSTAQPALVPKVISPK
SRPVKPKFYLSSTTTASTKKMLSSKELEPLGESPHTLSMPTYPKAKIPAQQDSGTLAQPT
LRTKPTILVPSNSGTLLPLKPGSLSHQNPDILPTNSRTQVPPVKPGIPASPKADTVKCVT
PLHSKPGAPSHPQLGALTSQAPKGLPQSRPGVSTLQVPKYPLHTRPRVPAPKTRNNMPHL
GPGILLSKTPKILLSLKPSAPPHQISTSISLSKPETPNPHMPQTPLPPRPDRPRPPLPES
LSTFPNTISSSTGPSSTTTTSVLGEPLPMPFTPTLPPGRFWHQYDLLPGPQRTRQVLGPS
RPGVPTMSLLNSSRPTPEGSPPNLPILLPSSILPEAISLLLLPEELELLSESMVESKFVE
SLNPPAFYTFLTPDEDGSPNWDGNSEEILGGAGGSMESQGSSVGLAKGTLPSIFTFSSSV
DGDPHFVIQIPHSEEKICFTLNGHPGDLLQLIEDPKAGLHVSGKLLGAPPRPGHKDQTRT
YFQIITVTTDKPRAYTITISRSSISLRGEGTLRLSWDQPALLKRPQLELYVAAAARLTLR
LGPYLEFLVLRHRYRHPSTLQLPHLGFYVANGSGLSPSARGLIGQFQHADIRLVTGPMGP
CLRRHHGPDVPVILGKRLLKDSPRLLPRWASCWLVKRSHVELLLGHPYLSYVL

N-glycosylation site
83-87
374-378
540-544
594-598
971-975
1231-1235

Glycosaminoglycan attachment site
238-242

cAMP- and cGMP-dependent protein kinase phosphorylation site
172-176
439-443
621-625

*FIG._2A*

N-myristoylation site
139-145
335-341
483-489
484-490
517-523
537-543
642-648
644-650
795-801
804-810
843-849
979-985
1050-1056
1051-1057
1054-1060
1060-1066
1064-1070
1068-1074
1118-1124
1234-1240

Amidation site
1274-1278

von Willebrand factor type A domain
283-469

FIG._2B

ATGCTGCCTATTCTGGGCAGTAGGCACCCCTCTAGCCTAGGGCAGGCCCGAAAGTTGGAT
TATAGGCCATCCAAGAGCCAATGCCTAGAACCTGGGACCCCAAGAGCCTGCTTGGTGCTC
TACACCCTTGTGGCCAAGCTATTTATAATGACTATTTCATTCACTACTTTGCCCCCAGAG
GCCTTCCACCTATGGAGAAGAATGTGGTTTTTGTTATTGACATAAGTGGCTTCATGTTTG
GTACCAAGATGAAACAGGATCACAACTTCTCACTAGCAAGGGAACAAAACTGGACAGAGA
ATGAGTTTGACAAATTGATGGAAGTAGACTTCAGAAGGTGGGTAATAACAAACTCCTCCG
AGCTAAAGGAGCATGTTCTAACCCAATGCAAGGAAGCTAACAACCTTGAAAAAAGGTTAG
ATGAATTGCTAACTAGAATAACCAGTGTAGAGAAGAACATAAATGACCTGATGGAGCTGA
AAAACACAGCAGGAGAACTTCGTGAAGCATACACAAGTTTCAATAGCTGCAATGATCAAG
CAGAAGAAAGGATAACAGTGATTTA

**FIG._3**

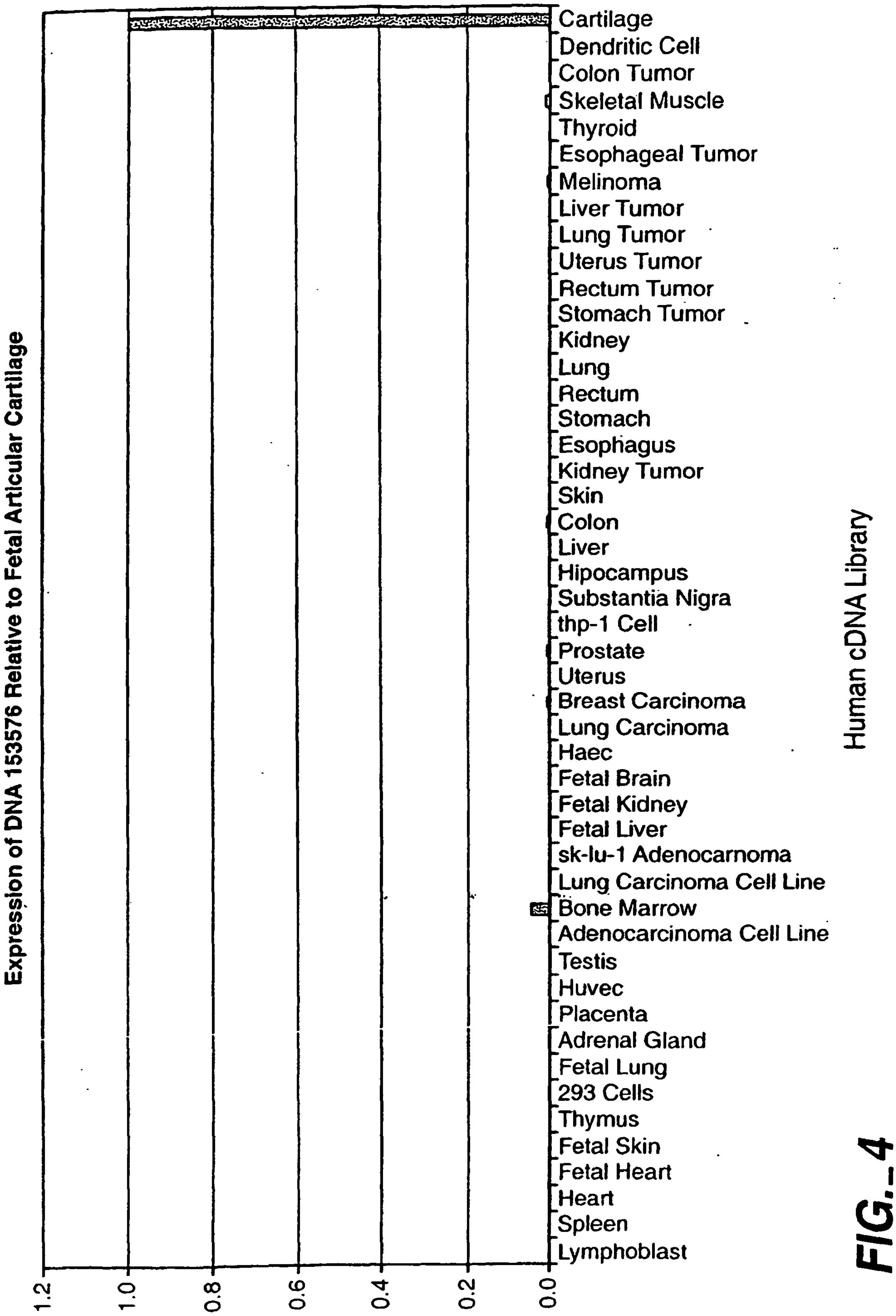
Expression of DNA 153576 Relative to Fetal Articular Cartilage
1.2
1.0
0.8
0.6
0.4
0.2
0.0
Cartilage
Dendritic Cell
Colon Tumor
Skeletal Muscle
Thyroid
Esophageal Tumor
Melinoma
Liver Tumor
Lung Tumor
Uterus Tumor
Rectum Tumor
Stomach Tumor
Kidney
Lung
Rectum
Stomach
Esophagus
Kidney Tumor
Skin
Colon
Liver
Hipocampus
Substantia Nigra
thp-1 Cell
Prostate
Uterus
Breast Carcinoma
Lung Carcinoma
Haec
Fetal Brain
Fetal Kidney
Fetal Liver
sk-lu-1 Adenocarnoma
Lung Carcinoma Cell Line
Bone Marrow
Adenocarcinoma Cell Line
Testis
Huvec
Placenta
Adrenal Gland
Fetal Lung
293 Cells
Thymus
Fetal Skin
Fetal Heart
Heart
Spleen
Lymphoblast
Human cDNA Library

FIG._4

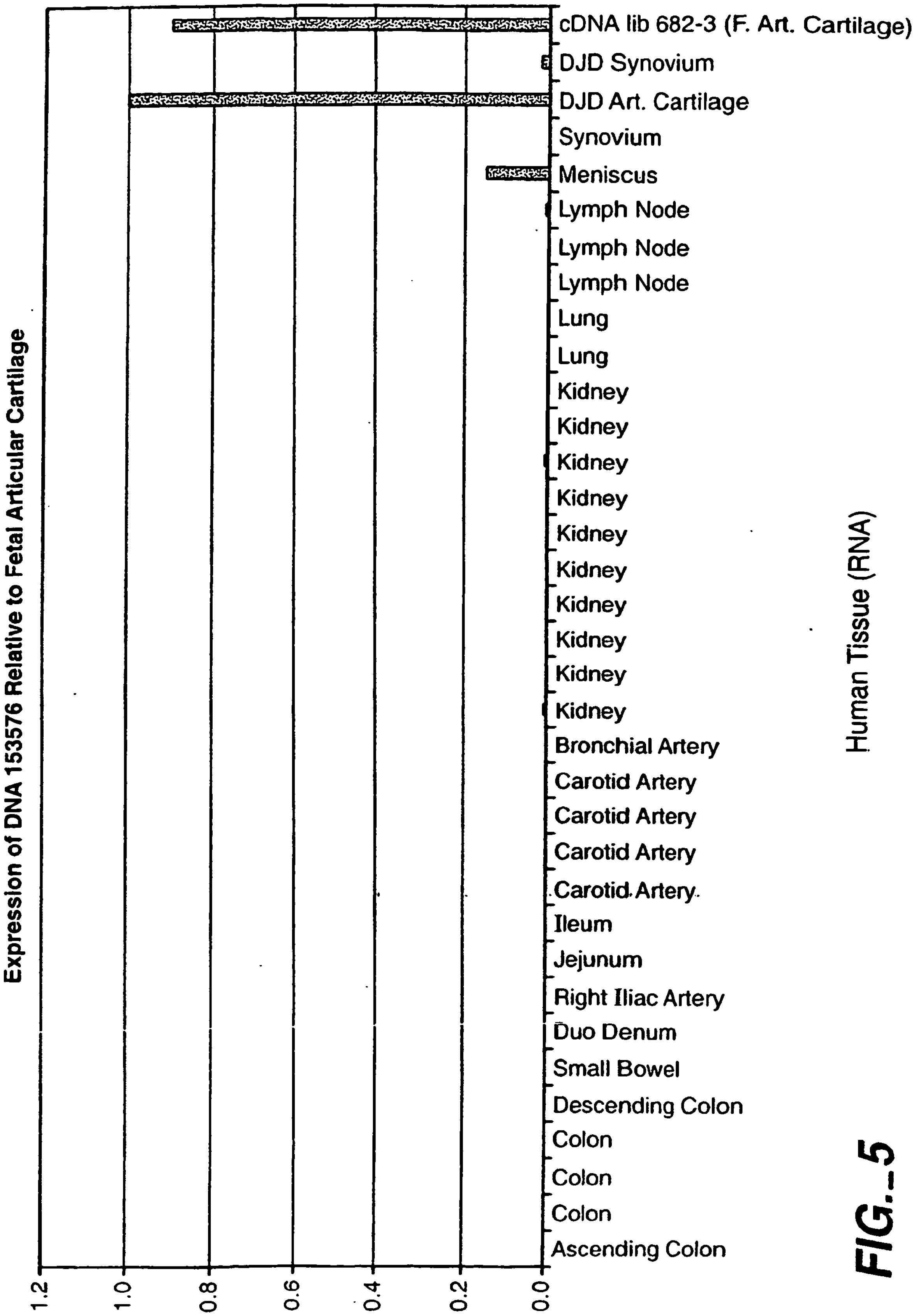
Expression of DNA 153576 Relative to Fetal Articular Cartilage
1.2
1.0
0.8
0.6
0.4
0.2
0.0
cDNA lib 682-3 (F. Art. Cartilage)
DJD Synovium
DJD Art. Cartilage
Synovium
Meniscus
Lymph Node
Lymph Node
Lymph Node
Lung
Lung
Kidney
Kidney
Kidney
Kidney
Kidney
Kidney
Kidney
Kidney
Kidney
Kidney
Bronchial Artery
Carotid Artery
Carotid Artery
Carotid Artery
Carotid Artery
Ileum
Jejunum
Right Iliac Artery
Duo Denum
Small Bowel
Descending Colon
Colon
Colon
Colon
Ascending Colon
Human Tissue (RNA)

FIG._5

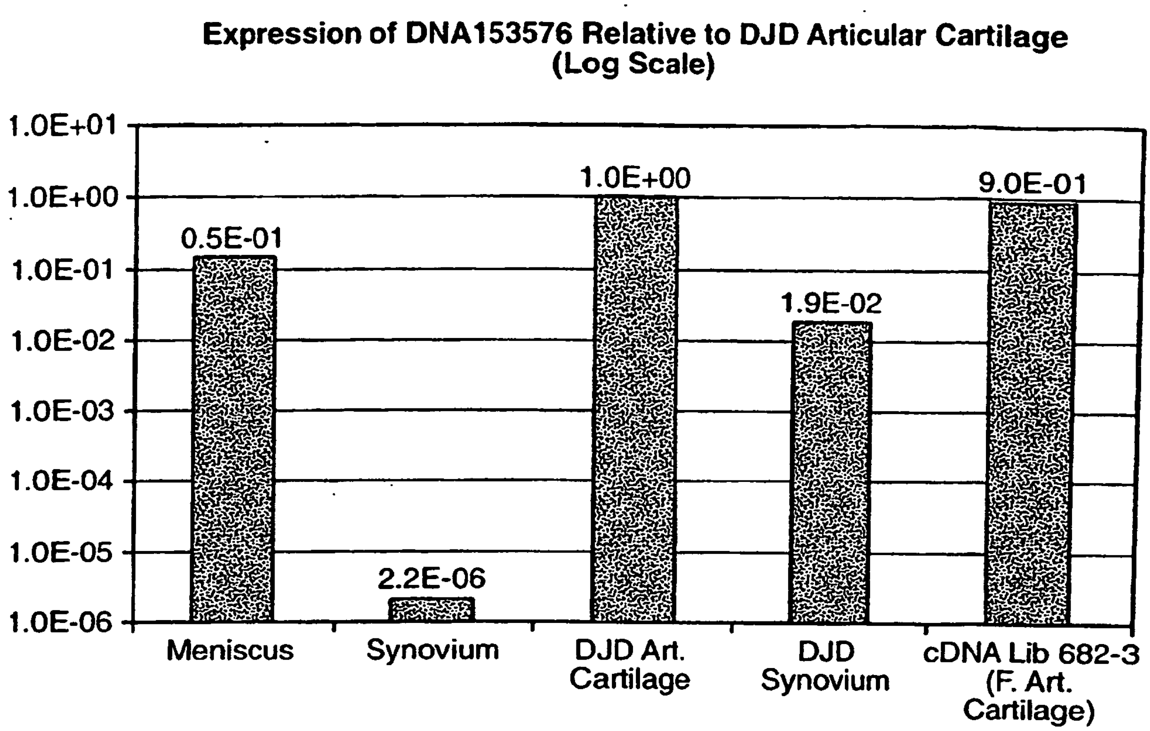
Expression of DNA153576 Relative to DJD Articular Cartilage
(Log Scale)
1.0E+01
1.0E+00
1.0E-01
1.0E-02
1.0E-03
1.0E-04
1.0E-05
1.0E-06
0.5E-01
2.2E-06
1.0E+00
1.9E-02
9.0E-01
Meniscus
Synovium
DJD Art. Cartilage
DJD Synovium
cDNA Lib 682-3 (F. Art. Cartilage)

*FIG._6*